# EUROPEAN PATENT APPLICATION

(11) **EP 1 206 906 A1**
(43) Date of publication of application: **22.05.2002**
(21) Application number: 00951968.7
(22) Date of filing: 11.08.2000
(51) Int. Cl.: A01K 67/027, C12N 15/00, C12Q 1/68, C12N 15/85, C12N 5/10, G01N 33/50, G01N 33/15

(54) **MOUSE HAVING HUMAN CYTOCHROME P450 TRANSFERRED THEREIN**

(30) Priority: 13.08.1999 JP 22909499
(71) Applicant: KIRIN BEER KABUSHIKI KAISHA, Tokyo 104-8288 (JP)
(72) Inventor: Ishida, Isao Kirin Beer Kabushiki Kaisha, Takasaki-shi Gunma 370-1202 (JP); Tomizuka, Kazuma Kirin Beer Kabushiki Kaisha, Takasaki-shi Gunma 370-1202 (JP); Kuroiwa, Yoshimi Kirin Beer Kabushiki Kaisha, Takasaki-shi Gunma 370-1202 (JP); Ohshima, Takeshi Kirin Beer Kabushiki Kaisha, Maebashi-shi Gunma 371-0853 (JP); Suzuki, Mutsumi Kirin Beer Kabushiki Kaisha, Maebashi-shi Gunma 371-0853 (JP); Itoh, Kunio Daiichi Kagakuyakuhin Kabushiki Kaisha, Tokai-mura, Naka-gun Ibaraki 319-1112 (JP)
(74) Representative: HOFFMANN - EITLE
(86) International application number: JP0005424
(87) International publication number: WO0111951

(57) **Abstract**

A partial fragment of chromosome 7 derived from human normal fibroblast is introduced into a mouse ES cell (embryonic stem cell) by a microcell method, using the ES cell, a chimeric mouse is obtained which harbors human chromosome fragment in a normal tissue, and which expresses human CYP3A4 gene in liver and small intestine due to induction by a drug. Also, to disrupt mouse endogenous P450 genes, a physical map relating to Cyp3a genes on mouse chromosome 5 is prepared, and also vectors necessary for gene targeting are prepared based on the physical map. By using the vectors, a mouse that has human P450 genes (CYP3A family) and in which mouse endogenous P450 genes (Cyp3a family) have been disrupted is prepared.

SEQUENCE LISTING

<110> Kirin Brewery Co., Ltd.

<120> Human Cytochrome P450 Gene-Introduced Mouse

<130> OP1056PCT

<140> PCT/JP00/05424
<141> 2000-08-11

<150> JP 11-229094
<151> 1999-08-13

<160> 86

<170> PatentIn Ver. 2.0

<210> 1
<211> 20
<212> DNA
<213> Artificial Sequence

<220>
<223> Description of Artificial Sequence:primer for
      detecting CYP3A4

<400> 1
gtctgtctgg gtttggaagg                                              20

<210> 2
<211> 20
<212> DNA
<213> Artificial Sequence

<220>
<223> Description of Artificial Sequence:primer for
      detecting CYP3A4

<400> 2
tctgtgttgc tctttgctgg                                              20

<210> 3
<211> 22
<212> DNA
<213> Artificial Sequence

<220>
<223> Description of Artificial Sequence:primer for
      detecting CYP3A5

<400> 3
atagaagggt ctgtctggct gg                                                22

<210> 4
<211> 22
<212> DNA
<213> Artificial Sequence

<220>
<223> Description of Artificial Sequence:primer for
      detecting CYP3A5

<400> 4
tcagctgtgt gctgttgttt gc                                                22

<210> 5
<211> 20
<212> DNA
<213> Artificial Sequence

<220>
<223> Description of Artificial Sequence:primer for
      detecting CYP3A7

<400> 5
accctgaaat gaagacgggc                                                   20

<210> 6
<211> 22
<212> DNA
<213> Artificial Sequence

<220>
<223> Description of Artificial Sequence:primer for
      detecting CYP3A7

<400> 6
gagttaatgg tgctaactgg gg                                                22

<210> 7
<211> 20
<212> DNA
<213> Artificial Sequence

<220>
<223> Description of Artificial Sequence:primer for
      detecting CYP3A cDNA

<400> 7
caagacccct ttgtggaaaa                                                   20

<210> 8
<211> 20
<212> DNA
<213> Artificial Sequence

<220>
<223> Description of Artificial Sequence:primer for
      detecting CYP3A cDNA

<400> 8
tctgagcgtt tcattcacca                                                   20

<210> 9
<211> 20
<212> DNA
<213> Artificial Sequence

<220>
<223> Description of Artificial Sequence:primer for
      detecting CYP3A4 cDNA

<400> 9
gccatagaga caagggcaag                                                    20

<210> 10
<211> 20
<212> DNA
<213> Artificial Sequence

<220>
<223> Description of Artificial Sequence:primer for
      detecting CYP3A4 cDNA

<400> 10
tcagtgaggc tgttggattg                                                    20

<210> 11
<211> 20
<212> DNA
<213> Artificial Sequence

<220>
<223> Description of Artificial Sequence:primer for
      detecting CYP3A5 cDNA

<400> 11
ctggtcaccc accatgtgta                                                    20

<210> 12
<211> 20
<212> DNA
<213> Artificial Sequence

<220>
<223> Description of Artificial Sequence:primer for
      detecting CYP3A5 cDNA

<400> 12
tatcctctca cctgcccttg                                                    20

<210> 13
<211> 21

<212> DNA
<213> Artificial Sequence

<220>
<223> Description of Artificial Sequence:primer for
      detecting CYP3A11 cDNA

<400> 13
gacacaattg tcaagggatg g                                                21

<210> 14
<211> 20
<212> DNA
<213> Artificial Sequence

<220>
<223> Description of Artificial Sequence:primer for
      detecting CYP3A11 cDNA

<400> 14
gctgacaaac aagcagggat                                                  20

<210> 15
<211> 20
<212> DNA
<213> Artificial Sequence

<220>
<223> Description of Artificial Sequence:primer for
      detecting CYP3A13 cDNA

<400> 15
atgtacctgc cctttgggag                                                  20

<210> 16
<211> 20
<212> DNA
<213> Artificial Sequence

<220>
<223> Description of Artificial Sequence:primer for

detecting CYP3A13 cDNA

<400> 16
ggagtgggtt ttctggttga                                                    20

<210> 17
<211> 20
<212> DNA
<213> Artificial Sequence

<220>
<223> Description of Artificial Sequence:primer for
      detecting CYP3A16 cDNA

<400> 17
tggtctccca tatctcaggc                                                    20

<210> 18
<211> 20
<212> DNA
<213> Artificial Sequence

<220>
<223> Description of Artificial Sequence:primer for
      detecting CYP3A16 cDNA

<400> 18
caatgtacca gtgagagcgc                                                    20

<210> 19
<211> 21
<212> DNA
<213> Artificial Sequence

<220>
<223> Description of Artificial Sequence:primer for
      detecting CYP3A25 cDNA

<400> 19
agaagaagag aaaggggagc c                                                  21

<210> 20
<211> 21
<212> DNA
<213> Artificial Sequence

<220>
<223> Description of Artificial Sequence:primer for
      detecting CYP3A25 cDNA

<400> 20
cattcaccac catgtccagg                                              20

<210> 21
<211> 20
<212> DNA
<213> Artificial Sequence

<220>
<223> Description of Artificial Sequence:primer for
      detecting CYP3A25 cDNA

<400> 21
aaaccagaag aaccgagtgg                                              20

<210> 22
<211> 20
<212> DNA
<213> Artificial Sequence

<220>
<223> Description of Artificial Sequence:primer for
      detecting CYP3A25 cDNA

<400> 22
gtgacaggtg ccttattggg                                              20

<210> 23
<211> 20
<212> DNA
<213> Artificial Sequence

<220>
<223> Description of Artificial Sequence:SP6 primer for
      clone 679

<400> 23
tagggtgaag acatggtggc                                                20

<210> 24
<211> 21
<212> DNA
<213> Artificial Sequence

<220>
<223> Description of Artificial Sequence:SP6 primer for
      clone 679

<400> 24
ggaggagagg gagatgttga t                                              21

<210> 25
<211> 21
<212> DNA
<213> Artificial Sequence

<220>
<223> Description of Artificial Sequence:T7 primer for
      clone 9736

<400> 25
agatgtgtgg ccttgttaag g                                              21

<210> 26
<211> 20
<212> DNA
<213> Artificial Sequence

<220>
<223> Description of Artificial Sequence:T7 primer for
      clone 9736

<400> 26

tttctggatg tcactggtcc 20

<210> 27
<211> 20
<212> DNA
<213> Artificial Sequence

<220>
<223> Description of Artificial Sequence:SP6 primer for
      clone 29H12

<400> 27
tagaagccca actttgggac 20

<210> 28
<211> 20
<212> DNA
<213> Artificial Sequence

<220>
<223> Description of Artificial Sequence:SP6 primer for
      clone 29H12

<400> 28
agccaacaag agggtgtacg 20

<210> 29
<211> 25
<212> DNA
<213> Artificial Sequence

<220>
<223> Description of Artificial Sequence:primer for
      CYP3A25

<400> 29
ggcctgaact gctaaaggaa agcta 25

<210> 30
<211> 26
<212> DNA

<213> Artificial Sequence

<220>
<223> Description of Artificial Sequence:primer for
      CYP3A25

<400> 30
ggtgaacatc cacaactacc attgag                                          26

<210> 31
<211> 23
<212> DNA
<213> Artificial Sequence

<220>
<223> Description of Artificial Sequence:SP6 primer for
      clone 703

<400> 31
acagggagta ggtgacactg ttg                                             23

<210> 32
<211> 22
<212> DNA
<213> Artificial Sequence

<220>
<223> Description of Artificial Sequence:SP6 primer for
      clone 703

<400> 32
gggttgttgg actcacagag ag                                              22

<210> 33
<211> 22
<212> DNA
<213> Artificial Sequence

<220>
<223> Description of Artificial Sequence:T7 primer for
      clone 703

<400> 33
gacctcacac tggcaagaag tc                                                22

<210> 34
<211> 22
<212> DNA
<213> Artificial Sequence

<220>
<223> Description of Artificial Sequence:T7 primer for
      clone 703

<400> 34
atggtgctca gcaatagtag gg                                                22

<210> 35
<211> 21
<212> DNA
<213> Artificial Sequence

<220>
<223> Description of Artificial Sequence:T7 primer for
      clone 220H2

<400> 35
gcaggatacc agatatgtgg c                                                 21

<210> 36
<211> 20
<212> DNA
<213> Artificial Sequence

<220>
<223> Description of Artificial Sequence:T7 primer for
      clone 220H2

<400> 36
aaacccctta aacccacagc                                                   20

<210> 37

<211> 20
<212> DNA
<213> Artificial Sequence

<220>
<223> Description of Artificial Sequence:SP6 primer for
      clone 220H2

<400> 37
ggtgtcacac gcctttcatc                                                    20

<210> 38
<211> 20
<212> DNA
<213> Artificial Sequence

<220>
<223> Description of Artificial Sequence:SP6 primer for
      clone 220H2

<400> 38
ctgctcaaag gagaacaggg                                                    20

<210> 39
<211> 20
<212> DNA
<213> Artificial Sequence

<220>
<223> Description of Artificial Sequence:T7 primer for
      clone 130A11

<400> 39
ccacacattc tgccaatcag                                                    20

<210> 40
<211> 20
<212> DNA
<213> Artificial Sequence

<220>

<223> Description of Artificial Sequence:T7 primer for
clone 130A11

<400> 40
agtacagcac cgggctaaag 20

<210> 41
<211> 20
<212> DNA
<213> Artificial Sequence

<220>
<223> Description of Artificial Sequence:SP6 primer for
clone 130A11

<400> 41
gcaacaataa cccaactgcc 20

<210> 42
<211> 20
<212> DNA
<213> Artificial Sequence

<220>
<223> Description of Artificial Sequence:SP6 primer for
clone 130A11

<400> 42
caggtggtaa taagctgccc 20

<210> 43
<211> 20
<212> DNA
<213> Artificial Sequence

<220>
<223> Description of Artificial Sequence:T7 primer for
clone 29B19

<400> 43
gcatgaagca ctgtctcgtc 20

<210> 44
<211> 21
<212> DNA
<213> Artificial Sequence

<220>
<223> Description of Artificial Sequence:T7 primer for
      clone 29B19

<400> 44
ggggtcctcc tttcaatatc c                                                21

<210> 45
<211> 21
<212> DNA
<213> Artificial Sequence

<220>
<223> Description of Artificial Sequence:SP6 primer for
      clone 29B19

<400> 45
tctctgcctc tcatcttagc c                                                21

<210> 46
<211> 20
<212> DNA
<213> Artificial Sequence

<220>
<223> Description of Artificial Sequence:SP6 primer for
      clone 29B19

<400> 46
tgtgtgtggg ttggtgtctc                                                  20

<210> 47
<211> 20
<212> DNA
<213> Artificial Sequence

<220>
<223> Description of Artificial Sequence:SP6 primer for
      clone 173D23

<400> 47
cggctctcac aaatagaccc                                                    20

<210> 48
<211> 20
<212> DNA
<213> Artificial Sequence

<220>
<223> Description of Artificial Sequence:SP6 primer for
      clone 173D23

<400> 48
ccaggttatc gctggtcttc                                                    20

<210> 49
<211> 21
<212> DNA
<213> Artificial Sequence

<220>
<223> Description of Artificial Sequence:SP6 primer for
      clone 86H16

<400> 49
tcacgacctt atgcaaagac c                                                  21

<210> 50
<211> 20
<212> DNA
<213> Artificial Sequence

<220>
<223> Description of Artificial Sequence:SP6 primer for
      clone 86H16

<400> 50
cgggaagtat tgctttcagc                                                    20

<210> 51
<211> 21
<212> DNA
<213> Artificial Sequence

<220>
<223> Description of Artificial Sequence:T7 primer for
      clone 86H16

<400> 51
tcagtcgtta ccagcctttt g                                                  21

<210> 52
<211> 20
<212> DNA
<213> Artificial Sequence

<220>
<223> Description of Artificial Sequence:T7 primer for
      clone 86H16

<400> 52
gagtgggctc tggtcatttc                                                    20

<210> 53
<211> 20
<212> DNA
<213> Artificial Sequence

<220>
<223> Description of Artificial Sequence:SP6 primer for
      clone 204L9

<400> 53
tctcagcata gttcaggggc                                                    20

<210> 54
<211> 20

<212> DNA
<213> Artificial Sequence

<220>
<223> Description of Artificial Sequence:SP6 primer for
      clone 204L9

<400> 54
tttctttcag ctctgggtgg                                                20

<210> 55
<211> 50
<212> DNA
<213> Artificial Sequence

<220>
<223> Description of Artificial Sequence:T7 primer for
      clone 741

<400> 55
gttcctctgt aattctaatg tacatccact ggttgaaagg tctatgataa               50

<210> 56
<211> 21
<212> DNA
<213> Artificial Sequence

<220>
<223> Description of Artificial Sequence:SP6 primer for
      lambda DOWN12

<400> 56
ttcgggaggc cagtactatt c                                              21

<210> 57
<211> 20
<212> DNA
<213> Artificial Sequence

<220>
<223> Description of Artificial Sequence:SP6 primer for

lambda DOWN12

<400> 57
cacacacgtc tttttggcag                                                    20

<210> 58
<211> 21
<212> DNA
<213> Artificial Sequence

<220>
<223> Description of Artificial Sequence: primer for
      Cyp3aY

<400> 58
acggtttatg catgctaggt g                                                  21

<210> 59
<211> 20
<212> DNA
<213> Artificial Sequence

<220>
<223> Description of Artificial Sequence: primer for
      Cyp3aY

<400> 59
tccatgacaa taaggctgcc                                                    20

<210> 60
<211> 22
<212> DNA
<213> Artificial Sequence

<220>
<223> Description of Artificial Sequence: primer for
      identification of cyp3A deleted strain

<400> 60
tgttctcctc ttcctactct cc                                                 22

<210> 61
<211> 22
<212> DNA
<213> Artificial Sequence

<220>
<223> Description of Artificial Sequence: primer for
      identification of cyp3A deleted strain

<400> 61
tgaaggtagt gaccagtgtt gg                                                22

<210> 62
<211> 21
<212> DNA
<213> Artificial Sequence

<220>
<223> Description of Artificial Sequence: primer for
      CYP3A4 and MDR1

<400> 62
ctcagaaact gtcaagcatg c                                                 21

<210> 63
<211> 20
<212> DNA
<213> Artificial Sequence

<220>
<223> Description of Artificial Sequence: primer for
      CYP3A4 and MDR1

<400> 63
cctaaaggaa acgaacagcg                                                   20

<210> 64
<211> 14
<212> DNA
<213> Artificial Sequence

<220>
<223> Description of Artificial Sequence: SfiI linker

<400> 64
pggccgcwgc ggcc 14

<210> 65
<211> 36
<212> DNA
<213> Artificial Sequence

<220>
<223> Description of Artificial Sequence: primer for
COL1A2

<400> 65
cgtaggtacc caagtcaggc ttacatttta tttgtg 36

<210> 66
<211> 36
<212> DNA
<213> Artificial Sequence

<220>
<223> Description of Artificial Sequence: primer for
COL1A2

<400> 66
cgtaggtacc gagaatgagt ttgaccttat ccattt 36

<210> 67
<211> 38
<212> DNA
<213> Artificial Sequence

<220>
<223> Description of Artificial Sequence: primer for
AF006752

<400> 67
cgtaggatcc atgtttctgg aatacttgct gttattag 38

<210> 68
<211> 28
<212> DNA
<213> Artificial Sequence

<220>
<223> Description of Artificial Sequence: primer for
      AF006752

<400> 68
caaaacaaac actattatca cttcacaa                                    28

<210> 69
<211> 20
<212> DNA
<213> Artificial Sequence

<220>
<223> Description of Artificial Sequence: primer for
      preparing PS11 probe

<400> 69
agctttgggg gacaaaacat                                             20

<210> 70
<211> 24
<212> DNA
<213> Artificial Sequence

<220>
<223> Description of Artificial Sequence: primer for
      preparing PS11 probe

<400> 70
caaaacagtc tgaacagaca ttcc                                        24

<210> 71
<211> 30
<212> DNA
<213> Artificial Sequence

<220>
<223> Description of Artificial Sequence: primer for
      identification of recombinant

<400> 71
tttctaggaa actgtaagaa aggtaagagt                                    30

<210> 72
<211> 30
<212> DNA
<213> Artificial Sequence

<220>
<223> Description of Artificial Sequence: primer for
      identification of recombinant

<400> 72
tggtattcac tttaatatca cattatctcc                                    30

<210> 73
<211> 30
<212> DNA
<213> Artificial Sequence

<220>
<223> Description of Artificial Sequence: primer for
      confirmation of recombinant

<400> 73
tatccttttt aaagttattt tgaacactca                                    30

<210> 74
<211> 30
<212> DNA
<213> Artificial Sequence

<220>
<223> Description of Artificial Sequence: primer for
      confirmation of recombinant

<400> 74
atggtttcta caaagatcgt tatgtttatc                                        30

<210> 75
<211> 30
<212> DNA
<213> Artificial Sequence

<220>
<223> Description of Artificial Sequence: primer for
      confirmation of recombinant

<400> 75
atttattatt gtcctgtttg tatctttcct                                        30

<210> 76
<211> 30
<212> DNA
<213> Artificial Sequence

<220>
<223> Description of Artificial Sequence: primer for
      confirmation of recombinant

<400> 76
atggtttcta caaagatcgt tatgtttatc                                        30

<210> 77
<211> 20
<212> DNA
<213> Artificial Sequence

<220>
<223> Description of Artificial Sequence: primer for
      RNR2

<400> 77
tcgagaattcagtagctggcactatctttttggccatc

<210> 78
<211> 37

<212> DNA
<213> Artificial Sequence

<220>
<223> Description of Artificial Sequence: primer for
      RNR2

<400> 78
tcgagaattc ggagaaagaa cacacaagga ctcggtc                                37

<210> 79
<211> 23
<212> DNA
<213> Artificial Sequence

<220>
<223> Description of Artificial Sequence: primer for
      identification of recombinant

<400> 79
tggatgtatc ctgtcaagag acc                                               23

<210> 80
<211> 22
<212> DNA
<213> Artificial Sequence

<220>
<223> Description of Artificial Sequence: primer for
      identification of recombinant

<400> 80
cagacactct atgcctgtgt gg                                                22

<210> 81
<211> 20
<212> DNA
<213> Artificial Sequence

<220>
<223> Description of Artificial Sequence: primer for

detection of human chromome 7

<400> 81
gcaagactgt gagccagtga                                                    20

<210> 82
<211> 20
<212> DNA
<213> Artificial Sequence

<220>
<223> Description of Artificial Sequence: primer for
      detection of human chromome 7

<400> 82
ggctgcatca gcatcatcta                                                    20

<210> 83
<211> 20
<212> DNA
<213> Artificial Sequence

<220>
<223> Description of Artificial Sequence: primer for
      detection of human chromome 14

<400> 83
agtgagataa gcagtggatg                                                    20

<210> 84
<211> 20
<212> DNA
<213> Artificial Sequence

<220>
<223> Description of Artificial Sequence: primer for
      detection of human chromome 14

<400> 84
gttgtgctac tcccatcact                                                    20

<210> 85
<211> 20
<212> DNA
<213> Artificial Sequence

<220>
<223> Description of Artificial Sequence: primer for
      pgk

<400> 85
atagcagctt tgctccttcg                                                    20

<210> 86
<211> 20
<212> DNA
<213> Artificial Sequence

<220>
<223> Description of Artificial Sequence: primer for
      gfp

<400> 86
ttctctcctgcacatagccc                                                     20

## Description

### TECHNICAL FIELD

The present invention has an object of providing a nonhuman animal that shows human type drug metabolism, and it relates to a nonhuman animal having introduced therein a human chromosome fragment containing cytochrome P450 genes and a method of preparing the same.

### BACKGROUND

*In vivo* metabolism of drugs is mainly performed by cytochrome P450 (hereinafter referred to as "P450") existing in the liver. P450 forms a super family consisting of many genes. P450s having a homology on an amino acid sequence in excess of 40% are grouped into the same family, and those having an amino acid sequence homology of 55% or more are classified into a subfamily (Nelson et al., Pharmacogenetics, 6:1, 1996). Human P450 and rat P450 belonging to the same subfamily show a difference in their properties upon comparison and sometimes a difference in a substance serving as a substrate or in a metabolite may be observed. For this reason, information on the metabolism of a certain drug in rat cannot be applied as it is to humans, and hence development of a test system for accurately anticipating the metabolism of a drug in human is desired (Funae et al., Bioscience and Industry, 55:81, 1997).

Basically, when examining the metabolism of drugs in human, it is the best way to use human liver microsome. However, it is difficult to obtain the human liver microsome. On the other hand, it has been becoming possible to relatively easily prepare human enzymes by genetic engineering techniques by which enzymes having the same standard can be stably supplied, and thus utilization of them may be contemplated (Kamataki, Reports of Anhyo Center Laboratory, Incorporated Foundation, 7:27, 1997).

On the other hand, *in vitro* systems have been constructed in order to examine influence of drugs metabolized and activated by P450 on living organisms. In this approach, liver microsome and a drug are added to cell culture medium and influence of a metabolite having been subjected to extracellular metabolism on the above-mentioned cells is observed. In this case, the activated substance is adsorbed on a cell membrane and only a portion thereof can reach inside of the cells. Therefore, there is a possibility that the influence of the metabolite on the cells cannot be accurately grasped. In a case where cells themselves express P450, a drug that has invaded into the cells without being adsorbed on the cell membrane may be considered to be activated in the cells, so that it is considered that influences in which the metabolite gives including toxicity can be properly reproduced. From this point of view, it has been considered that it is desirable to use cells in which human P450 gene has been introduced for an evaluation of toxicity of metabolites (Kamataki et al., Toxicology Letters, 82-83:879, 1995).

However, under the present circumstances, the above-mentioned respective methods using an *in vitro* expression system have certain problems. Although the expression system using yeast having introduced therein human P450 (for example, Kovaleva et al., Biochem. Biophys. Res. Commun., 221:129, 1996) has advantages that a certain amount of expression of P450 is obtained and expression can occur without modifying P450 cDNA, the system has a problem that the system itself contains P450 of yeast itself. Although the expression system using Escherichia coli (for example, Gillam et al., Arch. Biochem. Biophys., 305:123, 1993) is easy to handle and a large amount of enzyme can be obtained, it is necessary to modify N-terminal amino acid of P450 to be expressed in order to perform expression stably. Also there are problems that a possibility that this modification will influence enzyme activity is suggested and that Escherichia coli has no reductase necessary for exhibiting P450 activity so that a reductase must be additionally added and the like. The system using insect cells and Baculovirus (for example, Asseffa et al., Arch. Biochem. Biophys., 274:481, 1989) gives a large expression amount and needs no modification of the N-terminal amino acid; however, a certain skill is required for a manipulation for the expression. The system using a human hepatoma-derived Hep G2 cell and a vaccinia virus (for example, Shou et al., Mol. Carcinog., 10:159, 1994) and the system using human B lymphocyte may use human cells and P450 is considered to be expressed in a state closer to the *in vivo* environment. However, in the case where use of the vaccinia virus is involved or the case where Hep G2 cell microsome is used, consideration must be given to safety (Funae et al., Bioscience and Industry, 55 : 81, 1997).

On the other hand, biological role and control of drug metabolizing enzymes have not been completely elucidated yet. These experiment systems using animal cells, yeast, insects and bacteria can be models for examining a role of P450 in the *in vitro* metabolism of drugs and chemical carcinogenesis; however, they must be used based on a possibility that the *in vivo* state may be insufficiently reflected due to other elements such as parameters of pharmacokinetics (Wolf et al., J. Pharm. Pharmacol., 50:567, 1998).

Once an experimental animal that has introduced therein human P450 gene and produces in the body the same metabolite as that in human has been established, a great advantage can be obtained such as verification of not only pharmacological effects but also toxicity by use of animals even if the metabolite is one specifically produced in humans (Kamataki et al., Pharmacokinetics, 13:280, 1998). Therefore, research on transgenic mouse having introduced therein a P450 gene has been prepared. For example, Ramsden et al. prepared a transgenic mouse having introduced therein a rat Cyp2B2 gene. The expression of the rat Cyp2B2 gene is known to be controlled tissue-specifically or development-specifically and also the expression is known to be induced with phenobarbital. It has been shown that, for the expression and induction of the transgene in a transgenic mouse with phenobarbital, use of an 800-bp promoter sequence alone is not sufficient and further that an upstream gene sequence is necessary. Also, it has been shown that, in controlling the expression of transgene, the sequence by several tens kilobases upstream of a transcription initiation point is necessary and a possibility that the expression amount and tissue specificity can be reproduced by use of them has been shown (Ramsden et al., J. Biol. Chem., 268:21722, 1993).

In addition, Li et al. prepared a transgenic mouse having CYP3A7 that is specifically expressed in human fetus (Li et al., Archs. Biochem. Biophys., 329:235, 1996). In this example, metallothionein promoter was used and tissue-specific inductive expression of a P450 gene was not observed. That is, in only one line out of 6 produced lines of the transgenic mouse, expression of the introduced CYP3A7 gene was observed in liver, but in other lines their expression in various organs was observed. Therefore, in order to express the P450 gene liver-specifically, metallothionein promoter is considered to be insufficient.

As for CYP3A, studies on application as a tool for the study of toxicity in a stage of fetus are being conducted (Kamataki et al., Toxicology Letters, 82-83:879, 1995). Campbell et al. prepared a transgenic mouse having introduced therein a gene obtained by ligating the promoter of a rat Cyp1A1 gene and the sequence upstream thereof to a lacZ gene, and by use of the mouse progressed the analysis on the control of gene expression by the Cyp1A1 promoter (Campbell et al., J. Cell Sci., 109:2619, 1996). Hitherto there has been no report on transgenic animals having introduced therein one subfamily (composed of approximately 2 to 7 genes) of a human P450 gene in such a form that drugs metabolized by the P450 gene products induce their expression.

Not only a transgenic mouse but also a P450 gene knockout mouse have been developed, which are expected to be an important tool for elucidating the influence of P450 on development and homeostasis on a cell level and the role of P450 in toxicity *in vivo* through drugs and chemical substances (McKinnon et al., Clin. Exp. Pharmacol. Physiol., 25:783, 1998). For example, endogenous Cyp1a2 knockout mice were prepared by two research groups. The Cyp1a2 knockout mouse prepared by Pineau et al. (Pineau et al., Proc. Natl Acad. Sci. USA., 92:5134,1995) was normal when it is heterozygous but died when it is homozygous shortly after the birth. On the other hand, the Cyp1a2 knockout mouse prepared by Liang et al. showed no abnormality in phenotype of homozygous individual (Liang et al., Proc. Natl Acad. Sci. USA., 93:1671, 1996). The difference is considered to be attributable to a difference in a sequence of the gene to be deleted. In addition, there have been reports on the influence of and metabolic abnormality due to deletion of the P450 gene (for example, Genter et al., Biochem. Pharmacol., 55:1819, 1998), and the role of Cyp1a2 in a metabolic system has been being elucidated by use of such a knockout mice. Also, as for Cyp2e1, known as a main enzyme in metabolizing ethanol, a knockout mouse was prepared (Lee et al., J. Biol. Chem., 271:12063, 1996). Cyp2e1 is known to participate in metabolism of, besides ethanol, acetaminophen, acetone and arachidonic acid. Even a homologous individual of which Cyp2e1 was completely deficit, it did not apparently look different from a wild type mouse but it had increased resistance to acetaminophen. This and results of pathological observation suggested that Cyp2e1 participated in hepatotoxicity due to acetaminophen. The respective P450 gene knockout mice were prepared in order to elucidate the function of a gene by the knockout of the gene and not to increase the expression level of an exogenous P450 gene to be introduced.

### DISCLOSURE OF THE INVENTION

Based on the above-mentioned background art, an object of the present invention is to provide a modified model animal for examining *in vivo* metabolism of drugs, in particular a model animal that harbors a human P450 gene and performs human type metabolism of drugs.

The present inventors have successfully introduced a partial fragment of a human normal fibroblast-derived chromosome 7 into a mouse ES cell (embryonic stem cell) by means of a microcell method and therby obtained a cell line harboring the fragment. In addition, by use of the ES cell, they have obtained a chimeric mouse that harbors the human chromosome fragment in normal tissues and expresses a human CYP3A4 gene in liver and small intestine by induction with a drug. Furthermore, in order to disrupt the mouse endogenous P450 genes they prepared a physical map on a Cyp3a gene on the mouse chromosome 5 and prepared a vector necessary for gene targeting based on the physical map. This made it possible to make a mouse that has human P450 genes (CYP3A family) and in which the mouse endogenous P450 genes (Cyp3a family) have been disrupted, i.e., a so-called gene-substituted mouse. Thus, the present invention has been completed.

The present invention provides the following:
(1) A nonhuman mammalian which harbors at least one human cytochrome P450 gene, whererin expression of the gene is induced by a compound serving as a substrate for a product of the gene.
(2) A nonhuman mammalian according to (1), wherein the human cytochrome P450 gene belongs to a CYP3A family.
(3) A nonhuman mammalian according to (1), wherein the human cytochrome P450 gene is introduced by a YAC vector containing the gene or microcell fusion using a chromosome fragment containing the gene.
(4) A nonhuman animal according to (3), wherein the mammalian is a chimeric animal.
(5) A nonhuman animal, which is obtained by mating a wild-type nonhuman animal of the same kind as the nonhuman animal according to the above item (4) with the nonhuman animal according to the above item (4) and which harbors a chromosome fragment containing a human cytochrome P450 gene.
(6) A nonhuman mammalian according to (1), wherein a cytochrome P450 gene inherent to the nonhuman mammalian that is a homolog of the human cytochrome P450 gene has been disrupted and expression of the inherent gene has been reduced or lost.
(7) A nonhuman mammalian according to (6), wherein disruption of the cytochrome P450 gene inherent to the nonhuman mammalian has been performed using a Cre-loxP system.
(8) A nonhuman mammalian according to any one of the above items (1) to (7), wherein the mammalian is a mouse.
(9) A cell, or organ or tissue containing the cell, the cell being derived from the nonhuman mammalian according to any one of the above items (1) to (8) and being capable of expressing a human cytochrome P450 gene.
(10) A method for preparing a physical map for determining arrangement of mouse Cyp3a genes on a chromosome, comprising the steps of:
   (a) screening a mouse BAC library by PCR or hybridization using PCR primers or a probe for hybridization for specifically detecting each mouse Cyp3a genes;
   (b) repeating a cycle of screening the BAC library several times to prepare a BAC contig, the cycle comprising determining a terminal nucleotide sequence of the BAC clones selected in the above step and preparing a primer or a probe based on the nucleotide sequence; and
   (c) determining both ends of a Cyp3a gene cluster by preparing a full-length cDNA probe of an unprescribed mouse Cyp3a gene and performing hybridization of the above-mentioned BAC contig using the probe under the gentle conditions.
(11) A physical map that can be prepared by the method according to the above item (10) and having elucidated the arrangement of the mouse Cyp3a genes on the chromosome.
(12) A method of preparing a targeting vector for deleting mouse Cyp3a genes, comprising cloning a genome DNA corresponding to respective terminals of a mouse Cyp3a gene cluster based on the physical map according to (11) and inserting each of the obtained cloned fragments into a vector containing a loxP sequence, which is a recognition sequence of recombinase Cre derived from bacteriophage P1.
(13) A pair of targeting vectors for deleting mouse Cyp3a genes which can be prepared by the method according to the above item (12), wherein the respective vectors are to be incorporated in a mouse chromosome and only when the enzyme Cre is present, homologous recombination occurs between the loxP sequences, thereby deleting the whole Cyp3a gene cluster.
(14) A method of deleting Cyp3a genes of a mouse cell, comprising the steps of introducing the vector according to the above item (13) into a cell retaining pluripotency of mouse and expressing enzyme Cre.
(15) A mouse cell that can be prepared by the method according to (14), being deficient in Cyp3a genes and retaining pluripotency.
(16) A method for preparing a knockout mouse deficient in Cyp3a genes, comprising the step of differentiating the mouse cell according to the above item (15).
(17) A chimeric mouse according to the above item (16) or a progeny thereof prepared by the method, being deficient in Cyp3a genes.
(18) A mouse or a progeny thereof deficient in Cyp3a genes, obtained by mating the chimeric mouse according to the above item (17) or a progeny thereof with a wild-type mouse.
(19) A tissue derived from the chimeric mouse according to the above item (16) or a progeny thereof or the mouse according to the above item (17) or a progeny thereof.
(20) A cell derived from the chimeric mouse according to the above item (16) or a progeny thereof or the mouse according to the above item (17) or a progeny thereof.
(21) A mouse or a progeny thereof obtained by mating the mouse according to the above item (8) or a progeny thereof with the chimeric mouse according to the above item (17) or a progeny thereof or the mouse according to the above item (18) or a progeny thereof, the mouse or a progeny harboring a human chromosome containing human P450 genes and being deficient in mouse Cyp3a genes.
(22) A method of producing biologically active human cytochrome P450, comprising the steps of cultivating an individual, tissue or cell of the mouse according to the above item (8) or a progeny thereof, expressing the human cytochrome P450 genes harbored therein to produce biologically active human cytochrome P450, and recovering the human cytochrome P450.
(23) A method of producing biologically active human cytochrome P450, comprising the steps of cultivating an individual, tissue or cell of the mouse according to the above item (21) or a progeny thereof, expressing the human cytochrome P450 genes harbored therein to produce biologically active human cytochrome P450, and recovering the human cytochrome P450.
(24) A method of examining pharmacological effect and/or metabolism of a drug, comprising the step of administering the drug to an individual, tissue or cell of the mouse according to the above item (8) or a progeny thereof or the mouse according to the above item (21) or a progeny thereof.

Also, the present invention provides a nonhuman animal that harbors at least CYP3A4 of the human cytochrome P450 gene. The present invention provides a nonhuman animal that harbors at least CYP3A4, CYP3A5, and CYP3A7 of the human cytochrome P450 gene. The present invention provides a nonhuman animal that harbors a human chromosome 7 fragment in which a CYP3A family of the human cytochrome P450 gene is located. Furthermore, the present invention provides a human artificial chromosome containing the human chromosome 7 fragment where the CYP3A family of the human cytochrome P450 gene is located and a human chromosome 14 fragment and a nonhuman animal that harbors the artificial chromosome.

Hereinafter, the cytochrome P450 is sometimes referred to simply as "P450" in the present specification.

Hereinafter, the outline of the present invention will be described.

To introduce a human P450 gene into a mouse and examine the metabolism of drugs *in vivo,* the expression amount, tissue specificity, pattern of enzyme induction and the like of the induced gene need to be reproduced in the same manner as the gene originally functions in humans. It is required that the gene to be introduced is free of artificial modification as much as possible and it is introduced as a large unit including an expression control region. The method of introducing the gene into a mouse is not particularly limited as far as it satisfies these requirements. Methods using a YAC vector (Jakobovits et al., Nature, 362:255, 1993, etc.) may be used. Also, a method in which a chromosome fragment is introduced into a mouse ES cell by microcell fusion to make a chimeric mouse as disclosed in WO97/07671 may be used. Particularly preferred is the latter method, which can introduce a gene of a longer region (hereinafter, this method is referred to as "microcell fusion method" in the present specification for convenience' sake). The reason for this is as follows.
(1) Generally, a P450 molecular species is considered to form a cluster for every family (for example, Gray et al., Genomics, 28:328, 1995; Hoffman et al., J. Mol. Evol., 41:894, 1995). It is anticipated that in a case where a cluster is formed, the respective genes have at least partially a common function and complement their respective functions with each other; and thus it is considered more desirable to introduce the gene in a form of a whole cluster including the gene rather than the gene alone. The microcell fusion method has an advantage of length of the gene that can be introduced.
(2) It is quite conceivable that control of the expression of P450 gene is influenced not only by a promoter but also by a cis-acting element distal to a region encoding 450 protein. In the transgenic mouse having rat having introduced therein the above-mentioned Cyp2B2 gene, it has been shown that the sequence contained within 19 kbp upstream of the promoter is necessary for liver-specific expression of Cyp2B2 (Ramsden et al., J. Biol. Chem., 268:21722, 1993), which clearly indicates that merely using P450 gene linked to a high-power promoter in an expression system is not means for reproducing tissue specificity and enzyme induction which is close to those in a living organism. It is expected that introduction of a sequence extending from a region where the gene is encoded to a region as remote as possible from the gene-encoded region will result in reproduction of expression control in a human in a mouse.
(3) In the above-mentioned report by Ramsden et al., the animal prepared is a transgenic mouse and it may be considered that expression of a gene is influenced by the position where the intoduced gene is integrated (Wolf et al., J. Pharm. Pharmacol., 50:567, 1998). In the microcell fusion method, it is conceivable that human chromosomes can be introduced into a cell in an independent state without being integrated into a genome of a host, and thus such a positional effect can be avoided.

Further, it is desirable that a model animal relating to drug metabolism using a transgenic animal is free of influence of the background of the animal species. For example, in a case where a human-specific P450 gene is introduced, mouse P450 originally expressed in a mouse may hide changes of modes of metabolism of drugs and expression of toxicity into those of a human type (Wolf et al., J. Pharm. Pharmacol., 50:567, 1998). Therefore, if a human P450 gene is to be introduced into a mouse, it is desirable that a counterpart gene that the mouse originally has, i.e., mouse homolog of a P450 gene is knocked out. Also, it is conceivable that, for an important gene, there exists another gene that complements its function so that there will be no severe influence on the living organism in a case where its function is lost. Introduction of a gene together with such a complementary gene and knockout are considered to give rise to a state closer to human.

One specific aspect of the present invention relates to introduction of CYP3A genes out of a human P450 gene family into a nonhuman mammalian. In the case of human, P450 belonging to CYP1 to 4 participates in the metabolism of xenobiotics including various drugs. In particular, CYP3A4 belonging to the CYP3A family exists in human liver microsome with the highest content and the number of drugs metabolized by CYP3A4 is large so that it has very broad substrate selectivity (Funae et al., Bioscience and Industry, 55 : 81, 1997).

As will be described in examples described hereinbelow, a chimeric mouse having introduced therein a human chromosome 7 or its fragment containing CYP3A genes according to the method described in WO97/07671 is prepared. Further, in a preferred aspect, endogenous mouse Cyp3a genes are disrupted in order to efficiently express human CYP3A genes and to make the drug metabolism by the CYP3A genes on the human chromosome 7 closer to that of a human type. That is, for a mouse in which a human gene has been introduced, the gene of the mouse is disrupted. Conversely, it is possible to introduce a human gene to a mouse whose endogenous Cyp3a genes have been disrupted. Furthermore, a desired mouse can be obtained by mating a human gene introduced mouse with a mouse gene-disrupted mouse.

Mouse endogenous Cyp3a genes are considered to form a cluster on the mouse chromosome 5 similarly to the human CYP3A genes (for example, D. R. Nelson et al., Pharmacogenetics, 6:1, 1996). Up to now, at least 4 genes, i.e., Cyp3a11 13, and 16 (for example, D. R. Nelson et al., Pharmacogenetics, 6:1-42, 1996) and Cyp3a25 (only the nucleotide sequence of cDNA was reported on the NCBI database) have been identified. In order to completely disrupt the mouse endogenous Cyp3a genes, at least these 4 genes must be disrupted. When a conventional gene disruption (knockout) method (for example, Mansour et al., Nature, 336:348, 1988) is used, these 4 genes must be disrupted individually, and thus a knockout experiment must be performed at least 4 times. When two or more genes are to be disrupted, if the target genes exist on a chromosome physically sufficiently remote from each other, it may be considered to make knockout mice in which the respective genes are disrupted and then mate them to make a knockout mouse of a plurality of genes (for example, N. Longberg et al., Nature, 368:856, 1994).

However, in the present invention, the Cyp3a genes which are the target genes, form a cluster, and therefore it is difficult to conceive that the genes existing on the chromosome are physically remote enough to allow recombination of the chromosome by way of crossing upon meiotic division. In short, it is necessary to perform knockout experiments at least 4 times in the same mouse ES cell (for example, Evans et al., Nature, 292:154, 1981) to prepare a knockout mouse rather than to make knockout mice individually and then mate them. Furthermore, the Cyp3a genes to be disrupted are not always sufficient with these 4 genes, and that possibly is conceived in which there still exists a Cyp 3a gene that remains to be identified. Therefore, it must be determined first how many Cyp 3a genes to be disrupted are present. Then, the knockout must be performed for the same number of times as the number of genes thus identified.

An ES cell, which is an embryonic undifferentiated cell that has totipotency, which it can be differentiated into all the tissues of an individual, naturally loses its totipotency as the number of times of cloning in the knockout experiment increases; in particular, there is a risk of losing a useful chimeric mouse in which the ES cell has been differentiated into germ cell line. From these, it is considered that it is extremely difficult in the conventional method to disrupt mouse endogenous Cyp3a genes in the present invention.

In recent years, a mutant mouse preparing system combining a recombinase Cre derived from bacteriophage P1 and its recognition sequence, i.e., a 34mer loxP sequence, has been developed. This system has been used mainly for conditionally performing mutation (for example, Gu. H et al., Science, 265:103, 1994). That is, when it is intended to delete a certain gene region, a loxP sequence is first inserted into both ends of the gene region by homologous recombination so that recombination can occur between the loxP sequences only when a Cre enzyme is expressed, with the result that the target gene region is deleted. At this time, by rendering the Cre enzyme gene under the control of development stage-specific gene promoter or a tissue-specific gene promoter, the target gene can be deleted at a specific stage in development or in a specific tissue. Further, by using high recombination efficiency between the loxP sequences, a large part of the target gene region is deleted (for example, Z-W. Li et al., Proc. Natl. Acad. Sci. USA, 93:6158, 1996). Gene region deletion by usual homologous recombination exhibits lower recombination frequency the larger its deleted region becomes, and deletion of up to about 20 kb is considered to be a limit. In contrast, deletion of a gene region of about several hundreds kb is becoming possible by using a Cre-loxP system. That is, it is considered to be possible to delete not only a single gene but also a gene together with several genes adjacent thereto as a whole.

Accordingly, in the present invention, based on the mouse endogenous Cyp3a genes forming a cluster as described above, it has been considered to attempt to insert a loxP sequence to each end of the cluster and delete the genes together with the whole cluster. However, it has been completely unknown as to how large the Cyp3a genes cluster is and what kinds of genes constitute both ends thereof; therefore, at a first stage, a physical map of the cluster must be prepared. First, a procedure is adopted in which so-called chromosome walking including first using known Cyp3a11, 13, 16, and 25 as starting materials to clone a macro DNA containing them and further cloning an adjacent macro DNA using the both ends thereof as a probe (for example, I. B-Moreau et al., Genomics, 46:183, 1997) is performed to make a continuous macro DNA set (contig). For preparing the contig, it is necessary to clone a macro DNA and in a conventional system, a cosmid using Escherichia coli as a host or YAC (Yeast Artificial chromosome) vector using yeast as a host have been used. The cosmid has a cloning capacity of about 50 kb and with this size much labor and effort are required for preparing a physical map of a cluster extending over several hundreds kb.

On the other hand, the YAC vector can clone a macro DNA of 2 Mb at the maximum and hence has been exclusively used for preparing a rough physical map on a level of a chromosome (for example, S. W. Scherer et al., Hum. Mol. Genet., 2:751, 1993). However, because of such a high cloning capacity, cloning may occur in a vector with genome fragments differing in their positions on the chromosome from each other being coupled (so-called YAC chimera) or deletion of a part of YAC or recombination may occur during cultivation in yeast as a host; therefore, YAC is not so stable.

On the other hand, in recent years, a system of BAC (Bacterial Artificial chromosome) has been developed that is stable in a host and has a cloning size between those of cosmid and YAC (for example, H. Shizuya et al., Proc. Natl. Acad. Sci. USA, 89:8794, 1992). The host is Escherichia coli and the system is constructed based on F factor plasmid existing in the bacterial cell body as a single copy. The cloning size is about 300 kb at the maximum and 100 to 120 kb in average. In an Escherichia coli cell, the system exists as a single copy and is very stable, so that it can be handled in the same manner as ordinary plasmids. Therefore, it allows sequence analysis to be performed very easily and hence becomes a powerful tool for a human genome project being performed on a worldwide scale. The fact that sequencing can be performed means that it is possible to make a terminal probe (STS primer) and readily perform screening of adjacent BAC clones. Based on these advantages, it is preferable to use the BAC system in preparing the physical map of the mouse Cyp3a gene cluster according to the present invention.

As a method of preparing a specific physical map using the BAC system, the following method is conceivable. First, a PCR primer or a probe for hybridization is prepared that can specifically detect Cyp3a11, 13, 16, and 25, and a mouse BAC library is screened. Then terminal sequencing of the obtained BAC clone is performed to make a primer or probe and again the BAC library is screened. The screening is repeated for several times to make a BAC contig extending over several hundreds kb. Finally, in order to determine both ends of the cluster, a full-length cDNA probe of any one of the Cyp3a genes is prepared and hybridization of the obtained BAC contig is performed under mild conditions (i.e., under conditions where a BAC clone containing at least Cyp 3a11, 13, 16, and 25 becomes positive). Of the BAC contigs that are judged positive with this probe, the outermost positioned BAC clones are considered to correspond to both ends of the cluster. It is conceivable that once both ends of the cluster are determined by this method, unknown Cyp3a genes in the cluster, in whatever numbers they may be, can be deleted as a whole.

Next, after preparing the physical map of the mouse Cyp3a gene cluster by the BAC system, genome DNAs corresponding to both ends of the cluster are cloned to make an actual targeting vector. This vector contains the loxP sequence as described above and can be inserted into both ends of the cluster in an ES cell by homologous recombination. Then the Cre expression vector is transfected into the ES cell to cause recombination at both ends of the cluster by means of a Cre-loxP system, with the result that the Cyp3a gene cluster is deleted. It cannot be anticipated how frequent deletion of the cluster supposed to extend over several hundreds kb will occur by means of the Cre-loxP system. Generally, it is believed that the larger the gene region to be deleted, the lower the recombination efficiency, and hence the deletion efficiency of the cluster of several hundreds kb achieved in the present invention is expected to be by no means sufficiently high. Accordingly, a positive selection system that enables selection of cells having undergone recombination between the loxPs must be thought about.

In recent years, A. J. H. Smith et al. (Nature Genet., 9:376, 1995) were successful in mutually translocating a chromosome 12 and a chromosome 15 in a mouse ES cell by using Cre-loxP system. They inserted the 5'-side portion of an Hprt gene (containing loxP) and the 3'-side portion (containing loxP) to specific regions of the chromosomes 12 and 15, respectively, in an Hprt (hypoxanthine guanine phosphoribosyl transferase) gene-deficit ES cell by homologous recombination. Then Cre is expressed, and, only when recombination occurs between the loxPs, the Hprt gene is reconstituted, resulting in that the ES cell comes to be able to proliferate even in a medium containing no hypoxanthine (HAT medium). This selection method can be used only for Hprt gene-deficient ES cells and is not utilized for any ES cell. Accordingly, in the present invention, the following selection method has been thought out so that the selection method can be utilized for any ES cell.

In recent years, a GFP (green fluorescent protein) gene derived from bowl jellyfish has been developed (for example, Prasher, D. C et al., Gene, 111:229, 1992). Luminescence of GFP requires no substrate and can be detected by fluorescence, and thus it can be monitored in a living cell in a short time. This is considered to be important when considering retaining of the totipotency of the ES cell. As stated above, in the present invention, it has been determined to use a GFP gene as a positive selection marker of a loxP recombinant. Specifically, a GFP gene is inserted into one end of a Cyp 3a cluster and a promoter necessary for the expression of GFP is inserted into the remaining end. When recombination due to Cre occurs between the loxP sequences, the promoter and the GFP gene are coupled to express GFP. This recombinant ES cell emits fluorescence so that selection becomes possible.

A chimeric mouse is prepared from the obtained ES cell (Cyp 3a gene cluster is deleted heterozygously) and mated with a wild type mouse to obtain an F1 mouse which deletes Cyp3a gene cluster heterozygously. On the other hand, a chimeric mouse harboring a human chromosome 7 fragment is mated with a wild type mouse to obtain an F1 mouse harboring a human chromosome 7 fragment. This F1 mouse and the above-mentioned F1 mouse of which the Cyp3a gene cluster is heterozygously deleted are mated to obtain an F2 mouse of which the Cyp3a gene cluster is heterozygously deleted and which harbors the human chromosome 7 fragment. By mating this F2 mouse and the F1 mouse of which the Cyp3a gene cluster is heterozygously deleted, a final mouse (of which the Cyp3a gene cluster is homozygously deleted and which harbors the human chromosome 7 fragment) can be obtained.

Hereinafter, the present invention will be described in more detail. In the following embodiments, explanation will be made on mice as the nonhuman mammalian. However, the present invention is not limited to use of mice.

A mouse individual which harbors a human chromosome or its fragment containing human P450 genes on its own chromosome in an expressible form and is deficient in counterpart mouse endogenous P450 genes can be obtained through the processes of:
(1) preparing a chromosome donor cell harboring a chromosome or its fragment containing labeled human P450 genes,
(2) transferring the human chromosome or its fragment to a mouse cell retaining pluripotency by a microcell method,
(3) preparing a chimeric mouse using the above-mentioned mouse cell,
(4) harboring the human chromosome containing the human 450 genes and confirming the expression of the human P450 genes in the chimeric mouse,
(5) preparing a physical map of mouse endogenous P450 genes,
(6) preparing a knockout vector based on the above-mentioned physical map,
(7) gene targeting in a mouse cell retaining pluripotency,
(8) preparing a knockout mouse using the above-mentioned mouse cell,
(9) confirming deletion of the mouse P450 genes in the knockout mouse, and
(10) mating the mouse harboring the human chromosome containing the human P450 genes with the mouse lacking the mouse endogenous P450 genes.

The mouse having introduced therein human P450 gene or P450 genes by such a procedure or further a mouse deleting mouse endogenous P450 genes is sometimes referred to as "human P450 gene introduced mouse".

Hereinafter, an example of a method of preparing a human P450 gene introduced mouse will be illustrated in detail step by step.
(1) Preparing a chromosome donor cell harboring a chromosome or its fragment containing a labeled human P450 gene

As the chromosome donor cell, a cell which 1) harbors a human chromosome labeled with a marker that can be selected in a recipient cell, 2) contains no human chromosome other than the above chromosome, and 3) has high microcell forming ability is desirable.

As a material for providing a human chromosome, all the cell lines, cancer cells, and primary culture cells derived from a human can be used. considering low possibility of occurrence of abnormality such as deletion or amplification of chromosome and of ease of culture, a normal fibroblast is suitable.

First, as for the above item 1), the human cell may be transformed by a vector that expresses a marker gene such as a drug (G418, puromycin, hygromycin, or blasticidin) resistant gene. The promoter for controlling the expression of a marker used herein is desirably the one that can efficiently work not only in a human cell but also in a recipient cell such as a mouse ES cell. For this purpose, a promoter consisting of a SV40 enhancer and a herpes simplex virus thymidine kinase promoter coupled thereto (Katoh et al., Cell Struct.Funct., 12:575, 1987), a mouse PGK-1 promoter (Soriano et al., Cell, 64:693, 1991) and the like may be used.

By transforming a human cell with a DNA fragment containing the marker gene ligating a promoter as needed by electroporation or the like (Ishida et al., Introduction to Cell Engineering Experiment Manipulation, Kodansha Publishing, 1992) and selecting a cell that expresses the marker gene, a human cell transformant library in which introduced marker gene is inserted at random into a human chromosome of 23 kinds and 46 in number.

As for the above item 3), since many of the normal human cells have very low microcell forming ability, the above-mentioned transformant is subjected to whole cell fusion with a cell having high microcell forming ability, for example, a mouse A9 cell (Oshimura, M., Environ. Health Perspect., 93:57, 1991) to impart microcell forming ability to the obtained mouse. It is known that in a mouse-human hybrid cell, the human chromosome is selectively lost. In contrast, in the fused cell line obtained here, the marked human chromosome can be stably harbored by selection by means of the above-mentioned marker.

Further, in order to satisfy the condition in the above item 2), it is desirable that a microcell is acquired from the cell fused cell line and fused again with a mouse A9 cell. In this case, it is considered that most of the microcell fused cell line thus obtained satisfy the conditions of the above items 1), 2) and 3) above by selection by means of a marker on the human chromosome. The marked human chromosome can be identified by examining in the mouse-human monochromosome hybrid cell obtained in the final stage by PCR (polymerase chain reaction, Sakaki et al., Science, 239:487, 1988), Southern blot analysis (Ausubel et al., Current protocols in molecular biology, John Willy & Sons, Inc., 1994), FISH (Fluorescence in situ hybridization, Lawrence et al., Cell, 52:51,1988) analysis or the like.

In a case where a chromosome containing the human P450 gene is introduced, the above-described procedure is repeated for many human cell transformant clones to search for the clones with marked objective chromosomes. Alternatively, the above-mentioned procedure is implemented for a mixture of human cell transformant clones and a large number of mouse-human monochrosome hybrid cells obtained may be subjected to identification of a chromosome containing human P450 genes. Furthermore, a marker gene can be inserted at a specified site by homologous recombination targeting a DNA sequence on the chromosome containing human P450 genes (Thomas et al., Cell, 51:503, 1987).

By irradiating gamma rays to a microcell prepated from a mouse-human hybrid cell, a chromosome containing the marked human P450 genes can be fragmented and then introduced into a mouse A9 cell. In addition, when no gamma rays is irradiated to a microcell, a chromosome containing human P450 genes partially fragmented at a certain ratio might be transferred to the microcell. In these cases, the obtained microcell fused cell line harbors the partial fragment of the chromosome containing the marked human P450 genes. These can be used when introduction of a partial fragment of the chromosome containing human P450 genes into a recipient cell is desired.

The human chromosome introduced into a recipient cell such as a mouse ES cell may undergo various modifications such as deletion, translocation, substitution, inversion, polyploidization or the like as far as introduction of the objective cytochrome P450 gene is possible. Conventionally, as the human antibody-producing mouse prepared by Tomizuka et al. (Nature Genet., 16:133-143, 1997), a spontaneously fragmented human chromosome fragment or full-length human chromosome it self has been used for its introduction into a mouse. As such, in the case where a spontaneously fragmented human chromosome fragment or full-length human chromosome is used, there could arise a problem that, depending on the human chromosome introduced, the chromosome or its fragment is unstable in the mouse or the gene on the human chromosome gives an influence on the development of the mouse so that no human chromosome-introduced mouse itself is given birth to. Therefore, in order to make a mouse that stably harbors all the human chromosome fragments in the future, there is a demand for a technology of freely processing a chromosome, such as cleaving the human chromosome at a desired site or splicing only a desired chromosome fragment to another stable chromosome in order to serve the intended purpose rather than using spontaneously formed chromosome fragments. Such a technology is called chromosome engineering and has been utilized for site-specifically cleaving an endogenous mouse chromosome mainly in a mouse ES cell (WO 98/54348), or causing recombination (translocation) between homologous chromosomes to cause deletion, inversion or polyploidization of a specified gene region to make a mutant mouse having such a modified chromosome (R.Ramirez-Solis et al., Nature 378:720, 1995). On the other hand, no report has been made yet on nonhuman animals such as mice harboring human chromosomes having undergone various artificial modifications.

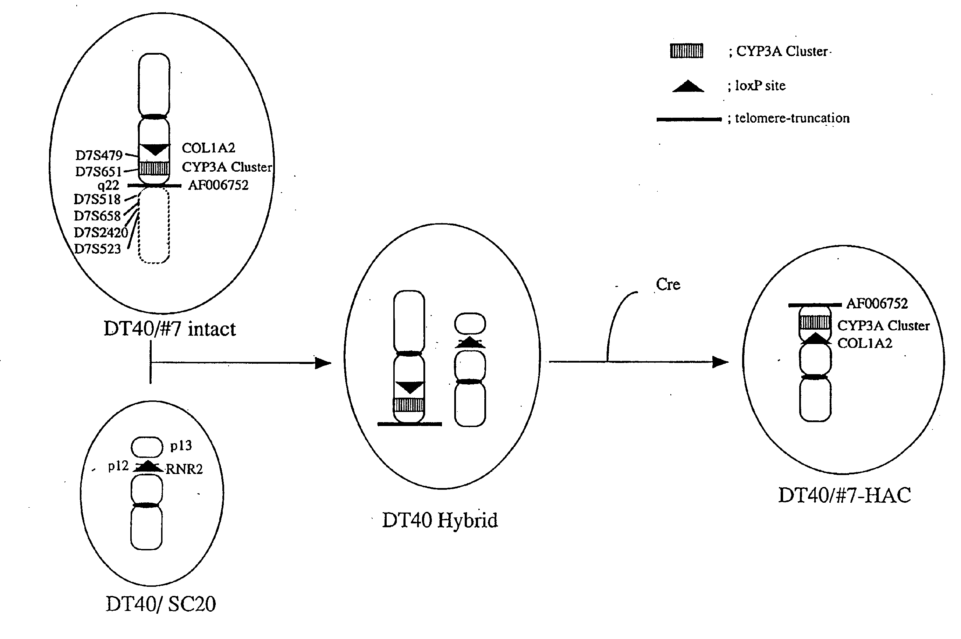

Accordingly, the present invention discloses a construction of a human artificial chromosome that harbors a human chromosome 7 fragment (hereinafter referred to as "#7-HAC") in order to stably and efficiently express CYP3A genes on the human chromosome 7 in a mouse. The construction of this #7-HAC can be implemented, for example, by the following procedure (see Fig. 12).

### (i) Modification of human chromosome 7

A human CYP3A gene cluster is present in 7q21-22 on the chromosome 7 (for example, B.A.Brooks et al., Am. J. Hum. Genet., 43:280, 1988). In order to use only a periphery of the chromosome region in preparing an artificial chromosome, a human telomere sequence is first inserted in a chromosome site (for example, ARC41, etc.) just telomeric to the CYP3A gene cluster by homologous recombination to effect telomere translocation (for example, Kuroiwa et al., Nucleic Acid Research 26:3447, 1998), thus performing cleaving.

Incidentally, the human chromosome 7 fragment containing the CYP3A gene cluster may further contain an MDR gene cluster (PGy1,3) . On a just centromeric side of the CYP3A gene cluster, there is the MDR gene cluster (PGy1,3 (NCBI database). The MDR1 gene participates in extracellular excretion of drugs (for example, Juliano,R.L. et al., Biochemi.Biophys. Acta. 455:152, 1976) and it has been reported that it differs depending on the species for a specified drug (for example, Lecureur V. et al., Carcinogenesis, 17:1157, 1996). Since most substrates of CYP3A4 are also substrates of MDR1 (for example, Schuetz. EG et al., Proc. Natl Acad. Sci. USA., 93:4001, 1996), it is considered that introduction of the MDR gene cluster in addition to the human CYP3A gene cluster in preparing humnan type drug metalbolism model animals is useful. Accordingly, an loxP sequence, which is a recognition sequence for recombinase Cre, is inserted into a chromosome site just centromeric to the MDR gene cluster (for example, AC004082 NCBI database) by homologous recombination and only an MDR-CYP3A fragment is used in preparing artificial chromosome. Also in MDR gene, like P450 a gene, it is desirable that a homologous gene on the side of a host animal be deficient or inactivated. In mice, cases of knockout of Mdr1a and Mdr1b genes have been reported (Schinkel et al., Proc. Natl Acad. Sci. USA., 94:4028, 1997; and Schinkel et al., Cell, 77:491, 1994), and Mdr gene deficit mouse can be prepared according to the method described in these articles. By incorporating the Mdr gene deficit mouse into the above-mentioned mating program, a mouse homozygously deleting the mouse Cyp3a gene cluster and the mouse Mdr1a and/or Mdr1b genes and that harbors a chromosome fragment containing a human CYP3A gene cluster and a human MDR gene cluster can be obtained.

### (ii) Modification of human chromosome 14 fragment SC20

In the human antibody-producing mouse prepared by Tomizuka et al. (Nature Genet., 16:133-143, 1997), it has been shown that the human chromosome 14 fragment SC20 is harbored in the mouse by approximately 100% and transferred to descendents (WO98/37757). Accordingly, in the present invention, it is contemplated to use the SC20 as a human artificial chromosome vector. That is, the CYP3A human chromosome 7 fragment described in the above item (i) above is translocated to SC20 to construct a human artificial chromosome #7-HAC that harbors a human chromosome 7 fragment. By inserting the loxP sequence in the RNR2 gene locus located at 14p12 on the chromosome 14 by homologous recombination and using a Cre-loxP system (for example, R. Ramirez-Solis et al., Nature, 378:720, 1995), the CYS3A human chromosome 7 fragment is translocated to the RNR2 gene locus on SC20.

As a host cell for efficiently performing the homologous recombination described in th above items (i) and (ii), a chicken DT40 cell (Riken Cell Bank:RCB1464, ATCC:CRL-2111) may be used (for example, Dieken et al., Nature Genet., 12:174, 1996). Hereinafter, an example of using the DT40 cell will be described. Note that, homologous recombination is possible also in an ES cell although the efficiency is low as compared with the DT40 cell.

### (iii) Translocation of CYP3A human chromosome 7 fragment onto SC20 by Cre-loxP system

DT40 cells that harbor the modified human chromosome 7 fragment and modified SC20 obtained as shown in the above items (i) and (ii), respectively, are prepared. Then, in order to translocate the CYP3A human chromosome 7 fragment onto the SC20 by the Cre-loxP system, the DT40 cells harboring respective modified human chromosome fragments are fused to make a DT40 cell hybrid that harbors two modified human chromosome fragments. Thus, all the materials for translocation are available.

Next step is to express Cre recombination enzyme in the DT40 cell hybrid for translocation. Since it has been reported that recombination (translocation) frequency between two nonhomologous chromosomes is very low (for example, A. J. Smith et al., Nature Genet., 9:376, 1995), and further since in the case of the present invention, translocation occurs not between endogenous chromosomes but between exogenous human chromosomes, it may be possible that the recombination efficiency will be lower. Hence, it is preferable that a positive selection system that enables selection of a cell in which recombination has occurred between loxPs as expected is thought out. Accordingly, in a preferred aspect of the present invention, as already stated in the item of deletion of an endogenous mouse Cyp3a gene cluster, a system is used in which a GFP gene is expressed in the cell in which translocation of a chromosome has occurred between loxPs and the objective cell is cloned by sorting by means of FACS. Furthermore, in order to increase recombination frequency, the Cre recombination enzyme is expressed not temporarily but in a stable manner. The translocation between two chromosomes occur in a mutual fashion; when stable expression of the Cre recombination enzyme is performed, there is a possibility that the chromosome that has once undergone translocation undergoes, though at low frequencies, recombination (translocation) again to revert to the original state. Considering, usually in such an experiment, it is contemplated that the Cre recombination enzyme is expressed temporarily or expression of the Cre recombination enzyme is strictly controlled. However, in the present invention, immediately after the translocation in the DT40 hybrid, the objective translocated human artificial chromosome is transferred to a Chinese hamster CHO cell by a microcell fusion method, and thus the CHO cell is not influenced by the Cre recombination enzyme. Thus, Cre recombination enzyme can be simply and stably expressed.

As disclosed in the above items (i), (ii) and (iii), by using telomere translocation and chromosome translocation by means of a Cre-loxP system in a chiken DT40 cell, any human chromosome fragment having any size(exceeding cloning size of a YAC vector) can also be cloned on the loxP site (14p12) on a stable SC20 chromosome vector; thus a human artificial chromosome construction system is proposed.

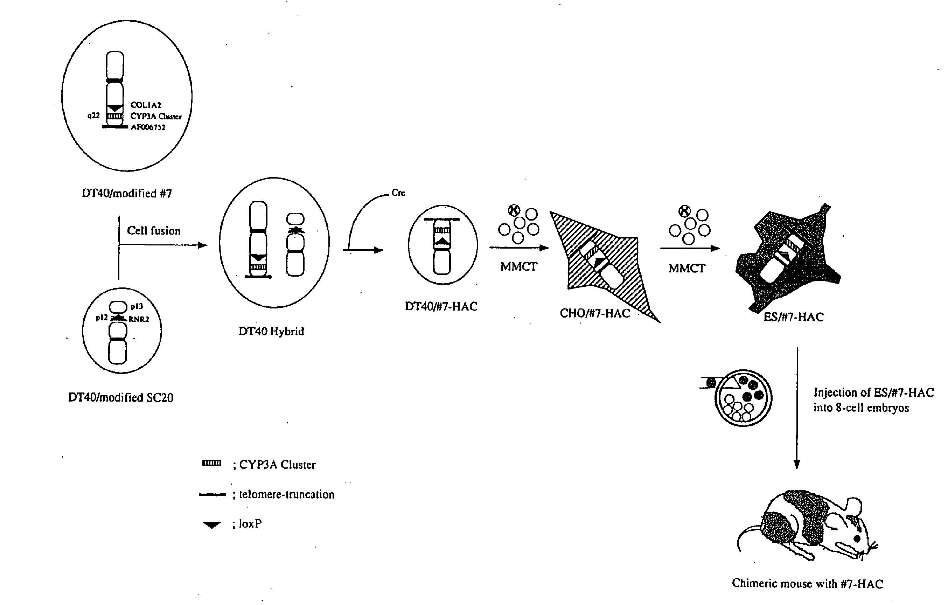

#7-HAC prepared by using the above-mentioned human artificial chromosome construction system should be introduced in a Chinese hamster CHO cell before it is introduced in a mouse ES cell. Dieken et al. (Nature Genet., 12:174, 1996) transferred modified human chromosomes from chicken DT40 cells to mouse MEL cells (a kind of cancer cell) but the chromosomes were transferred mostly in a fragmentized state. The present inventors also have tried to transfer human chromosomes from chicken DT40 cells to mouse A9 cells or the like, but in all cases fragmentation of human chromosomes was observed, and in no case they were transferred in an intact state. However, the present inventors have for the first time found that only when the human chromosomes were transferred into Chinese hamster CHO cells, they could be transferred in an intact state. Therefore, in the examples described hereinbelow, #7-HAC prepared by using a human artificial chromosome construction system was once transferred to a CHO cell. It has been known that CHO cells, like moue A9 cells, efficiently form microcells (for example, M. Koi et al., SCIENCE 260:361, 1993), and it is conceivable that this makes it possible to transfer a modified human chromosome from a CHO cell to a mouse ES cell to prepare #7-HAC harboring chimeric mouse. An outline of the above procedure is shown in Fig. 16.

### (2) Transfer of a chromosome or its fragment containing a human P450 gene into a mouse cell having pluripotency

Hitherto, it has been reported that injection of an embryonic carcinoma cell (EC cell, Hanaoka et al., Differentiation, 48:83, 1991), an embryonic stem cell (ES cell, Evans et al., Nature, 292:154, 1981), and an embryonic germ cell (EG cell, Matsui et al., Cell, 70:841, 1992) derived from various mouse lines into an early embryo of a mouse, or co-cultivation of such cells with an early embryo of a mouse results in a change (differentiation) of the various cells which are undifferentiated into somatic cells having various forms or functions through differentiation; that is, it is possible to make a chimeric mouse. Es and EG cells are particularly high in that ability and in many cases also contribute to germ cells and can make progenies derived from the cells. EC cells are obtained mainly from germ cell cancer. ES cells are obtained from an inner cell mass of blastocyst. EG cells are obtained from primordial germ cells expressed in an early stage of development. In the present invention, a recipient cell that can be used for the transfer of a chromosome containing a human P450 gene includes these cell lines and mutants thereof, and further all the undifferentiated cells that can differentiate into all or a part of normal somatic cells in a mouse individual.

As a material for the transfer of a human chromosome into a recipient, a microcell prepared from a chromosome donor cell containing the human P450 gene obtained in the above item (1) or a microcell irradiated with gamma ray can be used. The transfer into the recipient cells can be performed by fusing the recipient cells with a micro cell by a method described in (Motoyuki Shimizu, Cell Engineering Handbook, Youdosha, 1992) and the like. The microcell donor cell harbors a marker by which a chromosome or its fragment containing a human P450 gene can be screened in the recipient cells. Of the recipient cells, a cell line that harbors a human P450 gene or a chromosome or fragment thereof containing the human P450 gene is selected by PCR, Southern blot analysis, FISH analysis or the like in the same manner as in the above item (1) 1, which makes it possible to introduce a chromosome or its fragment containing a human P450 gene. Also, by sequentially introducing a plurality of chromosomes or fragments thereof containing human P450 genes having different selection markers, recipient cells simultaneously harboring them can be obtained.

The fact that the recipient cell selected by a marker (G418 resistance, etc.) on the chromosome containing a human P450 gene harbors all or a part of the chromosome containing a human P450 gene or a human P450 gene that has been harbored in the donor cell is confirmed as follows. That is, it is detected by chromosome analysis such as fluorescence in situ hybridization (FISH) using genome DNA extracted from the selected recipient cell and as a probe a human-specific repeated sequence (L1. Alu, etc., Korenberg et al., Cell, 53:391, 1988) or human chromosome specific probe (Lichter et al., Human Genetics, 80:224, 1988), a PCR method by means of a human P450 gene specific primer, or Southern analysis using a human P450 gene specific sequence probe.

### (3) Preparation of a chimeric mouse from a mouse cell having introduced therein a chromosome containing a human P450 gene

Preparation of a chimeric mouse from a mouse cell line such as the ES cell line obtained in the above item (2) above is performed by the method described in (Shinichi Aizawa, Biomanual Series 8 Gene Targeting, Youdosha, 1995) and the like. As for selection of a stage of development and phylogeny of a host embryo for efficiently performing preparation of a chimeric mouse, it is desirable that conditions already studied for the respective ES cell lines are used. In a case of, for example, a TT2 cell derived from CBA X C57BL/6 F1 (wild-type color, Yagi et al., Analytical Biochemistry, 214:70, 1993), it is desirable to use 8-cell stage embryo derived from Balb/c (white, CLEA Japan, Inc.) or ICR (CLEA Japan, Inc.) as a host embryo.

### (4) Confirmation of retention of a chromosome containing human P450 genes and of expression of a human gene in a chimeric mouse

A contribution ratio of an ES cell in a mouse born from an embryo to which an ES cell has been injected can be roughly judged by its hair color. However, if no contribution is observed in the hair color, it cannot be judged that it makes no contribution to other tissues. Retention of a human chromosome in each tissue of chimeric mouse in more detail can be confirmed by Southern analysis, PCR or the like using genome DNA extracted from each tissue.

The expression of a human P450 gene on the introduced chromosome can be confirmed as follows. That is, the expression of mRNA derived from a chromosome containing a human P450 gene can be detected by an RT-PCR method (Kawasaki et al., Proc. Natl. Acad. Sci. USA, 85:5698, 1988) or Northern blotting method (Ausubel et al., supra) using RNA derived from each of the tissues. Expression on a level of protein can be detected by Western blotting (Ausubel et al., supra) or testosterone 6β-hydroxylation activity measurement using chimeric mouse liver microsome or the like. Further, retention of a chromosome containing human P450 genes and expression of the gene on the chromosome in the cells of chimeric mouse can be confirmed by emergence of resistant cells due to the expression of a drug resistance marker gene in primary culture cells derived from the chimeric mouse.

In a case where the ES cell that harbors a chromosome containing human P450 genes are differentiated into germ cells in the chimeric mouse, the chromosome or its fragment containing human P450 genes is also found in the progeny and the gene on the chromosome or its fragment is expressed in the progeny.

### (5) Preparation of a physical map of mouse endogenous P450 genes

In order to make a mouse deficient in mouse endogenous P450 genes (Cyp3a cluster) that will be a counterpart to the introduced human P450 genes, it is necessary to determine a sequence order in which each Cyp3a gene in the cluster is arranged, namely, a physical map. For preparing such a physical map, a system having small cloning size such as plasmid is unsuitable and a system that can clone a macro DNA is indispensable. For this purpose, hitherto, physical maps have been prepared by using a YAC (for example, S. W. Scherer et al., Human Molecular Genetics, 2:751, 1993) vector, which is a yeast artificial chromosome that can clone a cosmid with a cloning size of about 40 kb or DNA with cloning size of up to 2 Mb at the maximum. By using a cosmid vector, maps with high accuracy can be prepared due to its small cloning size. On the other hand, use of the YAC vector enables preparation of a rough map over a wide range on the chromosome level. Alternatively, a recently developed bacteria artificial chromosome BAC (H. Shizuya et al., Proc. Natl. Acad. Sci. USA, 89:8794, 1992) vector with a cloning size between the above two (100-300 kb) may be used. The BAC vector displays its power in chromosome walking, large-scale sequencing of a genome and so on as a result of an advantage that it enables to perform terminal sequencing of inserted DNA without difficulty. By using these vector systems, a physical map of a mouse endogenous Cyp3a cluster on a chromosome can be prepared.

For example, a physical map using the BAC vector can be prepared as follows. That is, a PCR primer or a probe for hybridization that can specifically detect respective (Cyp3a11, 13, 16 and 25) mouse Cyp3a genes is prepared, with which a commercially available mouse BAC library can be screened. The obtained BAC clone is subjected to terminal sequencing, and a primer or a probe is prepared. Again BAC library is screened therewith. By repeating the terminal sequencing and screening several times, the BAC contig over several hundreds kb can be prepared. Finally, in order to determine both ends of the cluster, a full-length cDNA probe of any one of the mouse Cyp3a genes is prepared and the obtained BAC contig is subjected to hybridization under mild conditions. As for the conditions,it is desirable that all the BAC clones containing at least any one of the 4 genes, i.e., Cyp3a11 13, 16 and 25 that have been identified up to now be positive. Among the BAC clones that turn into positive with this probe, the clones located outermost of the contig correspond to both ends of the cluster, and thus the physical map of the cluster can be completed. Alternatively, as performed in genome projects, this can be achieved by determining the whole sequence of this BAC contig.

### (6) Preparation of a targeting vector based on the above-mentioned physical map

After preparing the physical map of the mouse Cyp3a gene cluster using a BAC system, the genome DNA corresponding to both ends of the cluster may be cloned to prepare an actual targeting vector. This vector contains therein loxP sequences as described above, which can be inserted into both ends of the cluster by homologous recombination in an ES cell. Then, a Cre expression vector is transfected into the ES cell to cause recombination at both ends of the cluster by means of the Cre-loxP system, resulting in that the Cyp3a gene cluster is deleted.

As a similar system, a yeast FLP/FRT system (for example, S. Jung et al., Science, 259:984, 1993) may be used. In a case where a cluster presumably extending to several hundreds kb is deleted by a Cre-loxP system, it cannot be anticipated at what efficiency the deletion may occur. Accordingly, as a positive selection system for selecting cells in which recombination has occurred between the loxPs, for example, expression of a GFP gene can be used as a marker. Specifically, a GFP gene containing no promoter is inserted into one end of the Cyp3a cluster, and a promoter necessary for the expression of GFP is inserted into the remaining end. When recombination takes place between the loxP sequences by Cre, the promoter and the GFP gene are coupled to express GFP. Since such a recombinant ES cell emits fluorescence, selection becomes possible.

As another selection marker, it is possible to use a certain kind of drug resistance gene. In this case, as a result of the Cre-loxP recombination, the drug resistance gene is expressed, which makes it possible to select recombinant ES cells in the selection medium containing the drug. In any case, the selection method is desirably one that gives as less influence as possible on the totipotency of the ES cell.

### (7) Gene targeting in mouse cells retaining pluripotency

The targeting vector prepared in the above item (6) is introduced into a mouse cell retaining pluripotency, for example, an ES cell. At this time, it is desirable that the introduction be performed under conditions established for introducing genes into ES cells, such as electroporation as described in: <Shinichi Aizawa, Biomanual Series 8 Gene Targeting, Youdosha, 1995>. As stated above, if the Cre-loxP system is to be used in order to delete the gene as the whole cluster containing the gene, it is desirable to make confirmtion as to whether or not homologous recombination has occurred by PCR, Southern analysis or the like and at the same time as to whether or not an ES cell has pluripotency every time when loxP sequences are introduced to upstream and downstream of the cluster. That is, a chimeric mouse is prepared by using ES clones having loxP sequences obtained by introduction of the respective vectors and homologous recombination in the genome, and the efficiency of the preparation is studied. Similarly, the vector that expresses Cre enzyme is introduced into ES cells harboring loxP sequences at both ends of the cluster of a mouse endogenous Cyp3a gene by electroporation or the like.

Cre is not necessarily incorporated in a mouse genome and it may be a promoter or a vector structure that is temporarily expressed. Finally, for the selection of ES cells deficient in a Cyp3a gene as a cluster, it is convenient to use not only methods for directly analyzing genes such as PCR and Southern analysis but also selection with a reporter gene such as GFP in combination.

### (8) Preparation of knockout mouse using the above-mentioned mouse cell

The preparation of a knockout mouse is the same as the method described in the above item (3).

### (9) Confirmation of deficiency of mouse P450 genes in a knockout mouse

A contribution ratio of an ES cell in the prepared knockout mouse can be roughly judged by its hair color as in the case of the human chromosome-introduced chimeric mouse. Furthermore, it is desirable to confirm the deficiency of an endogenous gene by Southern analysis, PCR or the like using the genome DNA extracted from the knockout mouse. Also, in a case where GFP is inserted in a targeting portion, expression is also observed in a mouse individual; hence selection of the knockout mouse can be readily achieved by means of fluorescence as in the selection in the ES cells.

### (10) Preparation of a mouse harboring a human chromosome containing human P450 genes and lacking mouse endogenous P450 genes

A human P450 mouse harboring a human chromosome containing the human P450 genes and lacking the mouse endogenous P450 genes is obtained by mating a chimeric mouse harboring a human chromosome containing human P450 genes prepared by a method described above or its progeny with a chimeric mouse deficient in the endogenous mouse P450 genes as a whole cluster prepared by a method described above or its progeny. As for "harboring a human chromosome" and "deficient in the endogenous P450 gene", hereditary determinant of the both are considered to be transmitted essentially according to the Mendel's law. Various patterns are possible for a method of mating mice. Specifically, the mating can be implemented as follows. First, a chimeric mouse heterozygously deleting the Cyp3a gene cluster is mated with a wild-type mouse to obtain an F1 mouse heterozygously deleting the Cyp3a gene cluster (F1 mouse A). On the other hand, a chimeric mouse harboring a human chromosome 7 fragment is mated with a wild-type mouse to obtain an F1 mouse harboring a human chromosome 7 fragment (F1 mouse B). F1 mouse A and F1 mouse B are mated to obtain an F2 mouse heterozygously deleting the Cyp3a gene cluster and retaining a human chromosome 7 fragment. By mating this F2 mouse with the F1 mouse A, a final mouse (homozygously deleting a mouse Cyp3a gene cluster and retaining a human chromosome 7 fragment) is obtained.

Also, a mouse endogenous P450 gene deficit mouse is preferably obtained by using a heterozygous individual for mating rather than preparing a homozygous individual and using it. This is because it has been reported that homozygously gene deficit individuals of knockout mice are often lethal and there is a possibility that they will die in a stage of fetus or neonate.

By using the chimeric mouse obtained as described above, biologically active human P450 protein can be obtained from organs, tissues and cells thereof. For example, by extracting a fraction containing human P450 from liver microsome of the chimeric mouse or its progeny and purifying it, human P450 protein can be extracted. Alternatively, the human P450 protein can be recovered from a culture by establishing a cell line from a tissue of the chimeric mouse harboring a chromosome containing a human P450 gene or its progeny and cultivating it. Further, the obtained chimeric mouse or its progeny or a human P450 introduced mouse obtained by mating them with a knockout mouse deficient in the mouse endogenous P450 genes are considered to express in their bodies human P450 in the same pattern as it is originally expressed in a human body; therefore, they are highly useful as test animals for studying pharmacological effects, toxicity, pharmacokinetics at a level of an individual by administering a specified drug thereto. Furthermore, the mechanism of metabolism of drugs and toxicity of drugs per tissue of human can be known without actually administering the drugs to human.

### BRIEF DESCRIPTION OF THE DRAWINGS

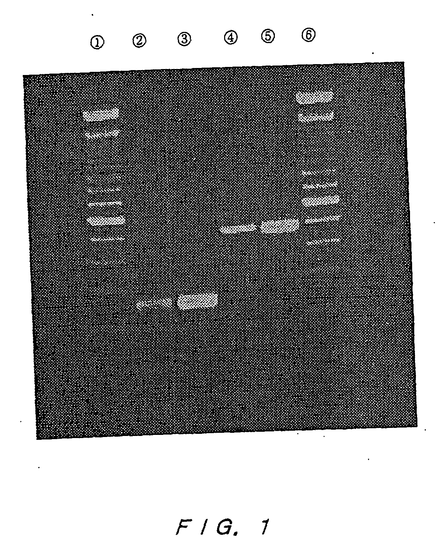

Fig. 1 is an electrophoretic photograph of RT-PCR using RNA derived from liver of rifampicin-administered chimeric mouse, showing expression of human CYP3A4 in the liver of rifampicin-administered chimeric mouse.
   [1], [6] : Marker
   [2]: *Eco*RI-treated PCR product of RNA derived from rifampicin-administered mouse liver
   [3]: *Eco*Rl-treated PCR product of RNA derived from human liver
   [4] : PCR product of RNA derived from rifampicin-administered mouse liver
   [5]: PCR product of RNA derived from human liver
Fig. 2 is an electrophoretic photograph (RT-PCR) showing results of examination on the expression of human CYP3A4 in various organs of rifampicin-administered chimeric mouse, indicating expression of human CYP3A4 in the liver and small intestine.
   [1], [8] : Marker
   [2] : Liver
   [3]: Small intestine
   [4] : Kidney
   [5] : Spleen
   [6] : Lung
   [7] : Heart
Fig. 3 is a diagram illustrating preparation of contig of BAC clone containing mouse Cyp3a11, 13, and 25.
Fig. 4 is a diagram showing the structures of cassette vectors ploxPUP and ploxPDOWN for inserting loxP sequences at the both ends of Cyp3a gene cluster.
Fig. 5 is a diagram showing a scheme of targeting with respect to mouse Cyp3a region.
Fig. 6 is a diagram showing the structure of a targeting vector ploxP3aUP for deleting mouse Cyp3a gene cluster.
Fig. 7 is a diagram showing the structure of a targeting vector plox3aDOWN for deleting mouse Cyp3a gene cluster.
Fig. 8 is a diagram showing the structures of cassette vectors ploxPHyg and ploxPbsr for inserting a loxP sequence on a human chromosome.
Fig. 9 is a diagram showing the structures of targeting vectors pBSCOL1A21ox(F) and pBSCOL1A21ox(R) for inserting loxP sequences in a genome region (COL1A2) located on the COL1A2 gene locus on the human chromosome 7 and showing identification of a homologous recombinant.
Fig. 10 is a diagram showing the structure of a cassette vector pTELPuro for inserting human telomere sequence onto a human chromosome.
Fig. 11 is a diagram showing the structures of targeting vectors pTELPuro5.3(F) and pTELPuro5.3(R) for inserting human telomere sequences in a genome region (AF006752) located on the CYP3A gene locus cluster on the human chromosome 7 and showing identification of a homologous recombinant.
Fig. 12 is a diagram showing existence of a gene and a polymorphic marker on the human chromosome 7'and construction of human artificial chromosome #7-HAC.
Fig. 13 is a diagram showing the structure of a targeting vector pRNR21oxPbsr for inserting loxP sequences to the RNR2 gene locus on the human chromosome 14 and showing identification of a homologous recombinant.
Fig. 14 is a diagram showing the structure of a Cre recombinase stable expression vector pBS185hisD.
Fig. 15 is a diagram showing the detection of site-specific translocation of human chromosome 7 fragment to chromosome 14 fragment SC20.
Fig. 16 is a diagram showing an outline of preparation of chimeric mouse harboring #7-HAC.

### Best Mode for carrying out the Invention

Hereinafter, the present invention will be described in more detail with reference to examples. However, the present invention is not limited thereto.

### Example 1 Screening of mouse A9 cell harboring human chromosome 7 fragment

### (1) Preparation of mouse A9 cell library harboring a human chromosome

A mouse A9 cell library harboring various human chromosomes was prepared according to the method described in WO97/07671. Specifically, this was performed as described below.

Plasmid pSTneoB containing G418 resistant gene (Katoh et al., Cell Struct. Funct., 1 2:575, 1987; Japanese Collection of Research Biologicals (JCRB), Deposit Number: VE039) was linearized with restriction enzyme *Sal*I (Takara Shuzo Co., Ltd.) and introduced into human normal fibroblast HFL-1 (obtained from RIKEN Cell Bank, RCB0251). The HFL-1 cells were treated with trypsin and suspended in Dulbecco's phosphate buffer (PBS) in a concentration of 5×10⁶ cells/ml and then electroporated in the presence of 10 µg of DNA by use of a gene pulser (Biorad) (Ishida et al., Introduction to Cell Engineering Experiment Manipulation, Kodansha, 1992). 1,000 V of voltage at a capacitance of 25 µF was applied at room temperature by use of a 4 mm-long electroporation cuvet (165-2088, Biorad). The electroporated cells were plated onto 3 to 6 100-mm tissue culture plastic dishes (Corning) containing 15% fetal calf serum (FBS)-added Eagle F12 medium (hereinafter, referred to as "F12"). After a day, it is replaced by 15% FBS-added F12 medium containing 200 µg/ml of G418 (GENETICIN, Sigma). After 2 to 3 weeks the appeared colonies were assorted into 52 groups taking about 100 colonies as one group, which were subcultured again in 100 mm dishes and cultivated.

Mouse A9 cells (Oshimura, Environ. Health Perspect., 93:57, 1991, JCRB0211) were cultivated in a 100 mm dish containing 10% fetal calf serum (FBS)-added Dulbecco's modified Eagle medium (hereinafter, referred to as "DMEM"). 52 groups of G418-resistant HFL-1 cells were cultivated in 100 mm dishes containing F12 to which 15% fetal calf serum (FBS) and 200 µg/ml G418 were added. Mouse A9 cells and HFL-1 cells were treated with trypsin and then mixed each in a quarter or half amount and the mixture was cultivated in an equivalent weight mixture of 10% fetal calf serum (FBS)-added DMEM and 15% fetal calf serum (FBS)-added F12 for a half day to an entire day. Cell fusion was performed according to the method described in Shimizu et al., Cell Engineering Handbook, Youdosha, p. 127-, 1992. After washing the cell surface with DMEM twice, the cells were treated with 2 ml of a PEG (1:1.4) solution for 1 minute and then after exchanging the solution for 2 ml of PEG (1:3) solotion and the cells were treated for 1 minute. The PEG solution was absorbed and the cells were washed with serum-free medium (DMEM) three times and then cultivated in ordinary medium (10% FBS, DMEM) for 1 day. The cells were dispersed by treatment with trypsin, suspended in a double selection medium (10% FBS, DMEM) containing ouabain (1×10⁻⁵ M, Sigma) and G418 (800 µg/ml), and plated onto three 100 mm dishes. After 3 weeks cultivation, the appeared colonies were dispersed into single cells by treatment with trypsin and cultivated in a selection medium (10% FBS, DMEM) containing G418(800 µg/ml).

The cells were dispersed by trypsin treatment and two groups were assorted into one. Then, they were cultivated in six 25-cm³ centrifugation flasks (Coaster, 3025) to a cell density of 70 to 80% saturation or so. After exchanging the medium for a culture solution (20% FBS, DMEM) containing colcemid (0.05 µg/ml, demecolcin, Wako Pure Chemical Industry Ltd.), cultivation was performed for 2 days to form microcells. The culture solution was removed, a preliminarily warmed (37°C) cytochalasin B (10 µg/ml, Sigma) solution was filled in the centrifugation flasks, and the centrifugation flasks were mounted in an acrylic resin made centrifugation vessel and centrifugation was performed at 34°C at 8,000 rpm for 1 hour. The microcells were suspended in serum-free medium and filtered through a filter to purify them. In the 25 cm³ flasks in which Mouse A9 cells had been cultivated to 80% saturation was added purified micronuclei and fused in PEG solution. The fused cells were cultivated in a selection medium containing G418 and colonies were isolated.

All the experimental manipulation and reagents and the like other than described above were according to Shimizu et al., Cell Engineering Handbook, Youdosha, p. 127-.

### (2) Identification of Mouse A9 cell clone harboring human chromosome 7 fragment

From the above-mentioned mouse A9 cell library, a clone harboring chromosome 7 fragment on which the human CYP3A gene cluster is present was identified by means of PCR using primers that could specifically detect human CYP3A4 gene as follows.

### (Primers for detecting CYP3A4)

Sense:     5'-GTCTGTCTGGGTTTGGAAGG-3' (SEQ ID No. 1)
Antisense   5'-TCTGTGTTGCTCTTTGCTGG-3' (SEQ ID No. 2)

In PCR, GeneAmp 9600 produced by Perkin-Elmer, Inc. was used as a thermal cycler, Ex Taq (Takara Shuzo Co., Ltd.) was used as a Taq polymerase, and as the buffer and dNTP (dATP, dGTP, dCTP and dTTP) those attached to Ex Taq (Takara Shuzo Co., Ltd.) were used under the recommended conditions. The cycle condition of PCR was such that after thermal denaturation at 94°C for 1 minute, the cycle of 98°C for 10 seconds, 65°C for 30 seconds, and 72°C for 1 minute was performed 35 times. As a result, 20 positive clones were obtained. Furthermore, these clones were subjected to PCR by use of a human chromosome 7 polymorphic marker (D7S664, D7S1496. D7S634. D7S648, end D7S505, BIOS, Corp.). The results are shown in Table 1. o indicates positive and x indicates negative.

In Table 1, for example, clones H5 and Z9 were considered to harbor the full-length of chromosome 7. On the other hand, clone E22 was considered to harbor a fragment that contains only the periphery of CYP3A4. Then, in order to examine how human chromosome 7 (fragment) exists in these clones, FISH analysis using human COT-1 DNA as a probe was performed. The method for this was in accordance with the method described in WO97/07671. As was expected from the results of PCR, it revealed that there existed independently full-length human chromosome 7 in clone H5 and small human chromosome 7 fragment in clone E22. The clones H5 and E22 were used as human chromosome 7 (fragment) donor cells.

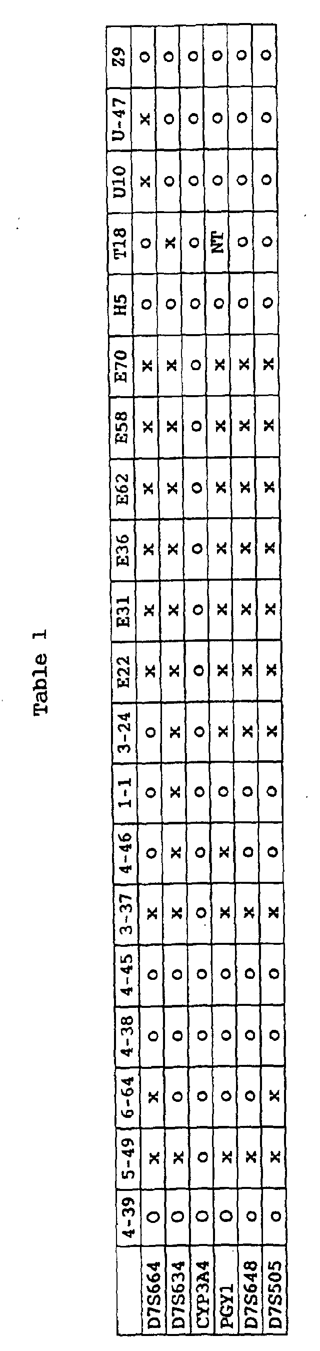

### Example 2 Introduction of human chromosome 7 fragment containing human CYP3A family gene, one molecular species of P450 gene into ES cell

Microcell fusion with a view to introducing human CYP3A family gene into ES cell was tried.

### (1) Cultivation of cells for preparing microcell

A9 cells for providing microcell were cultivated under the conditions described below. That is, mouse A9 cells (E22) having a human chromosome 7 marked with G418 resistant gene were cultivated in Dulbecco's modified Eagle medium (hereinafter, abbreviated as "DMEM", Nissui) to which 10% fetal calf serum (hereinafter abbreviated as "FBS", Sigma) and G418 (800 (µg/ml, Geneticin, Gibco) were added.

ES cells (TT2F, Lifetech Oriental) to be used for fusion with microcells had been cultivated by the procedure described below 1 week before the fusion was practiced. The microcell-fused ES cells (hereinafter, abbreviated as "MH cells" (microcell-hybrid ES) were handled according to the following contents. That is, the cultivation of ES cells was performed by use of 20% FBS (Gibco), 10 µM non-essential amino acid (Gibco), 10⁻⁴ M 2-mercaptoethanol (Sigma)- and 10³ U LIF (leucocyte growth inhibiting factor, Gibco)-added DMEM (hereinafter abbreviated as "ES use DMEM"). The ES cells were plated and cultivated in a 35 mm dish (Corning) in which feeder cells (Neo resistant primary culture cells, Lifetech Oriental) treated with mitomycin C (Sigma) had been pre-plated. The ES use DMEM was exchanged at intervals of 12 hours. Subculture was performed every three days when the cell density of ES cells reached 80% saturation as a guide. After sucking the ES use DMEM from the dish, the dish was washed with EDTA-PBS three times and then treated with 0.7 ml of 0.25% trypsin (Gibco) for 15 minutes. Then, ES use DMEM was added to the dish to terminate the reaction of trypsin and the cell suspension was recovered in a 15 ml tube (Falcon) by pipetting by use of Gilson Pipetteman (P-1000) and then centrifuged in a desktop centrifuge at 1,000 rpm for 5 minutes to obtain a pellet of cells. After sucking the supernatant, the cells were resuspended in a suitable amount of ES use DMEM and plated in a new dish that had been already pre-plated with feeder cells (Shinichi Aizawa, Biomanual Series 8 Gene Targeting, Youdosha, 1995).

### (2) Microcell fusion

Microcells were obtained in the following procedure. That is, E22 cells were cultivated until the cell density reached a state of 90% saturation in twenty four (24) 25 cm² flasks (Coaster 3025). The culture medium was exchanged for a culture medium containing colcemid (0.06 µg/ml, Demecolcin, Wako Pure Chemical Industry, Ltd.) and cultivation was performed for 2 days to form microcells. The culture medium was removed from the flasks and DMEM containing a cytochalasin B (10 µg/ml, Sigma) solution warmed at 37°C in advance was introduced to substantially fully fill the flasks. After mounting the flasks in an acrylic resin made vessel and pouring warm water (34°C) therein, the vessel was set in a rubber-made adapter and then centrifugation was performed at 34°C and at 8,000 rpm for 1 hour. After completion of centrifugation, the DMEM containing cytochalasin B was removed and the microcells accumulated on the bottom of the flasks were suspended in DMEM and recovered. The recovered microcell suspension was filtered through filters with membranes having pore diameters of 8 µm, 5 µm and 3 µm, respectively, in order to purify the microcell. The purified microcells were centrifuged at 1,500 rmp for 10 minutes and then resuspended in 5 ml of DMEM. Thereafter, the suspended cells were centrifuged again at 1,500 rpm for 5 minutes. The tubes after the centrifugation were stored at 4°C without removing the supernatant and provided for microcell fusion later on.

The microcell fusion was performed by the following procedure. That is, ES cells in two 60 mm dishes (Corning) were washed with EDTA-PBS three times and treated with trypsin to release the cells from the dishes. After suspending the cells in ES use DMEM, the suspension was recovered in a 15 ml tube and centrifuged at 1,500 rpm for 5 minutes to recover the cells and then the supernatant was sucked. After tapping the tube, the cells were washed with DMEM three times. After completion of washing, the cells were resuspended in 5 ml of DMEM. The suspension was gently overlaid on the solution of microcells previously recovered and centrifuged at 1,250 rpm for 5 minutes. After the centrifugation, the supernatant was sucked, and the cell mass was tapped to loosen them and left to stand for 1 minute. 0.5 ml of DMEM containing PEG1500 (polyethylene glycol, Wako Pure Chemical Industry, Ltd.) was added by flowing down on the inner wall of the tube and treated for 1.5 minutes. After 1.5 minutes from the initiation of the addition of PEG1500-containing DMEM, 9.5 ml of DMEM was slowly added to the tube to mildly suspend the cells. After completion of the suspending, the suspension was centrifuged at 1,250 rpm for 5 minutes and the supernatant was removed. The obtained cells were suspended in 6 ml of ES use DMEM and 2 ml aliquot of the suspension was added to three 100 mm dishes (Corning) that had already been plated with feeder cells and cultivation was performed with 10 ml of a culture medium per 100 mm dish. The cultivation was performed in ES use DMEM until 48 hours elapsed when ES cells were in a state of about 50% saturation. Thereafter, cultivation was performed in ES use DMEM to which G418 (Geneticin, Gibco) was added in an effective concentration of 150 µg/ml (hereinafter, abbreviated as "G418-added ES use DMEM"). In 8 to 10 days from the initiation of the cultivation, emergence of G418 resistance clone was observed. The emerged G418 resistant clone was isolated and plated onto 24-well plate (Corning) that had been already pre-plated with feeder cells, and then the scale of cultivation was increased by sequentially using a 12-well plate (Corning) and 35 mm dish that had been already pre-plated feeder cells, respectively.

### (3) PCR Analysis

As a result of selective cultivation, in total twenty nine (29) G418 resistant clones were obtained. From these clones genome DNA's were extracted by use of Puregene DNA Isolation Kit (Gentra System, Inc.) and provided for analysis by PCR. PCR was performed by use of the extracted genome DNAs as templates and primers prepared for the purpose of detecting CYP3A family genes (CYP3A4, CYP3A5, and CYP3A7). Thus, clones having a human chromosome 7 containing a CYP3A family gene was identified. As the primers for detecting CYP3A4, the above-mentioned primers of SEQ ID Nos. 1 and 2 were used. As the primer sequences for detecting CYP3A5 and CYP3A7, primers having the following sequences were used.

### (Primers for detecting CYP3A5)

Sense:      5'-ATAGAAGGGTCTGTCTGGCTGG-3' (SEQ ID No. 3)
Antisense   5'-TCAGCTGTGTGCTGTTGTTTGC-3' (SEQ ID No. 4)

### (Primers for detecting CYP3A7)

Sense:      5'-ACCCTGAAATGAAGACGGGC-3' (SEQ ID No. 5)
Antisense:  5'-GAGTTAATGGTGCTAACTGGGG-3' (SEQ ID No. 6)

The reaction mixture for PCR was prepared by adding sterilized distilled water to 3 µl of 10 x Ex Taq Buffer (Takara Shuzo Co., Ltd.), 4.8 µl of dNTP (dATP, dGTP, dCTP and dTTP, each 2.5 mM) (Takara Shuzo Co., Ltd.), 10 pmol of each primer, 100 ng of genome DNA, and 0.5 µl of Ex Taq (5U/ml) (Takara Shuzo Co., Ltd.) to make 30 µl, and dispensing the mixture in PCR use tubes (Perkin-Elmer). The manipulation of the whole procedure for preparing PCR reaction mixture was performed on ice. That is, a thermal cycler (GeneAmp PCR system 2400, Perkin-Elmer) was set to 85°C and after confirming that 85°C was reached, the tube was set. After performing pre-PCR consisting of a cycle of 85°C for 3 minutes, 94°C for 1 minute and 85°C for 1 minute, a cycle of 98°C for 10 seconds, 62°C for 30 seconds and 72°C for 1 minute was repeated 35 times. As a result, 25 clones from which the above-mentioned 3 types of human CYP3A family genes had been detected were observed. It was suggested that these clones were ES clones having a human chromosome 7 fragment containing CYP3A gene.

### Example 3 Preparation of chimeric mouse by use of ES cell harboring human chromosome 7 fragment containing a CYP3A family gene

A mouse harboring human chromosome 7 fragment containing a CYP3A family gene in somatic cells was prepared by use of human chromosome 7 fragment-harboring MH cells obtained by microcell fusion. The cultivation method for MH cells was the same as the cultivation method for ES cells described above but G418-added ES use DMEM was used for the maintenance and proliferation of MH cells.

### (1) Acquisition and cultivation of mouse embryo

The mouse embryo used for preparing a chimera was acquired from a MCH line (CLEA Japan) mouse. A 6 to 8 week MCH line female mouse was intraperitoneally injected with 7.5 IU of pregnant mare's serum gonadotropin (Serotropin, Teikoku Zoki) and after 48 hours it was further intraperitoneally injected with 7.5 IU of human chorionic gonadotropin (Puvelogen, Sankyo). The mouse was allowed to live together with a male mouse for one night for mating. 60 to 64 hours after the injection of human chorionic gonadotropin, the female mouse was sacrificed and 8-cell stage embryo was recovered from the oviduct by oviduct perfusion by use of M2 medium (Sigma). On a 60 mm dish (Corning) was dripped a droplet of 30 µl of ES use DMEM or M16 medium (Sigma) and further paraffin oil (Nakarai Tesk) was overlaid thereon. The recovered mouse embryo was sucked by a Pasteur pipette together with a small amount of M16 culture medium, the tip of the Pasteur pipette was introduced into the droplet on the dish and the mouse embryo sucked in therein was discharged. The mouse embryo in the dish was cultivated until it was needed under conditions 5% CO₂ and 37°C.

### (2) Preparation of a pipette for injecting a cell and a pipette for holding an embryo

The pipette for injecting a cell was prepared by setting a glass tube (Microcaps25, Dramont) in a microelectrode fabricating device (PN-3, Narishige), drawing the glass tube and working by use of a pincet having a sharp tip while observing the tip portion of the glass tube under a stereoscopic microscope (SMZ, Nikon). The pipette for holding an embryo was prepared by heating a glass tube (GDC-1, Narishige) by a small flame of a gas burner and drawing to make it thin, cuting it to a suitable length by use of an ample cutter, equipping the cut piece in a microforge (MF-90, Narishige), preparing the inner diameter of it to about 10 µm by use of a heating element after confirming that the outer diameter is about 150 µm and the cut surface is flat, and bending it at a point by a small flame of a gas burner.

### (3) Injection of MH cell into a mouse 8-cell stage embryo

The MH cells provided for preparing a chimera were treated with 0.25% trypsin (Gibco) and recovered by centrifugation at 1,000 rpm for 5 mintues and then suspended in ES use DMEM. The cell suspension was dipped on a 100 mm dish (Corning) in an amount of about 30 µl and paraffin oil (Nakarai Tesk) was overlaid thereon. This was placed stationary on the stage of an inverted microscope equipped with micro manipulators (MMW-202 and MMW-204, Narishige). A glass-made pipette for holding a mouse embryo was attached to a micro injector (IM-5, Narishige) whose inside was filled with sterilized distilled water and a glass-made pipette for injecting a cell was attached to a micro injector (IM-6, Narishige) whose inside was filled with air. Adjustment was made so that the spot of MH cell suspension was positioned in the center of visual field of the inverted microscope, and the glass-made pipette for injecting a cell was inserted in the spot and the MH cells were sucked. After acquisition of the eggs, 20 to 30 mouse 8-cell stage embryos being cultivated were transferred into the spot by use of a Pasteur pipette. A mouse embryo was retained and focused. Then, a pipette for injection was manipulated so as to penetrate a zona pellucida and injected 10 to 15 MH cells in perivitelline cavity. The manipulated embryos by repeating the above-mentioned manipulation were transferred in ES use DMEM or M16 medium placed on the 60 mm dish in the form of a droplet on which paraffin oil layer was overlaied. The embryos were cultivated until they developed to blastocysts and transplanted on the day next.

### (4) Transplantation of embryo

8-week or more MCH line female mouse (CLEA Japan) was allowed to live together with a vasectomized male mouse of the same line for one night for mating. On the day next, the female of which vaginal plug was observed was judged as a pseudopregnant femal mouse and used as a foster mother. By counting the day next of the mating is first day of pregnancy, the mouse on the fourth day of pregnancy was subjected to embryo transplantation : For anesthetization, Avertin prepared by dissolving tribromoethanolamine (Aldrich) in amyl alcohol (Aldrich) and diluting the solution with physiological saline to a concentration of 2.5% was used. Avertin was intraperitoneally injected depneding on the body weight of mouse to anesthetize it. The hair on the back of the mouse was cut and the back was sterilized with alcohol cotton and then the integument was incised along the spine and inserting scissors between the muscular coat to ablate them. The muscular coat covering the ovary was incised and the adipose tissue was pinched with a pincette and the ovary, oviduct and an upper part of uterine horn were taken out onto sterilized filter paper. The pipette for transplantation was heated by use of a gas burner and one point thereof was bent and processed such that the inner diameter thereof was slightly greater than a single embryo.

Among the cultivated embryos, morphologicall normal blastocysts or morulas were washed with ES use DMEM (or M2 medium) and then sucked in a pipette for transplantation together with a small amount of ES use DMEM medium. The suction was performed as follows. That is, ES use DMEM medium was sucked into a pipette by utilizing capillarity and then air was introduced therein to form one or two small bubbles in the pipette. Then, about 10 embryos to be transplansted were sucked together with ES use DMEM medium such that there was no gap betwen the embryos and finally one small bubble of air was introduced in the pipette and the tip portion was filled with a small amount of ES use DMEM medium. Circumventing large blood vessels, the upper part of uterine horn was punctured with a 26G needle (Thermo), and the tip of pipette was inserted through the resulting hole and the embryo was gently injected. After transportation into right and left uterine horn was completed, the muscular coat was sutured and the integument was clipped with an autoclip (Falcon).

### (5) PCR Analysis

The foster mother gave birth to offsprings 16 to 17 days after the transplantation of embryos and were allowed to breed them until weaning after 3 weeks from the birth. Those foster mothers whose pregnancy was confirmed but who did not undergo delivery were subjected to cesarian section within 1 day after passage of the expected term of acouchement to take out offsprings and the offsprings were bred by another female mouse during breeding as foster children. The offsprings weaned 3 weeks after their birth were placed in different cages according to the sex and their ears were punched for identify individuals. For the mice after identification of individuals, the tail was cut to a length of about 1 to 1.5 cm and further sliced with surgical scissors. The obtained samples were transferred into 15 ml tubes and genome DNAs were extracted by use of Puregene DNA Isolation Kit (Gentra System, Inc.) and PCR was performed using them as templates.

By performing PCR by use of primers prepared for the purpose of detecting CYP3A family genes (CYP3A4, CYP3A5. and CYP3A7), mouse individuals having human chromosome 7s containing these CYP3A family genes were identified. As the primer sequences for detecting CYP3A4, SEQ ID Nos. 1 and 2 were used, as the primer sequences for detecting CYP3A5, SEQ ID Nos. 3 and 4 were used and as the primer sequences for detecting CYP3A7, SEQ ID Nos. 5 and 6 were used.

The reaction mixture for PCR was prepared by adding sterilized distilled water to 3 µl of 10 x Ex Taq Buffer (Takara Shuzo Co., Ltd.), 4.8 µl of dNTP (dATP, dGTP, dCTP and dTTP, each 2.5 mM) (Takara Shuzo Co., Ltd.), 10 pmol of each primer, 100 ng of genome DNA, and 0.5 µl of Ex Taq (5U/ml) (Takara Shuzo Co., Ltd.) to make 30 µl, and dispensing the mixture in PCR use tubes (Perkin-Elmer). The manipulation of the whole procedure for preparing PCR reaction mixture was performed on ice. That is, a thermal cycler (GeneAmp PCR system 2400; Perkin-Elmer) was set to 85°C and after confirming that 85°C was reached, the tube was set. After performing pre-PCR consisting of a cycle of 85°C for 3 minutes, 94°C for 1 minute and 85°C for 1 minute, a reaction of the cycle of 98°C for 10 seconds, 62°C for 30 seconds and 72°C for 1 minute was repeated 35 times. As a result, mouse individuals from which any one of the above-mentioned 3 types of human CYP3A family genes had been detected were observed. These mice were considered to be chimeric mice having a human chromosome 7 fragment containing CYP3A family gene.

### Example 4 Assay of expression of CYP3A4 gene in a chimeric mouse harboring human chromosome 7 fragment containing CYP3A family gene

Out of the prepared mice, an individual that was indicated by PCR analysis to be a chimeric mouse harboring a human chromosome 7 fragment containing CYP3A family gene was subjected to analysis by RT-PCR in order to confirm if the introduced CYP3A4 gene was expressed on the level of individual.

To simultaneously study the influence of rifampicin (Sigma) known as a substance for inducing the expression of CYP3A4 on the expression of CYP3A4 gene in a chimeric mouse, the mouse from which RNA was to be extracted were set as shown in Table 2. Note that the solution to be administered was prepared by suspending rifampicin in corn oil (Sigma). The administration amount each time was set to 100 mg/kg.

**Table 2**

| Specimen | Treating method |
|---|---|
| Chimeric mouse | 100 mg/kg of Rifampicin was intraperitomeally administered for 4 days |
| Chimeric mouse | Medium (corn oil) was intraperitoneally administered for 4 days |
| MCH line mouse | 100 mg/kg of Rifampicin was intraperitomeally administered for 4 days |
| MCH line mouse | Medium (corn oil) was intraperitoneally administered for 4 days |

From these mice, various organs, i.e., liver, small intestine, kidney, spleen, lung and heart were extracted and rapidly refrigerated by use of liquid nitrogen and then ruptured, followed by extraction of total RNA by ISOGEN (Nippon Gene). The extracted RNA was dissolved in a sterilized water containing no RNase (RNase free water) and then stored at -80°C. A portion of it was used for cDNA synthesis as needed.

Then, cDNA was prepared from a portion of RNA solution of each organ. First, in order to remove genomic DNA contaminating in the RNA solution, treatment with DNase was performed by charging in a 1.5 ml tube 1 to 5 µg of RNA, 1 µl of DnaseI (Gibco), 1 µl of 10 x DNaseI reaction buffer (Gibco), and RNase free water to make a total amount 10 µl and incubating the mixture at room temperature for 15 minutes. To this reaction mixture was added 1 µl of 25 mM EDTA to terminate the reacton of Dnase and further incubated at 65°C fo 10 minutes.

Then, cDNA synthesis was performed by use of Super Script™ Preamplification System (Gibco). That is, 1 µl of Randam hexamers (Gibco) was added to the Dnase-treated RNA solution and after 10 minutes' incubation at 70°C, the tube was transferred on ice. To the tube were added 2 µl of 10 x PCR buffer (Gibco), 2 µl of 25 mM MgCl₂, 1 µl of dNTP (dATP, dGTP, dCTP and dTTP, each 1OmM and 2 µl of 0.1M DTT. After mild mixing, the mixture was incubated at 25°C for 5 minutes. To this was added 1 µl of reverse transcriptase (Super ScriptII RT, Gibco) and incubated at 25°C for 10 minutes, at 42°C for 50 minutes and at 70°C for 15 minutes in order and then the tube was transferred on ice. Further, 1 µl of E. coli RNaseH (Gibco) was added and the mixture was incubated at 37°C for 20 minutes to remove RNA that remained in the solution.

Then, PCR was performed by use of the synthesized cDNA as a template. Using the primers prepared for the purpose of detecting cDNA of CYP3A4, expression of gene in each organ was studied. As the primer sequence, the sequence described below was used.

### (Primers sequence for detecting CYP3A cDNA)

Sense:     5'-CAAGACCCCTTTGTGGAAAA-3' (SEQ ID No. 7)

Antisense  5'-TCTGAGCGTTTCATTCACCA-3' (SEQ ID No. 8)

The reaction mixture for PCR was prepared by adding sterilized distilled water to 3 µl of 10 x Ex Taq Buffer (Takara Shuzo Co., Ltd.), 4.8 µl of dNTP (dATP, dGTP, dCTP and dTTP, each 2.5 mM) (Takara Shuzo Co., Ltd.), 10 pmol of each primer, 100 ng of genome DNA, and 0.5 µl of Ex Taq (5 U/ml) (Takara Shuzo Co., Ltd.) to make 30 µl, and dispensing the mixture in PCR use tubes (Perkin Elmer). The manipulation of the whole procedure for preparing PCR reaction mixture was performed on ice. Then, a thermal cycler (GeneAmp PCR system 2400, Perkin Elmer) was set to 94°C and after confirming that 95°C was reached, the tube was set. After performing pre-PCR at 94°C for 5 minutes, reaction of 35 cycles was performed, with one cycle consisting of 94°C for 30 seconds, 60°C for 1 minute and 72°C for 1 minute, followed by further teatment at 72°C for 15 minutes. A sample of liver from each mouse was electrophoresed to examine whether or not expression of CYP3A4 occurred. As a result, expression of CYP3A4 was observed in the liver of rifampicin-administered chimeric mouse.

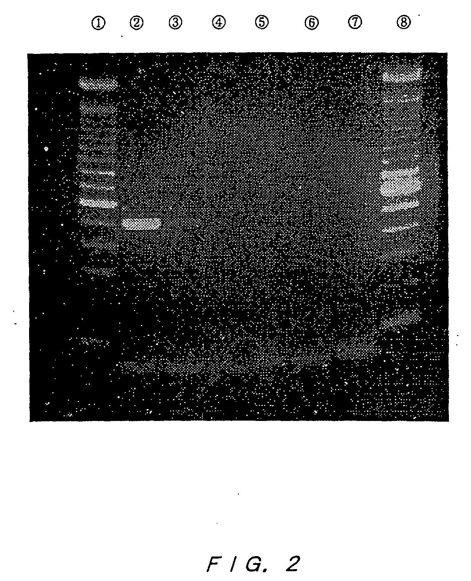

On the fear that the above-mentioned results were obtained by erroneous amplification of cDNA derived from mRNA of another gene such as mouse endogenous Cyp gene, the sample of rifampicin-administered chimeric mouse was treated with restriction enzyme *Eco*RI. If human CYP3A4 product was amplified, one site must have been cleaved by *Eco*RI and as expected two bands were obtained near 250 bp (Fig. 1). [2] in the figure indicates results of *Eco*RI treatment of the PCR product derived from the liver of a rifampicin-administered mouse, [3] indicates results of *Eco*RI treatment of the PCR product of RNA derived from the liver of a human, [4] indicates the PCR product derived from the liver of a rifampicin-administered mouse, and [5] indicates the PCR product of RNA derived from the liver of a human. Furthermore, as a result of analysis of the gene sequence of this PCR product, it was confirmed that it was identical with a part of the sequence of human CYP3A4 gene. For other organs, expression of CYP3A4 was examined (Fig. 2). As a result, expressionof human CYP3A4 gene was observed in the liver ([1] in the figure) and small intestine ([2] in the figure) of rifampicin-administered chimeric mouse.

### Example 5 Preparation of a TC (Trans-ChromoHomic) mouse harboring human chromosome 7 fragment- containing CYP3A family genes

By use of the prepared chimeric mouse as a founder, preparation of progeny mouse (TC mouse) having a human chromosome 7 fragment was contemplated.

As the founder chimeric mice, 100% chimera (individuals showing agouti color in a 100% portion of the body surface) mice were used in which human CYP3A4, 3A5, and 3A7, respectively, were confirmed. Since MH cells used were of TT2F origin, all the 100% chimeric mice showed female phenotypes. These were allowed to live together with MCH line male mice for mating and offsprings were obtained. For a litter of eleven (11) F1 generation offsprings thus obtained, the tail was cut to a length of about 1 to 1.5 cm and further sliced with surgical scissors. The obtained samples were transferred into 15 ml tubes and genome DNAs were extracted by use of Puregene DNA Isolation Kit (Gentra System, Inc.) and PCR was performed using them as templates. By use of primers prepared for the purpose of detecting CYP3A family genes (CYP3A4 and CYP3A5) TC mouse individual having a human chromosome 7 was identified.

### (Primers for detecting CYP3A4)

Sense:      5'-GCCATAGAGACAAGGGCAAG-3' (SEQ ID No. 9)
Antisense   5'-TCAGTGAGGCTGTTGGATTG-3' (SEQ ID No. 10)

### (Primers for detecting CYP3A5)

Sense:      5'-CTGGTCACCCACCATGTGTA-3' (SEQ ID No. 11)
Antisense   5'-TATCCTCTCACCTGCCCTTG-3' (SEQ ID No. 12)

The reaction mixture for PCR was prepared by adding sterilized distilled water to 3 µl of 10 x Ex Taq Buffer (Takara Shuzo Co., Ltd.), 4.8 µl of dNTP (dATP, dGTP, dCTP and dTTP, each 2.5 mM) (Takara Shuzo Co., Ltd.), 10 pmol of each primer, 100 ng of genome DNA, and 0.5 µl of Ex Taq (5U/ml) (Takara Shuzo Co., Ltd.) to make 30 µl, and dispensing the mixture in PCR use tubes (Perkin-Elmer). The manipulation of the whole procedure for preparing PCR reaction mixture was performed on ice. That is, a thermal cycler (GeneAmp PCR system 2400, Perkin-Elmer) was set to 85°C and after confirming that 85°C was reached, the tube was set. After performing pre-PCR consisting of a cycle of 85°C for 3 minutes, 94°C for 1 minute and 85°C for 1 minute, a cycle of 94°C for 30 seconds, 62°C for 45 seconds and 72°C for 1 minute was repeated 40 times. After that, a cycle of 72°C for 20 minutes was performed. As a result, 5 individuals were detected for CYP3A4 and 1 individual was detected for CYP3A5. From the results described above, one individual in which both of CYP3A4 and 3A5 were detected was judged to be a TC mouse harboring human chromosome 7 fragment containing CYP3A family genes.

### Example 6 Preparation of a physical map of mouse endogenous Cyp3a gene cluster

### (1) Isolation of BAC clones containing Cyp3a11 13, 16, and 25

From the BAC library (Kurabo) derived from a mouse (C57BL/6. 129/Sv), isolation of a BAC clone containing Cyp3a11 13, 16 or 25 was performed by PCR and hybridization.

As the PCR primers for detecting respective genes of Cyp3a11 13, 16 and 25, the following sequences were used.

### (Primers for detecting Cyp3a11

Sense:      5'-GACACAATTGTCAAGGATGG-3' (SEQ ID No. 13)
Antisense   5'-GCTGACAAACAAGCAGGGAT-3' (SEQ ID No. 14)

### (Primers for detecting Cyp3a13)

Sense:     5'-ATGTACCTGCCCTTTGGGAG-3' (SEQ ID No. 15)
Antisense   5'-GGAGTGGGTTTTCTGGTTGA-3' (SEQ ID No. 16)

### (Primers for detecting Cyp3a16)

Sense:      5'-TGGTCTCCCATATCTCAGGC-3' (SEQ ID No. 17)
Antisense   5'-CAATGTACCAGTGAGAGCGC-3' (SEQ ID No. 18)

### (Primers 1 for detecting Cyp3a25)

Sense:     5'-AGAAGAAGAGAAAGGGGAGCC-3' (SEQ ID No. 19)
Antisense   5'-CATTCACCACCATGTCCAGG-3' (SEQ ID No. 20)

### (Primers 2 for detecting Cyp3a25)

Sense:      5'-AAACCAGAAGAACCGAGTGG-3' (SEQ ID No. 21)
Antisense   5'-GTGACAGGTGCCTTATTGGG-3' (SEQ ID No. 22)

Furthermore, in the case of Cyp3a11 13 and 16, PCR was performed by use of a genome DNA derived from C57BL/6 mouse as a template and the product was used as probe for hybridization. As for Cyp3a25, PCR was performed by use of Mouse liver QUICK-Clone cDNA (Clonetech) as a template and the product was used as probe for hybridization. In PCR, GeneAmp 9600 produced by Perkin-Elmer, Inc. was used as a thermal cycler, Ex Taq (Takara Shuzo Co., Ltd.) was used as a Taq polymerase, and as the buffer and dNTP (dATP, dGTP, dCTP and dTTP) those attached to Ex Taq (Takara Shuzo Co., Ltd.) were used under the recommended conditions. The cycle condition of PCR was such that after thermal denaturation at 94°C for 1 minute, the cycle of 98°C for 10 seconds, 65°C for 30 seconds, and 72°C for 1 minute was performed 35 times (all the PCRs hereinafter referred to were also performed under the conditions unless otherwise indicated specifically).

Screening of actual mouse BAC library (Kurabo) was performed by contract screening services by Kurabo and the following results were obtained.

**Table 3**

| Gene Gene | Screening method | Origin of BAC | Number of positive clones |
|---|---|---|---|
| Cyp3a11 | PCR | 129/Sv | 1 Clone |
| | Hybridization | C57BL/6 | 3 Clones |
| Cyp3a13 | PCR | 129/Sv | 1 Clone |
| | Hybridization | C57BL/6 | 2 Clones |
| Cyp3a16 | PCR | 129/Sv | 3 Clones |
| | Hybridization | C57BL/6 | 8 Clones |
| Cyp3a25 | Hybridization | C57BL/6 | 2 Clones |

### (2) Preparation of contigs of BAC clones containing Cyp3a11, 13, 16 and 25

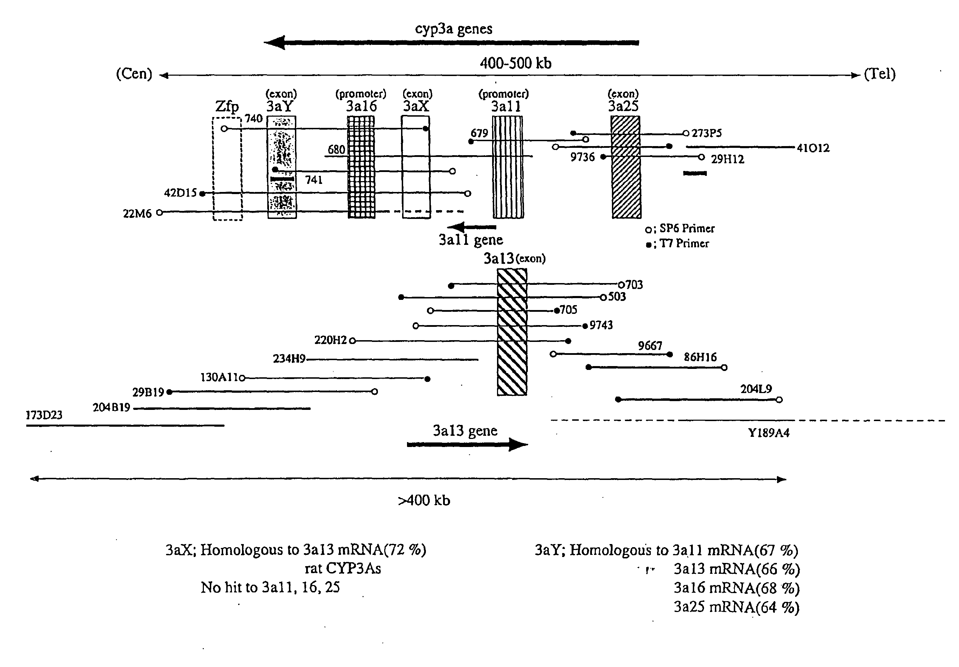

From the above-mentioned positive BAC clone (clone containing any one of Cyp3a11 13, 16 and 25) was extracted BAC DNA by use of a plasmid autoextracting apparatus (Kurabo) and spotted on Hybond-N+ membrfane (Amersham) and dot hybridization was performed by use of probes capable of specifically detecting Cyp3a11, 13, 16 and 25 (one used in screening of the BAC library) (preparation of probe and conditions being in accordance with Ausubel et al., Current Protocols in Molecular Biology, John Wiley & Sons, Inc., 1994). The results obtained are shown in Fig. 3 (not all the BAC clones but only typical ones being shown).

There existed several BAC clones that were positive in both the Cyp3a11 and 16 probes (for example, clone 680). As a result of sequence analysis performed on these clones by use of primers of SEQ ID Nos. 13 and 17 (outsourced to Toray Research Center), it was elucidated that surely both the sequences Cyp3a11 and 16 existed in the same BAC clone. This indicates that Cyp3a11 and 16 exist adjacent to each other at a distance within 100 kb. Then, in order to examine in what positional relation the BAC clone containing the 3a11-3a16 is with respect to the BAC clone containing 3a13 and 3a25, terminal sequencing of BAC clone containing 3a11 and 16 only (for example, clones 679 and 740, respectively) was performed (outsourced to Toray Research Center). In the Bac vector, sequences of T7 promoter and SP6 promoter exist at both ends of cloning site of the insert DNA and by use of them as primers, sequencing of the both terminals becomes possible. From the sequence on the SP6 side of clone 679, the following primers were designed and PCR was performed on the remaining BAC clones.

### (Primers on the SP6 side of clone 679)

Sense:      5'-TAGGGTGAAGACATGGTGGC-3' (SEQ ID No. 23)
Antisense:  5'-GGAGGAGAGGGAGATGTTGAT-3' (SEQ ID No. 24)

As a result, clones 9736 and 273P5 containing 3a25 were positive. As described above, as a result of sequencing of clones 9736 and 273P5 with the primer of SEQ ID No. 23 (outsourced to Toray Research Center), it was elucidated that surely clone 679 was linked to clones 9736 and 273P5. From this it was considered that Cyp3a genes were present on the chromosome in the order of 3a16-3a11-3a25 On the other hand, sequencing of BAC clone 740 conaining only 3a16 by use of SP6 primer was performed (outsourced to Toray Research Center) and homology search was performed. As a result, several zinc finger genes were hit. Actually translating the DNA sequence into amino acid sequence on a computer indicated the existence of an amino acid sequence which could be considered to be a C2H2 type zinc finger motif (for example, Kuroiwa et al., Nature Genet., 12:186-190, 1996). From this there is the possibility that zinc finger gene (called "Zfp") is present outside the 3a16-3a11-3a25 cluster (Zfp-3a16-3a11-3a25).

Also, as a result of sequencing of BAC clone 741 containing only 3a16 with T7 primer (outsourced to Toray Research Center) and performing homology search, it revealed that there is a sequence that shows a homology with 3a11, 13, 16, and 25 but is not identical with any of 4 genes. This suggests the existence of novel Cyp3a gene, which will be called tentatively as "Cyp3aY". Since BAC clone 41 contains only 3a16 and 3aY is present on its terminal, it is suggested that the genes are present on the chromosome in the order of Zfp-3aY-3a16-3a11-3a25 and 3aY is considered to be one end of the cluster. Furthermore, as a result of sequencing of BAC clone 740 with T7 primer was perfomed (outsourced to Toray Research Center) and performing homology search, it revealed that there is a sequence that shows high homology with the Cyp3A gene of rat but shows a weak homology with mouse 3a13 only. This also suggests the existence of a novel Cyp3a gene, which will be tentatively called as " Cyp3aX". Since BAC clone 740 contains only 3a16 and 3aX is present on its terminal, it is suggested that the genes are present on the chromosome in the order of Zfp-3aY-3a16-3aX-3a11-3a25.

Then, in order to study the possibility that 3a25 is the remaining end of the cluster, terminal sequencing of BAC clone 9736 containing 3a25 with T7 primer and SP6 primer was performed (outsourced to Toray Research Center). Based on this sequence, the following STS primesr were designed.

### (Primers on the T7 side of clone 9736)

Sense:      5'-AGATGTGTGGCCTTGTTAAGG-3' (SEQ ID No. 25)
Antisense:  5'-TTTCTGGATGTCACTGGTCC-3' (SEQ ID No. 26)

By use of these primers, PCR of the BAC clone in hand (BAC clones containing any one of Cyp3a11, 13; 16 and 25) was performed. As a result, it revealed that the T7 side was located outside of the cluster. Accordingly, further screening of mouse BAC library with these primers was performed. As a result, clone 29H12 was obtained. Similarly, terminal sequencing was performed and the following STS primers were designed.

### (Primers on the SP6 side of clone 29H12)

Sense:     5'-TAGAAGCCCAACTTTGGGAC-3' (SEQ ID No. 27)
Antisense:  5'-AGCCAACAAGAGGGTGTACG-3' (SEQ ID No. 28)

By use of these primers, PCR of the BAC clone in hand was performed. As a result, it revealed that the SP6 side is located outside of the cluster. Accordingly, further screening of mouse BAC library with these primers was performed. As a result, clone 41012 was obtained. Here, as described in the section of "Best Mode for carrying out the Invention", in order to determine the terminal of the cluster, all the obtained BAC clones (BAC clones containing any one of Cyp3a11, 13, 16 and 25 and BAC clones adjacent thereto) were subjected to hybridization under mild conditions by use of a cDNA probe close to the full-length of 3a25. The cDNA probe was prepared by performing PCR by use of the following primers and of Mouse liver QUICK-Clone cDNA (Clonetech) as a template.

### (Primers 3 for Cyp3a25)

Sense:      5'-GGCCTGAACTGCTAAAGGAAAGCTA-3' (SEQ ID No.
29)
Antisense:  5'-GGTGAACATCCACAACTACCATTGAG-3' (SEQ ID No.
30)

The hybridization was performed under the condition of 60°C and the washing was the same as described above except that it was performed at 60°C in the presence of 2 x SSC, 0.1% SDS. As a result, it revealed that only clone 41012 was negative and hence it was considered that the cluster was completed up to clone 29H12, and therefore concluded with 3aY-3a16-3aX-3a11-3a25

### (3) Preparation of BAC contig in the vicinity of Cyp3a13

BAC clones containing Cyp3a13 (clones 703, 503, and 705) did not ligate to any of BAC clones containing Cyp3a11, 16, and 25 within the range of the above-mentioned screening. Accordingly, chromosome walking as desribed above using clone 703 as a starting material was performed. First, terminal sequencing of clone 703 was performed (outsourced to Toray Research Center) and the following primers were designed.

### (Primers on the SP6 side for clone 703)

Sense:     5'-ACAGGGAGTAGGTGACACTGTTG-3' (SEQ ID No. 31)
Antisense: 5'-GGGTTGTTGGACTCACAGAGAG-3'  (SEQ ID No. 32)

### (T7 Primers for clone 703)

Sense:     5'-GACCTCACACTGGCAAGAAGTC-3' (SEQ ID No. 33)
Antisense:  5'-ATGGTGCTCAGCAATAGTAGGG-3' (SEQ ID No. 34)

By performing screening of BAC library by use of these primers, chromosome walking in the both directions toward SP6 side and T7 side of clone 703 can be performed. First, screening by use of 703/T7 primers was performed. As a result, clones 220H2 and 234H9 were obtained. Terminal sequencing of these clones was performed (outsourced to Toray Research Center) and the following primers were designed.

### (Primers on the T7 side for clone 220H2)

Sense:     5'-GCAGGATACCAGATATGTGGC-3' (SEQ ID No. 35)
Antisense:  5'-AAACCCCTTAAACCCACAGC-3' (SEQ ID No. 36)

### (Primers on the SP6 side for clone 220H2)

Sense:     5'-GGTGTCACACGCCTTTCATC-3' (SEQ ID No. 37)
Antisense:  5'-CTGCTCAAAGGAGAACAGGG-3' (SEQ ID No. 38)

By use of the primers, PCR of the BAC clone in hand (BAC clones containing any one of Cyp3a11, 13, 16 and 25 and BAC clones adjacent thereto) was performed. As a result, it revealed that the SP6 side was located outside of the cluster. Accordingly, further screening of the BAC library with these primers was performed. As a result, clones 130A11 and 29B19 were obtained. Then, terminal sequencing of these clones was performed and the following primers were designed.

### (Primers on the T7 side for clone 130A11)

Sense:        5'-CCACACATTCTGCCAATCAG-3' (SEQ ID No. 39)

Antisense: 5'-AGTACAGCACCGGGCTAAAG-3' (SEQ ID No. 40)

### (Primers on the SP6 side for clone 130A11)

Sense:     5'-GCAACAATAACCCAACTGCC-3' (SEQ ID No. 41)
Antisense: 5'-CAGGTGGTAATAAGCTGCCC-3' (SEQ ID No. `42)

### (Primers on the T7 side for clone 29B19)

Sense:     5'-GCATGAAGCACTGTCTCGTC-3' (SEQ ID No. 43)
Antisense:  5'-GGGGTCCTCCTTTCAATATCC-3' (SEQ ID No. 44)

### (Primers on the SP6 side for clone 29B19)

Sense:      5'-TCTCTGCCTCTCATCTTAGCC-3' (SEQ ID No. 45)
Antisense:  5'-TGTGTGTGGGTTGGTGTCTC-3'  (SEQ ID No. 46)

By use of these primers, PCR of the BAC clone in hand (BAC clones containing any one of Cyp3a11, 13, 16 and 25 and BAC clones adjacent thereto) was performed. As a result, it revealed that the T7 side of clone 29B19 was located outside of the cluster. Accordingly, further screening of the BAC library with these primers was performed. As a result, clones 204B19 and 173D23 were obtained. Then terminal sequencing of these clones was performed and the following primers were designed. (Primers on the SP6 side for clone 173D23)

Sense:     5'-CGGCTCTCACAAATAGACCC-3' (SEQ ID No. 47)
Antisense:  5'-CCAGGTTATCGCTGGTCTTC-3' (SEQ ID No. 48)

By use of these primers, PCR of the BAC clone in hand (BAC clones containing any one of Cyp3a11, 13, 16 and 25 and BAC clones adjacent thereto) was performed. As a result, it revealed that the SP6 side of clone 173D23 was located outside of the cluster.

On the other hand, as a result of screening of the BAC library with 703/SP6 primers, clone 86H16 was obtained. Then terminal sequencing of the clone was performed and the following primers were designed.

### (Primers on the SP6 side for clone 86H16)

Sense:     5'-TCACGACCTTATGCAAAGACC-3' (SEQ ID No. 49)
Antisense:  5'-CGGGAAGTATTGCTTTCAGC-3' (SEQ ID No. 50)

### (Primers on the T7 side for clone 86H16)

Sense:      5'-TCAGTCGTTACCAGCCTTTTG-3' (SEQ ID No. 51)

Antisense:  5'-GAGTGGGCTCTGGTCATTTC-3' (SEQ ID No. 52)

By use of the primers, PCR of the BAC clone in hand (BAC clones containing any one of Cyp3a11, 13, 16 and 25 and BAC clones adjacent thereto) was performed. As a result, it revealed that the SP6 side of clone 86H16 was located outside of the cluster. Accordingly, further screening of the BAC library with these primers was performed. As a result, clone 204L9 was obtained. Then terminal sequencing of the clone was performed (committed to TORAY RESEARCH CENTER. Inc.) and the following primers were designed.

### (Primers on the SP6 side for clone 204L9)

Sense:      5'-TCTCAGCATAGTTCAGGGGC-3' (SEQ ID No. 53)
Antisense:  5'-TTTCTTTCAGCTCTGGGTGG-3' (SEQ ID No. 54)

By use of the primers, PCR of the BAC clone in hand (BAC clones containing any one of Cyp3a11, 13, 16 and 25 and BAC clones adjacent thereto) was performed. As a result, it revealed that the SP6 side of clone 204L9 was located outside of the cluster. Accordingly, further screening of the BAC library with these primers was performed. However, no positive clone other than 204L9 itself was present in this library. In this stage, the screening was temporariry interrupted and hybridization of all the obtained BAC clones (contig of BAC clones containing 3a13) was performed by use of a cDNA probe of near full-length of 3a25 as described above under mild conditions. As a result, clones turned positive included 703, 503, 705, 9743, 220H2, 234H9, and 130A11 while the BAC clones 29B19, 204B19, 173D23, 9667, 86H16, and 204L9 existing outer than the positive clones were negative. This suggests that there is no other novel Cyp3a gene in the vicinity of 3a13 and 3a13 is present on the chromosome as isolated from the above-mentioned cluster 3aY-3a16-3aX-3a11-3a25.

Fig. 3 provides a summary of the experimental results described above. Red BAC clone indicates a clone that was positive with a cDNA probe of near full-length 3a25 and black BAC clone indicates a clone that was negative therewith.

### Example 7 Preparation of a targeting vector For deleting mouse Cyp3a gene cluster 3aY-3a16-3aX-3a11-3a25

### (1) Preparation of cassette vectors ploxPUP and ploxPDOWN

Cassette vectors ploxPUP and ploxPDOWN for inserting loxP at each end of Cyp3a gene cluster were prepared as follows. First, preparation of ploxPUP will be explained. Plasmid pBluescript II SK(-) (Toyobo) was cleaved with restriction enzyme *Eco*RV (Boehringer) and reacted with dephosphorylase CIAP (calf small intestine-derived alkaline dephosphorylase, Takara Shuzo Co., Ltd.) at 50°C for 30 minutes to dephosphorylate the cleaved ends. To this was ligated a DNA fraction cleaved from plasmid pBS302 (Gibco) with restriction enzymes *Spe*I and *Hin*dIII (Boehringer) and blunted with a DNA Blunting kit (Takara Shuzo Co., Ltd.) by use of a DNA Ligation kit (Takara Shuzo Co., Ltd.) to transform a competent cell DH5 (Toyobo) of Escherichia coli (plasmid pBS302HS). Blunting, ligation and transformation were performed according to the protocols attached to the respective kits.

Then, a DNA fragment cleaved from a plasmid pSTneo (Katoh et al., Cell Struct. Funct., 12:575, 1987) with a restriction enzyme *Xho*I (Boehringer) and blunted with a DNA Blunting kit (Takara Shuzo Co., Ltd.) was cloned to the SmaI site of plasmid pBS302HS (dephosphorylated in the same manner as described above) by use of a DNA Ligation kit (Takara Shuzo Co., Ltd.) to obtain a plasmid ploxPneo. Plasmid pMC1DT-A (Gibco) was cleaved with restriction enzyme ApaI (Boehringer), blunted, and dephosphorylated and a phosphorylating *Not*I linker (Takara Shuzo Co., Ltd.) was inserted thereto to obtain a plasmid pMC1DT-AN.

In an analogous fashion, *Spe*I linker (Takara Shuzo Co., Ltd.) was inserted to the *Sal*I site of plasmid pMC1DT-AN and a DNA fragment cleaved from this plasmid with restriction enzymes *Spe*I and *Not*I (Boehringer) was cloned to plasmid ploxPneo cleaved with *Spe*I and *Not*I*.* Finally, this plasmid was cleaved with restriction enzyme *Sal*I (Boehringer), dephosphorylated and cloned to a DNA fragment cleaved from plasmid pGKPuro (endowed from Dr. Peter W. Laird of WHITEHEAD INSTITUTE) with restriction enzymes *Sal*I and *Xho*I (Boehringer) to obtain a plasmid ploxPUP.

Next, preparation of a plasmid ploxPDOWN will be explained. First, an *Fse*I linker was inserted to the SacI site of plasmid pBluescript II SK(-) (Toyobo) in the manner as described above. The *Fse*I linker was obtained by synthesizing an oligo DNA having the following sequence and phosphorylating the 5'-end thereof (synthesis being outsourced to Griner).

### (FseI Linker)

### 5'-pGGCCGGCC-3'

This plasmid was cleaved with restriction enzyme *Bam*HI (Boehringer), dephosphorylated and cloned to a DNA fragment cleaved from plasmid pBS302 (Gibco) with respriction enzyme *Bam*HI (Boehringer). The obtained plasmid was cleaved with restriction enzymes *Sal*I and *Kpn*I (Boehringer) and cloned to a DNA fragment cleaved from plasmid pMC1DT-A (Gibco) with restriction enzymes *Sal*I and *Kpn*I (Boehringer) (plasmid ploxPDT-A). Then, to the *Cla*I site of plasmid pGREEN LANTERN-1 (Gibco) was inserted an *Spe*I linker (Takara Shuzo Co., Ltd.) as described above and a DNA fragment was cleaved with restriction enzyme *Spe*I (Boehringer). This was cloned to plasmid ploxPDT-A that had been cleaved with *Spe*I and then dephosphorylated. Finally, this plasmid was cleaved with restriction enzyme SmaI (Boehringer), dephosphorylated and cloned to a DNA fragment that had been cleaved from plasmid pGKPuro with restriction enzyme *Sal*I (Boehringer) and blunted (plasmid ploxPDOWN).

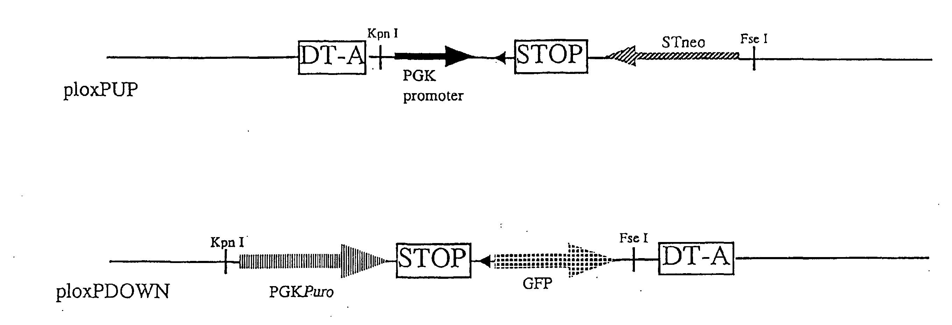

The structures of cassette vectors ploxPUP and ploxPDOWN constructed as described above are shown in Fig. 4.

### (2) Cloning of genome DNA at both ends of mouse Cyp3a gene cluster

Cloning of Cyp3aY gene region corresponding to one end of Cyp3a gene cluster will be described hereinbelow. As shown in Fig. 4, the genome region corresponding to the outermost of the Cyp3aY side of the cluster was T7 terminal region of BAC clone 741 and hence cloning of this region was performed. When cleaving BAC clone 741 with restriction enzyme *Xba*I (Boehringer), an about 13 kb DNA fragment containing T7 terminal region was obtained. This was ligated to λDASH II vector (Stratagene) cleaved with restriction enzyme *Xba*I by use of a DNA Ligation kit (Stratagene) and packaged by use of Gigapack III Gold packaging extract (Stratagene). The packaging was performed according to the protocol attached to the kit. This recombinant phage was subjected to plaque hybridization by use of T7 terminal probe of BAC clone 741 to obtain a recombinant phage containing a 13 kb DNA fragment of T7 terminal region of BAC clone 741. The obtained phage was called clone λUP. The T7 terminal probe of BAC clone 741 was performed by use of the following oligo DNA (its synthesis being outsourced to Griner).

### (Primers on the T7 side for clone 741)

Sense: 5'-

GTTCCTCTGTAATTCTAATGTACATCCACTGGTTGAAAGGTCTATGATAA3'

(SEQ ID No. 55)

Hybridization was performed in 6 x SSC, 0.5% SDS, or 5 x Denhardt at 57°C overnight and washing was performed in 2 x SSC or 0.5% SDS at 65°C.

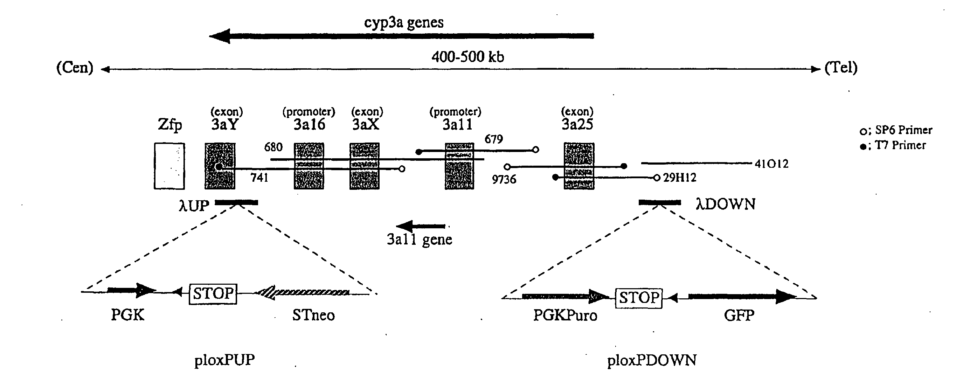

Then, cloning of Cyp3a25 gene region corresponding to the remaining end of Cyp3a gene cluster will be described. As shown in Fig. 5, the outermost genome region on the Cyp3a25 side of the cluster was SP6 terminal region of BAC clone 29H12 and hence cloning of ths portion was performed. Since the BAC clone was derived from 129/Sv mouse, the cloning was started from screening Genomic Library (Clonetech) derived from C57BL/6 mouse by use of SP6 terminal probe of 29H12. The SP6 terminal probe of 29H12 was subjected to plaque hybridization by use of a PCR product amplified with primers of SEQ ID Nos. 27 and 28.

As a result of screening about 2,000,000 plaques, 30 positive clones were obtained. Among them, 6 clones were optionally selected for performing preparation of a restriction enzyme map. When cleaving with restriction enzymes *Sal*I and *Eco*RI (Boehringer), about 8 kb DNA fragment occurred in one clone (λDOWN12). This fragment did not occur when cleaving with only *Eco*RI*.* Therefore, it was assumed that the terminal structures thereof were *Sal*I cleaving site and *Eco*RI cleaving site. The fragment was clone to plasmid pBluescript II SK(-) (Toyobo) that had been cleaved with restriction enzymes *Sal*I and *Eco*RI (Boehringer) to obtain a plasmid pDOWN. The inserted DNA fragment was sequenced (Toray Research Center) with primer M13-20. As a result, it revealed that this was SP6 terminal side of λ phage vector. From this sequence, the following primers were designed.

### (Primers on the SP6 side for clone λDOWN12)

Sense:      5'-TTCGGGAGGCCAGTACTATTC-3' (SEQ ID No. 56)
Antisense:  5'-CACACACGTCTTTTTGGCAG-3' (SEQ ID No. 57)

When PCR of BAC clones 29H12 and 41012 was performed by use of these primers, only 29H12 was positive. This revealed the positional relationship as shown in Fig. 5.

In consideration of the above results, as the clone corresponding to the both terminal ends genome regions of Cyp3a gene cluster, λ phageλUP and plasmid pDOWN were used in preparing targeting vectors described hereinbelow.

### (3) Preparation of targeting vectors ploxP3aUP and ploxP3aDOWN

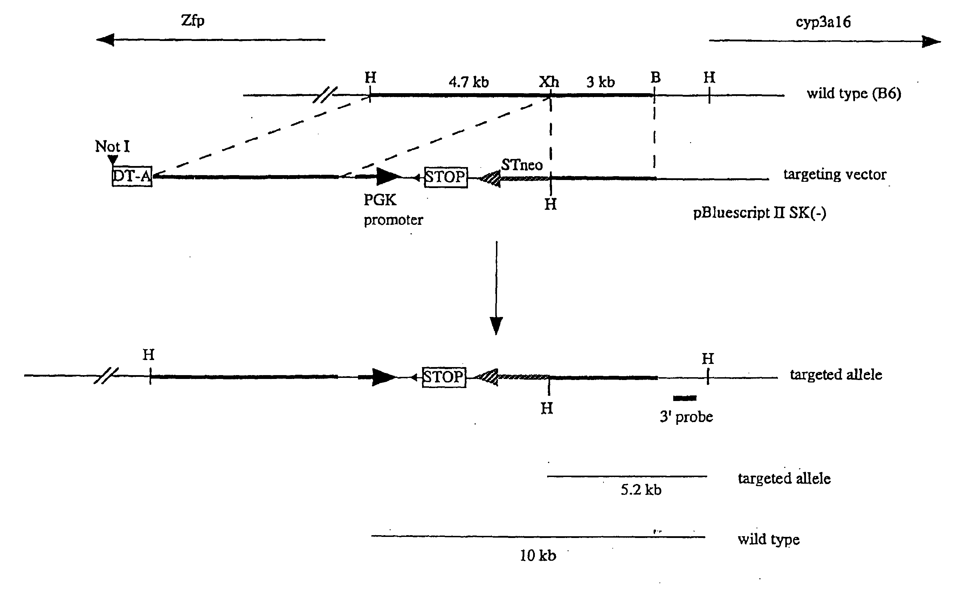

First, preparation of targeting vector ploxP3aUP will be described. Cleaving λ phage clone λUP with restriction enzymes *Bam*HI and *Hind*III (Boehringer) produced an about 7.5 kb DNA fragment. This was cloned to plasmid pBluescript II SK(-) (Toyobo) that had been cleaved with restriction enzymes *Bam*HI and *Hin*dIII to obtain a plasmid pUP. Note that the *Xho*I site of plasmid pBluescript II SK(-) was deleted in advance as described below. That is, plasmid pBluescript II SK(-) was cleaved with restriction enzyme *Xho*I (Boehringer), blunted, and self-ligated. In an analogous fashion, the *Kpn*I site of plasmid pUP was deleted and then cleaved with restriction enzyme *Xho*I (Boehringer), blunted, and inserted with *Kpn*I linker (Takara Shuzo Co., Ltd.). This plasmid was cleaved with restriction enzymes *Not*I and *Xba*I (Boehringer) and cloned with a DNA fragment that was cleaved from plasmid pMC1DT-AN with restriction enzymes *Not*I and *Spe*I (Boehringer). Finally, this plasmid was cleaved with restriction enzyme *Kpn*I (Boehringer), dephosphorylated and cloned with a DNA fragment from which plasmid ploxPUP was cleaved with restriction enzyme *Fse*l (Takara Shuzo Co., Ltd.), blunted and KpnI linker was inserted therein and then cleaved with restriction enzyme *Kpn*I (Boehringer) (targeting vector ploxP3aUP, Fig. 6).

Then, preparation of targeting vector ploxP3aDOWN will be described. First, plasmid pDOWN was cleaved with *Eco*RI (Boehringer) and blunted, and then *Sal*I linker (Takara Shuzo Co., Ltd.) was inserted therein. To the plasmid that was cleaved with restriction enzymes *Not*I and *Spe*I (Boehringer) was cloned a DNA fragment cleaved from plasmid pMC1DT-AN with restriction enzymes *Not*I and *Spe*I (Boehringer). Then, this plasmid was cleaved with restriction enzyme *Not I* (Boehringer) and blunted, and an *S*r*f*I linker was inserted therein. As the *Srf*I linker, the following synthetic oligo DNA (Griner) was used.

### (SrfI Linker)

### 5'-pGCCCGGGC-3'

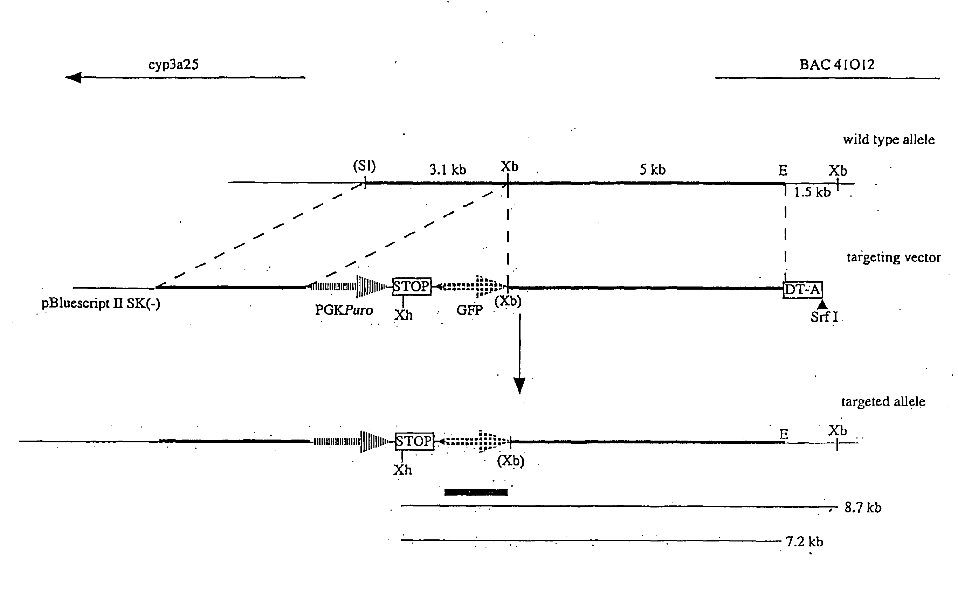

This plasmid was cleaved with restriction enzyme *Xba*I (Boehringer) and blunted, and then the above-mentioned *Fse*I linker was inserted therein. This plasmid was cleaved with restriction enzyme *Fse*I(Takara Shuzo Co., Ltd.) and dephosphorylated. To this plasmid was cloned a DNA fragment obtained by cleaving plasmid ploxPDOWN with restriction enzyme *Kpn*I (Takara Shuzo Co., Ltd.), blunting and inserting the *Fse*I linker and further cleaving it with restriction enzyme *Fse*I (Takara Shuzo Co., Ltd.) (targeting vector ploxP3aDOWN, Fig. 7).

### Example 8 Acquisition of a cell line in which loxP sequence has been inserted in one end of Cyp3a gene cluster of mouse ES cell

### (1) Acquisition of a loxP seqeunce-inserted cell line

### (Part 1)

In order to disrupt the mouse endogenous Cyp3a gene cluster, loxP sequences were inseted in both the ends, respectively, of Cyp3a gene cluster by use of two targeting vectors ploxP3aUP and ploxP3aDOWN as shown in the above examples. First, the targeting vector ploxP3aUP was linearized with restriction enzyme *Not*I (Takara Shuzo Co., Ltd.) and introduced into a mouse ES cell TT2F according to a conventional method (Shinichi Aizawa, Biomanual Series 8 Gene Targeting, Youdosha, 1995). Specifically, TT2F cells were treated wtih trypsin and suspended in cold HBS (HEPES-buffered saline) in a cell density of 2.5×10⁷ cells/ml and then 45 µg of vector was added thereto, followed by electroporation by use of Gene Pulser (Biorad) under conditions of 250 V and 960 µF. The electroporated cells were suspended in 8 ml of ES medium (Shinichi Aizawa, Biomanual Series 8 Gene Targeting, Youdosha, 1995) and inoculated in eight (8) 60-mm dishes in which feeder cells were pre-plated. After 48 hours' cultivation, elective culture was performed in a medium containing 300 µg/ml (apparent concentration) of G418 (GIBCO) for about 10 days. The appeared 144 G418 resistant colonies were picked up and cultivated on a 48-well plate until they became confluent. Thereafter, 1 well portion of the culture on the 48-well plate was inoculated in 2 wells of a 4-well plate. One well portion of the culture on the 4-well plate was stored under freezing at -80°C. The remaining one well portion was plated and cultivated in a gelatin-coated 3.5-cm dish as a portion for the acquisition of genome DNA. Other specific procedures of manipulation of mouse ES cells were in accordance with the methods described in WO97/07671 and WO98/37757.

Extraction of genome DNA was performed by a phenol method. The extracted genome DNA was digested with restriction enzyme *Hin*dIII(NEB) and separated by agarose gel electrophoresis. Thereafter, identification of homologous recombinant (called "ES/ loxP3aUP") was performed by Southern hybridization. As a probe for Southern blotting for detecting clones in which homologous recombination with the targeting vector ploxP3aUP occurred, a DNA fragment (about 400 bp) in the mouse Cyp3aY region was used. This DNA fragment was obtained by PCR amplification by use of C57BL/6 mouse DNA as a template under the following conditions.

### (Primers for Mouse Cyp3aY probe)

Sense:        5'-ACGGTTTATGCATGCTAGGTG-3' (SEQ ID No. 58)

Antisense: 5'-TCCATGACAATAAGGCTGCC-3' (SEQ ID No. 59)

In PCR, GeneAmp 9600 produced by Perkin-Elmer, Inc. was used as a thermal cycler, LA Taq (Takara Shuzo Co., Ltd.) was used as a Taq polymerase, and as the buffer and dNTP (dATP, dGTP, dCTP and dTTP) those attached to LA Taq (Takara Shuzo Co., Ltd.) were used under the recommended conditions. The temperature and cycle condition were such that after thermal denaturation at 85°C for 3 minutes and 94°C for 1 minute, the cycle of 98°C for 10 seconds, 65°C for 30 seconds, and 72°C for 1 minute was performed 35 times.

By Southern blotting analysis using the above-mentioned probe (for labeling the probe, Megaprime DNA labeling system of Amersham being used), homologous recombinants were detected. As a result, 1 clone out of 144 clones was a homologous recombinant. In wild-type TT2F cells, a band was detected at a position of about 10 kb by digestion with *Hin*dIII. In the homologous recombinant, this band was lost and a band was detected at a new postion of about 5 kb. From this it was confirmed that loxP sequence was inserted in the objective site by homologous recombination.

### (2) Acquisition of a loxP seqeunce-inserted cell line

### (Part 2)

In consideration of the fact that only 1 clone of a cell line in whcih a loxP seqeunce was inserted in one end of mouse Cyp3a cluster was obtained, isolation of homologous recombinants was performed again.

The sequence was itroduced into mouse ES cell TT2F by electroporation in the same manner as described in (1) above except that 15 µg of targeting vecgtor ploxP3aUP was used.

After the electroporation, cultivation was performed in ordinary ES medium (Shinichi Aizawa, Biomanual Series 8 Gene Targeting, Youdosha, 1995) for 24 hours, followed by elective culture in a medium containing 300 µg/ml (apparent concentration) of G418 (Gibco) for about 1 week. The produced 392 G418 resistant coloies were picked up and cultivated on a 24-well plate that had been plated with feeder cells until they became confluent. Then, they were plated onto 12-well plate that had been already inoculated with feeder cells. After cultivating on the 12-well plate for 2 days, the cells were recovered by treatment with typsin and a 3/4 portion thereof was stored under refrigeration at -135°C. The remaining 1/4 portion of cells was inoculated on a gelatin coated 12-well plate as a portion for the acquisition of genome DNA. Other specific procedures of manipulation of mouse ES cells were in accordance with the methods described in WO97/07671 and WO98/37757.

Extraction of genome DNAs was performed by use of Puregene DNA Isolation Kit (Gentra System, Inc.). The extracted genome DNA was digested with restriction enzyme *Hin*dIII (Boehringer) and separated by agarose gel electrophoresis. Thereafter, identification of homologous recombinant (called ES/loxP3aUP) was performed by Southern hybridization. As a probe for Southern blotting for detecting clones in which homologous recombination with the targeting vector ploxP3aUP occurred, the same probe as in (1) above was used.

By Southern blotting analysis using the above-mentioned probe (for labeling the probe, Megaprime DNA labeling system of Amersham being used), homologous recombinants were detected. As a result, 2 clones out of 392 clones were homologous recombinants. In wild-type TT2F cells, a band was detected at a position of about 10 kb by digestion with *Hin*dIII. In the homologous recombinants, this band was lost and a band was detected at a new position of about 5 kb (see Fig. 6). From this it was confirmed that loxP sequence was inserted in the objective site by homologous recombination.

### Example 9 Preparation of chimeric mouse from homologous recombinant ES cell (ES/loxP3aUP)

A chimeric mouse was prepared by use of the homologous recombinant TT2F cell line (ES/loxP3aUP) obtained in Example 8(2) in the same manner as in the preparation of a chimeric mouse by use of an ES cell harboring a human chromosome 7 fragment containing CYP3A family genes. That is, two clones 115 and 305, i.e., ES/loxP3aUP obtained in Example 8(2), were injected in amounts of 10 to 20 cells per embryo into 8-cell stage embryos obtained by mating MCH(ICR) (CLEA Japan) female and male mice with each other. The embryos were cultivated in ES cell medium (WO97/07671) for one night to be developed into blastlocysts. Thereafter, the embryos were transplanted in the uteri of temporary mother ICR mouse 2.5 days after pseudopregnancy treatment, in amounts of about 10 injected embryos per uterus on one side. As for the clone 115, 855 injected embryos were transferred into the uteri of foster mother mice and 29 chimeric mice were obtained and individuals of which the contribution ratio (chimeric ratio) of ES/loxP3aUP to somatic cells was near 100% were obtained. On the other hand, as for the clone 305, 429 injected embryos were transplanted into the uteri of foster mother mice and 1 chimeric mouse was obtained. As a result, it was judged that clone 115 was superior to clone 305 in chimera forming ability from the view point of the chimeric forming efficiency and chimera ratio and clone 115 was provided for further experiments.

### Example 10 Acquisition of a cell line having inserted loxP sequences at both ends of Cyp3a gene cluster of mouse ES cell

Of the homologous recombinants ES/loxP3aUP obtained in Example 8(2) (recombinants in which loxP sequences were inserted in the cyp3aY gene region in the mouse cyp3a gene cluster), clone 115 having higher' chimera forming ability was electroporated by use of targeting vector ploxP3aDOWN in the same manner as in Example 8. First, the targeting vector ploxP3aDOWN was linearized with restriction enzyme *Srf*I (Toyobo) and introduced into a mouse ES cell TT2F according to a conventional method (Shinichi Aizawa, Biomanual Series 8 Gene Targeting, Youdosha, 1995). Specifically, TT2F cells were treated wtih trypsin and suspended in HBS (HEPES-buffered saline) in a cell density of 2.5×10⁷ cells/ml and then 15 µg of a vector was added thereto, followed by electroporation by use of Gene Pulser (Biorad) under conditions of 250 V and 960 µF. After the electroporation, the cells were cultivated in ordinary ES medium (Shinichi Aizawa, Biomanual Series 8 Gene Targeting, Youdosha, 1995) for 24 hours and then subjected to selective cultivation performed in a medium containing 0.75 µg/ml of puromycin (Sigma) for about 1 week. The appeared 348 puromycin resistant colonies were picked up and cultivated for 3 days on a 24-well plate that had been already plated with feeder cells. Thereafter, the colonies were subcultured on a 12-well plate that had been already plated with feeder cells. After cultivation on the 12-well plate for 2 days, the colonies were treated with trypsin to recover cells. A 3/4 portion of the cells was stored under refrigeration at -135°C. The remaining 1/4 portion of the cells was inoculated on a gelatin-coated 12-well plate as a portion for the acquisition of genome DNA. Other specific procedures of manipulation of ES cells were in accordance with the methods described in WO97/07671 and WO98/37757. In the same manner as in Example 8, genome DNA was extracted from the puromycin resistant clone and identification of the homologous recombinant (ES/loxP3aUP+DOWN) was performed.

Extraction of genome DNA was performed by use of Puregene DNA Isolation Kit (Gentra System. Inc.) The extracted genome DNA was cleaved with restriction enzymes *Xho*I/*Xba*I and *Xho*I/*Eco*RI (Boehringer) and separated by agarose gel electrophoresis. Thereafter, identification of homologous recombinant (called ES/loxP3aUP+DOWN) was performed by Southern hybridization. As a probe for Southern blotting for detecting clones in which homologous recombination with the targeting vector ploxP3aDOWN occurred, a DNA fragment (about 700 bp) encoding GFP in the inside of the targeting vector ploxP3aDOWN was used. This DNA fragment was obtained by cleaving plasmid pGreen Lantern (Gibco) with restriction enzyme *Not*I (Boehringer).

By Southern blotting analysis using the above-mentioned probe (for labeling the probe, Megaprime DNA labeling system of Amersham being used), homologous recombinants were detected. As a result, 3 clones out of 348 clones were homologous recombinants. In wild-type TT2F cells, no band that hybridized with this probe was observed since they contained no GFP sequence. In the homologous recombinants, a band was observed at a position of about 9 kb by digestion with *Xho*I/*Xba*I and further a band was observed at a position of about 7 kb by digestion with *Xho*I/*Eco*RI (see Fig. 7). From this it was confirmed that loxP sequences were inserted in the both ends of mouse Cyp3a gene cluster by homologous recombination.

### Example 11 Preparation of a chimeric mouse from homologous recombinant ES cell (ES/loxP3aUP+DOWN)

In order to confirm if the homologous recombinant TT2F cell line (ES/loxP3aUP+DOWN) actually retained chimera forming ability, a chimera preparing experiment by use of homologous recombinant TT2F cell line (ES/loxP3aUP+DOWN) was performed in the same manner as in Example 9. That is, three clones 124, 125 and 147, i.e., ES/loxP3aUP+DOWN obtained in Example 10, were injected in amounts of 10 to 20 cells per embryo into 8-cell stage embryos obtained by mating MCH(ICR) (CLEA Japan) female and male mice with each other. The embryos were cultivated in ES cell medium (WO97/07671) for one night to be developed into blastlocysts. Thereafter, the embryos were transplanted in the uterus of foster mother ICR mouse 2.5 days after pseudopregnancy treatment, in amounts of about 10 injected embryos per uterus on one side. As for the clone 124, 422 injected embryos were transferred into the uteri of foster mother mice and 8 chimeric mice were obtained and individuals of which the contribution ratio (chimera ratio) of ES/loxP3aUP+DOWN to somatic cells was near 100% were obtained. On the other hand, as for the clones 125 and 147, 479 and 204, respectively, injected embryos were transplanted into the uteri of foster mother mice and 3 chimeric mice each were obtained. As a result, it was judged that clone 124 was superior to other clones in chimera forming ability in consideration of chimera forming efficiency and chimera ratio and clone 124 was provided for further experiments.

### Example 12 Acquisition of a mouse ES cell Cyp3a gene cluster deleted cell line

Of the ES cell lines ES/loxP3aUP+DOWN obtained in the two targeting experiments described above (recombinants in which loxP sequences were inserted at the both ends of the mouse cyp3a gene cluster), clone 124 having higher chimera forming ability was transfected with Cre recombination enzyme expression vector pBS185 (Gibco). This was expected to cause site-specific recombination between the loxP sequences at the both ends of the mouse endogenous cyp3a gene cluster to delete the Cyp3a gene cluster. Basic procedure was performed according to Example 8 above.

ES cell line ES/loxP3aUP+DOWN was treated with trypsin and suspended in HBS in a cell density of 1.0×10⁷ cells/ml. Then, 30 µg of pBS185 vector was added thereto and electroporation was performed by use of gene pulser under the same conditions as in Example 8. Thereafter, the cells were plated on one 100 mm dish It was considered when recombinant between loxPs occurred as expected, GFP (Green Fluorescent Protein) gene in vector is expressed, could be detected by emission of fluorescence. In consideration of this, after 72 hours' cultivation, GFP positive cell mass was separated by use of FACS (fluorescence activation cell sorter).

The separation of GFP-positive cells by means of FACS was performed by the following procedure. That is, after cultivation for 72 hours from the introduction of Cre expression vector pBS185, the cells were separated from the dish by treatment with trypsin. After suspending the cells in ES use DMEM, they were transferred into a 15 ml tube and centrifuged at 1,000 rpm for 5 minutes. Thereafter, the supernatant was sucked. After tapping the tube, the cells were resuspended in 10 ml of ES use DMEM, and plated in a gelatin coated 100-mm dish and cultivated for about 1 hour. Thereafter, the medium was gently recovered and centrifuged at 1,000 rpm for 5 minutes. After the centrifugation, the supernatant was sucked and the cell mass was tapped to loosen it, followed by suspending the cells in 4 ml of sorting buffer (PBS/EDTA, 5% FCS). After the suspending, the suspension was set to FACS through a mesh filter of a tube (Falcon) equipped with a cell strainer cap. The GFP-positive cell mass separated by FACS was mixed with 4 ml of ES use medium containing penicillin and streptomycin, and then plated in a 60 mm dish that had been pre-plated with feeder cells such that sufficiently separated several tens of colonies could emerge. At the point in time from 3 hours from the inoculation, 70% of the medium was gently removed and the same amount of fresh ES use medium was added and cultivation was performed for about 1 week. In the same manner as in example 8, the obtained colonies were cultivated and genome DNA was prepared therefrom, from which it was confirmed by PCR that the cell line was mouse Cyp3a cluster-deleted cell line.

### (Primers for identifying mouse Cyp3a cluster deletied cell line)

Sense (PGK-2):

5'-TGTTCTCCTCTTCCTACTCTCC-3' (SEQ ID No. 60)

Antisense (GFP-2):

5'-TGAAGGTAGTGACCAGTGTTGG-3' (SEQ ID No. 61)

The reaction mixture for PCR was prepared by adding sterilized distilled water to 3 µl of 10 x Ex Taq Buffer (Takara Shuzo Co., Ltd.), 4.8 µl of dNTP (dATP, dGTP, dCTP and dTTP, each 2.5 mM) (Takara Shuzo Co., Ltd.), 10 pmol of each primer, about 100 ng of genome DNA, and 0.3 µl of Ex Taq (5U/ml) (Takara Shuzo Co., Ltd.) to make 30 µl, and dispensing the mixture in PCR use tubes (Perkin-Elmer). The manipulation of the whole procedure for preparing PCR reaction mixture was performed on ice. That is, a thermal cycler (GeneAmp PCR system 9600, Perkin-Elmer) was set to 85°C and after confirming that 85°C was reached, the tube was set. After performing pre-PCR consisting of a cycle of 85°C for 3 minutes, and 94°C for 1 minute, a reaction of a cycle of 98°C for 10 seconds, 59°C for 30 seconds and 72°C for 30 seconds was repeated 30 times. As a result, in 16 clones out of 17 clones, a band of about 200 bp was detected and it was confirmed that the product was a recombinant in which mouse Cyp3a gene cluster was deleted (ES/Δ3a).

### Example 13 Preparation of a chimeric mouse from a recombinant ES cell (ES/Δ3a)

In the same manner as in Example 9, chimera preparing experiments were performed by use of 6 clones of the recombinant TT2F cell line (ES/Δ3a) obtained in Example 12. That is, ES/Δ3a cells were injected in amounts of 10 to 20 cells per embryo into 8-cell stage embryos obtained by mating MCH(ICR) (CLEA Japan) female and male mice with each other. The embryos were cultivated in ES use DMEM for one night to be developed into blastlocysts. Thereafter, the embryos were transplanted in the uterus of foster mother MCH mouse 2.5 days after pseudopregnancy treatment, in amounts of about 10 injected embryos per uterus on one side. 330 injected embryos were transplanted and 10 chimeric mice were acquired.

From 3 out of the 10 chimeric mouse thus obtained, a portion of tail was biopsied on day 10 from the birth and genome DNAs were extracted from the tissue pieces by use of Puregene DNA Isolation Kit (Gentra Systems, Inc.). PCR was performed by use of the extracted genone DNAs as templates and it was confirmed that these mice were knockout mice deficient in mouse Cyp3a cluster. If the obtained chimeric mouse was a knockout mouse, it showed a genotype in which the gene locus of Cyp3a cluster was heterozygously deleted and it was expected that upon deletion by means of Cre-loxP, PGK promoter and GFP gene would couple to each other. As primers for confirming knockout mouse, primers PGK-2 (SEQ ID No. 60) and GFP-2 (SEQ ID No. 61) respectively for identifying mouse Cyp3a cluster deleted cell lines, respectively, were used in the same manner as in Example 12.

The reaction mixture for PCR was prepared by adding sterilized distilled water to 3 µl of 10 x Ex Taq Buffer (Takara Shuzo Co., Ltd.), 4.8 µl of dNTP (dATP, dGTP, dCTP and dTTP, each 2.5 mM) (Takara Shuzo Co., Ltd.), 10 pmol of each primer, about 100 ng of genome DNA, and 0.3 µl of Ex Taq (5U/ml) (Takara Shuzo Co:, Ltd.) to make 30 µl, and dispensing the mixture in PCR use tubes (Perkin Elmer). The manipulation of the whole procedure for preparing PCR reaction mixture was performed on ice. That is, a thermal cycler (GeneAmp PCR system 2400, Perkin Elmer) was set to 85°C and after confirming that 85°C was reached, the tube was set. After performing pre-PCR consisting of a cycle of 85°C for 3 minutes and 94°C for 1 minute, a cycle of 98°C for 10 seconds, 59°C for 30 seconds and 72°C for 30 seconds was performed 30 times. As a result, in all of the three chimeric mice, a band of about 200 bp was detected and thus it was confirmed that the mice were knockout mice whose mouse Cyp3a gene cluster was deleted.

### Bxample 14 Introduction of human chromosome 7 into chicken DT40 cells

According to the method of Tomizuka et al. (Nature Genet., 16:133-143, 1997), microcells were purified from about 10⁸ mouse A9 cells H5 harboring human chromosome 7 and suspended in 5 ml of DMEM. After washing with DMEM twice, 1-2×10⁷ chicken DT40 cells were suspended in 5 ml of DMEM and were added to the centrifuged microcells. The mixture was centrifuged at 1,500 rpm for 10 minutes and the supernatant was completely removed. The precipitate was sufficiently loosened by tapping and 0.5 ml of PEG1500 solution (Boehringer) was added thereto. The mixture was sufficiently stirred for about 2 minutes. Thereafter, 10 ml of DMEM was slowly added thereto and the mixture was centrifuged at 1,500 rpm for 10 minutes. The cells were cultivated in RPMI1640 medium (Gibco) to which 10% fetal calf serum (Gibco, hereinafter referred to as "FBS"), 1% chicken serum (Gibco), and 10⁻⁴ M 2-mercaptoethanol (Sigma) were added (hereinafter, abbreviated as "DT40 medium"). After 24 hours, the medium was exchanged for a medium containing 1 mg/ml of G418 (hereinafter, abbreviated as "G418-DT40 medium") and elective culture was performed for about 3 weeks. Genome DNA was extracted from drug resistant colonies and PCR thereof was performed by use of primers for detecting CYP3A4 and MDR1.

### (Primers for detecting CYP3A4 and MDR1)

Sense:      5'-CTCAGAAACTGTCAAGCATGC-3' (SEQ ID No. 62)
Antisense:  5'-CCTAAAGGAAACGAACAGCG-3' (SEQ ID No. 63)

In PCR, GeneAmp 9600 produced by Perkin-Elmer, Inc. was used as a thermal cycler, Ex Taq (Takara Shuzo Co., Ltd.) was used as a Taq polymerase, and as the buffer and dNTP (dATP. dGTP. dCTP and dTTP) those attached to Ex Taq (Takara Shuzo Co., Ltd.) were used under the reccommended conditions. The temperature and cycle condition were such that after thermal denaturation at 94°C for 1 minute, a cycle of 98°C for 10 seconds, 56°C for 30 seconds, and 72°C for 30 seconds was performed 35 times. As a result, 2 clones out of about 30 clones were positive to CYP3A4 and MDR1. Further, FISH was performed by use of human COT1 DNA probe. As a result, it revealed that full-length human chromosome 7 was independently present. From the above results, it was confirmed that chicken DT40 cell harboring full-length human chromosome 7 (hereinafter, abbreviated as "DT40-#7" ) was cloned.

### Example 15 Preparation of cassette vectors ploxPHyg. ploxPbsr

Cassette vectors ploxPHyg and ploxPbsr for inserting loxP sequences, which are recognition sequence of Cre recombinase on a human chromosome were prepared as follows. As described above, for enabling positive selection of cells in which translocation occurred as expected, the cassette vectors were constructed such that the former cassette vector contained PGK promoter and the latter cassette vector contained GFP gene to be transcripted by the PGK promoter.

First, preparation of ploxPHyg will be described. Plasmid pBluescript II SK(-) (Toyobo) was cleaved with restriction enzyme *Eco*RV (Boehringer) and reacted with dephosphorylase CIAP (calf small intestine-derived alkaline dephosphorylase, Takara Shuzo Co., Ltd.) at 50°C for 30 minutes to dephosphorylate the cleaved ends. To this was ligated a DNA fraction cleaved from plasmid pBS302 (Gibco) with restriction enzymes *Spe*I and *Hin*dIII (Boehringer) and blunted with a DNA Blunting kit (Takara Shuzo Co., Ltd.) by use of a DNA Ligation kit (Takara Shuzo Co., Ltd.) to transform a competent cell DH5 (Toyobo) of Escherichia coli (plasmid pBS302HS). Blunting, ligation and transformation were performed according to the protocols attached to the respective kits.

Then, the plasmid pBS302HS was cleaved with restriction enzyme *Sal*I (Boehringer) and dephosphorylated. To this was cloned a PGK promoter fragment cleaved from plasmid pGKPuro (endowed by Dr. Peter W. Laird of WHITEHEAD INSTITUTE) with restriction enzymes *Sal*I and *Xho*I (Boehringer) to obtain a plasmid pBSPGK302HS. The plasmid was cleaved with restriction enzymes *Eco*RI and *Not*I (Boehringer), blunted and dephosphorylated, followed by ligation of *Not*I linker (Takara Shuzo Co., Ltd.) thereto (plasmid pBSPGK302HSN).

On the other hand, plasmid #1-133 (endowed by Professor Shunichi Takeda of Medical Department of Kyoto University) was cleaved with restriction enzyme *Bam*HI (Boehringer) to cut out a hygromycin B resistant gene cassette, the terminal of which was then blunted. Plasmid pBSPGK302HSN was cleaved with restriction enzyme *Sal*I (Boehringer) and blunted. Thereafter, the hygromycin B resistant gene cassette was cloned thereto (plasmid ploxPHyg).

Next, preparation of a plasmid ploxPbsr will be explained. First, an *Sfi*I linker was inserted to the SacI site of plasmid pBluescript II SK(-) (Toyobo) in the manner as described above. The *Sfi*I linker was obtained by synthesizing an oligo DNA having the following sequence and phosphorylating the 5'-end thereof (synthesis being outsourced to Griner).

### (SfiI Linker)

### 5'-pGGCCGC [A/T]GCGGCC-3' (SEQ ID No. 64)

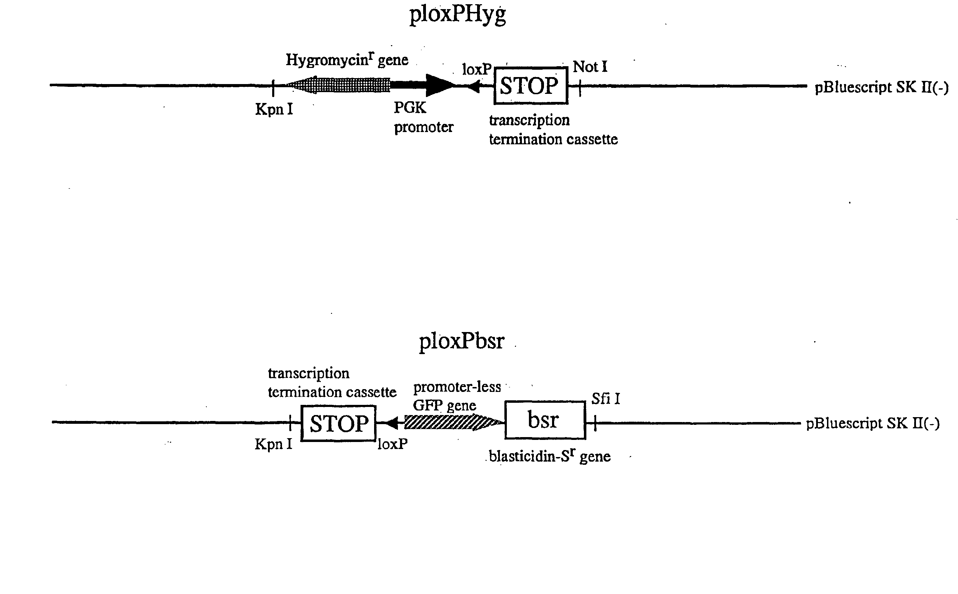

This plasmid was cleaved with restriction enzyme *Bam*HI (Boehringer), dephosphorylated and cloned to a DNA fragment cleaved from plasmid pBS302 (Gibco) with respriction enzyme *Bam*HI (Boehringer) (pBSSfK302B). Further, to the *Cla*I site of plasmid pGREEN LANTERN-1 (Gibco) was inserted an *Spe*I linker (Takara Shuzo Co., Ltd.) as described above and a DNA fragment was cleaved with restriction enzyme *Spe*I (Boehringer) to obtain a GFP gene cassette fragment. This was cloned to plasmid pBSSfK302B that had been cleaved with *Spe*I and then dephosphorylated (pBSSfK302BGFP). This palsmid was cleaved with restriction enzyme *Xba*I (Boehringer), blunted, and cloned with a DNA fragment (blastocidin S resistant gene cassette) obtained by cleaving plasmid #1-134 (endowed by Professor Shunichi Takeda of Medical Department of Kyoto University) with restriction enzyme *Bam*HI (Boehringer) and blunting (ploxPbsr). The structure of cassette vectors ploxPHyg and ploxPbsr are shown in Fig. 8.

### Example 16 Preparation of targeting vectors pBSCOL1A21ox(F) and pBSCOL1A2lox(R)

Targeting vectors pBSCOL1A21ox(F) and pBSCOL1A21ox(R) for inseting loxP sequences in the genome region (COL1A2) of pro alpha 2(I) collagen just centromeric to the CYP3A gene locus on the human chromosome 7 were prepared as follows. Note that since the direction of transcription of COL1A2 was unclear whether centromere -> telomere or telomere -> centromere, targeting vectors of two directions (F) and (R) were prepared.

First, the genome region COL1A2 was amplified by use of genome DNA of DT40-#7 obtained in Example 14 above as a template and the primers as shown below. On this occasion, the recognition site of restriction enzyme *Kpn*I was added to the 5' terminal of the primers in order for the amplified PCR product to be cloned to the vector.

### (Primers for amplifying gene region COL1A2)

Sense:
5'-CGTAGGTACCCAAGTCAGGCTTACATTTTATTTGTG-3' (SEQ ID No.
65)
Antisense:

5'-CGTAGGTACCGAGAATGAGTTTGACCTTATCCATTT-3' (SEQ ID No.
66))

In PCR, GeneAmp 2400 produced by Perkin-Elmer, Inc. was used as a thermal cycler, Ex Taq (Takara Shuzo. Co., Ltd.) was used as a Taq polymerase, and as the buffer and dNTP (dATP. dGTP. dCTP and dTTP) those attached to Ex Taq (Takara Shuzo Co., Ltd.) were used under the reccommended conditions. The temperature and cycle condition were such that after thermal denaturation at 94°C for 1 minute, a cycle of 98°C for 10 seconds and 65°C for 15 minutes was performed 35 times. After treating the PCR product with phenol/chloroform twice, it was subjected to gel filtration by use of CHROMA SPIN-TE400 (Clonetech). Thereafter, it was cleaved with restriction enzyme *Kpn*I (Rosche Diagnostics) and treated with phenol/chloroform twice, followed by gel filtration by use of CHROMA SPIN-TE1000 (Clonetech). On the other hand, the *Not*I site of plasmid pBluescriptII was cleaved with restriction enzyme *Not*I (Boehringer), blunted and self-ligated to delete the *Not*I site and further an *Srf*I linker was inserted to the SacII site thereof to prepare plasmid vector [pBS(N)Sr]. Note that as the *Srf*I linker, an oligo DNA having the following sequence was used.

### (SrFI Linker)

### 5'-pGCCCGGGC-3'

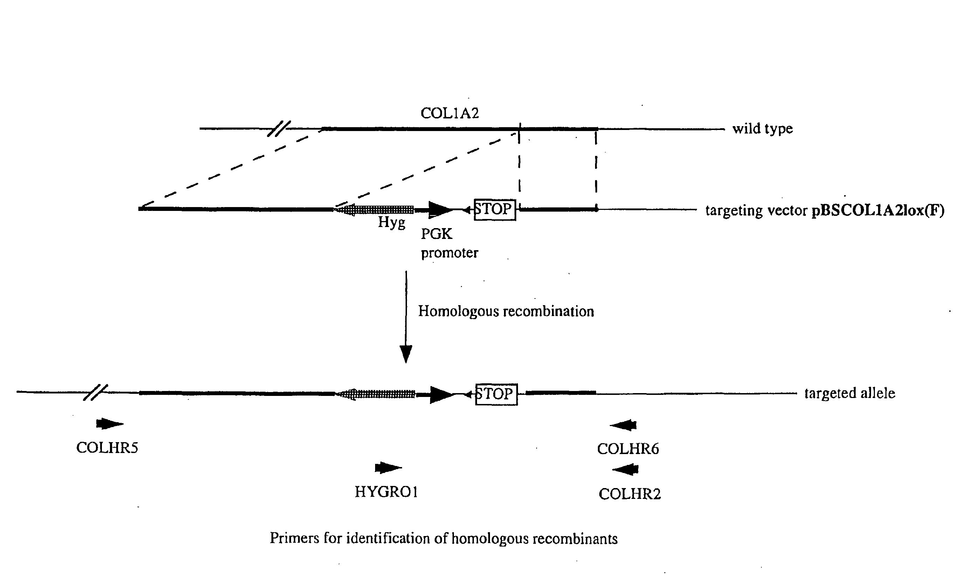

A plasmid pBSCOL1A2 was prepared by cloning a PCR fragment of COL1A2 gene having the above-mentioned *Kpn*I terminal at the *Kpn*I site of plasmid [pBS(N)Sr]. This plasmid was cleaved with restriction enzyme StuI (Rosch Diagnostics) and dephosphorylated and then an *Not*I linker (Takara Shuzo Co., Ltd.) was inserted therein to prepare pBSCOL1A2Not. On the other hand, an *Not*I linker (Takara Shuzo Co., Ltd.) was inserted at the *Kpn*I site of cassette vector ploxPHyg, and cleaved with restriction enzyme *Not*I (Boehringer) to excise a DNA fragment containing a loxP sequence, which was cloned to the *Not*I site of plasmid pBSCOL1A2Not to prepare a plasmid pBSCOL1A2lox. The plasmid of which the direction of the loxP sequence was in the same direction as the cloned genome fragment COL1A2 was named (F) and the plasmid of which the direction of the loxP seqence was in the opposite direction was named (R) [pBSCOL1A21ox(F) and (R), Fig. 9].

### Example 17 Preparation of cassette vector pTELPuro

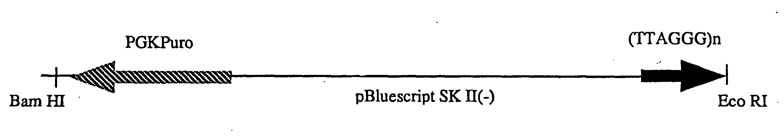

A cassette vector pTELPuro for inserting a human telomere sequence into a human chromosome was prepared as follows. That is, the human telomere sequence was synthesized by PCR according to J. J. Harrington et al. (Nature Gnet., 15, 345-355, 1997) and cloned to the *Eca*RV site of plasmid pBluescript II SK(-) (Toyobo) (pTEL). Then, a DNA fraction obtained by substituting the *Eco*RI site of plasmid pGKPuro(endowed by Dr. Peter W. Laird of WHITEHEAD INSTITUTE) for *Not*I site and cleaving it with restriction enzyme *Not*I (Boehringer) (puromycin resistant gene cassette) was cloned to the *Not*I site of plasmid pTEL (pTELPuro, Fig. 10).

### Example 18 Preparation of targeting vectors pTELPuro5.3(F) and pTELPuro5.3(R)

Targeting vectors pTELPuro5.3(F) and pTELPuro5.3(R) for inserting a human telomere sequence into an AF006752 marker (AF006752), which was a genome region located just telomeric to CYP3A gene locus cluster on human chromosome 7 were prepared as follows. Note that since the direction of sequence of AF006752 was unclear as to whether centromere -> telomere or telomere -> centromere, targeting vectors of two directions (F) and (R) were prepared.

First, the genome region of AF006752 gene locus was amplified by PCR by use of genome DNA of DT40-#7 obtained in Example 14 above as a template and the primers as shown below. On this occasion, the recognition site of restriction enzyme *Bam*HI was added to the 5' terminal of the sense primer in order for the amplified PCR product to be cloned to the vector.

### (Primers for amplifying gene region AF006752)

Sense:
5'-CGTAGGATCCATGTTTCTGGAATACTTGCTGTTATTAG-3' (SEQ ID
No. 67)
Antisense:

5'-CAAAACAAACACTATTATCACTTCACAA-3' (SEQ ID No. 68))

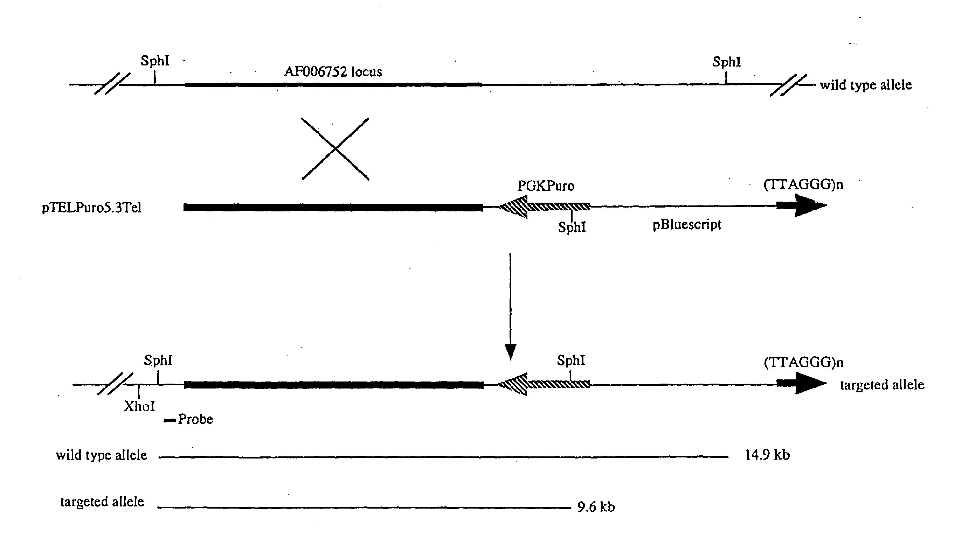

In PCR, GeneAmp 2400 produced by Perkin-Elmer, Inc. was used as a thermal cycler, Ex Taq (Takara Shuzo Co., Ltd.) was used as a Taq polymerase, and as the buffer and dNTP (dATP. dGTP. dCTP and dTTP) those attached to Ex Taq (Takara Shuzo Co., Ltd.) were used under the reccommended conditions. The temperature and cycle condition were such that after thermal denaturation at 94°C for 1 minute, a cycle of 98°C for 10 seconds and 65°C for 15 minutes was performed 35 times. After treating the obtained about 8 kb PCR product with phenol/chloroform twice, it was subjected to gel filtration by use of CHROMA SPIN-TE100 (Clonetech). Thereafter, it was cleaved with restriction enzyme *Bam*HI (Rosche Diagnostics) and treated with phenol/chloroform twice, followed by gel filtration by use of CHROMA SPIN-TE1000 (Clonetech). The PCR product after the gel filtration was subjected to electrophoresis on 0.6% agarose gel. Thereafter, about 5.3 kb *Bam*HI fragment was cut out of the agarose gel. The *Bam*HI fragment of PCR product was extracted from the cut out gel piece with GeneCleanII (Bio10l) and cloned to the *Bam*HI site of plasmid pTELPuro. The cloned 5.3 kb genome fraction of AF006752 in the same direction as that of human telomere sequence was named (F) and that in the opposite direction was named (R) [pTELPuro5.3(F) and (R), Fig. 11].

### Example 19 Site-specific cleavage of human chromosome 7 in chicken DT40 cells

It was contemplated to transfect the targeting vectors pTELPuro5.3(F) and (R) prepared in Example 18 above to DT40-#7 obtained in Example 14 above to insert human telomere sequence to the genome region AF006752, thereby cleaving chromosome 7 at the inserted site.

Cultivation of DT40-#7 was performed in G418-DT40 medium in the same manner as in Example 14. That is, 10⁷ cells of DT40-#7 were washed with FBS-free RPMI1640 medium once and suspended in 0.5 ml of FBS-free RPMI1640 medium. After adding thereto 25 to 30 µg of targeting vector pTELPuro5.3(F) or pTELPuro5.3 (R) linearized with restriction enzyme *Sfi*I (Toyobo), the suspension was transferred into a cuvette (Biorad) for electroporation and left to stand at room temperature for 10 minutes. The cuvette was set in Gene Pulser (Biorad) and voltage was applied thereto under conditions of 550 V and 25 µF. After standing at room temperature for 10 minutes, the cells were incubated in DT40 medium for 24 hours. After 24 hours, the medium was exchanged for DT40 medium containing puromycin (0.3 µg/ml) (hereinafter, abbreviated as "Puro-DT40 medium") and the culture was dispensed to five (5) 96-well cultivation plates and selective cultivation was performed for about 2 weeks. Genome DNA was extracted from puromycin resistant clones by use of Puregene DNA Isolation Kit (Gentra System, Inc.). This genome DNA was treated with restriction enzymes SphI (Takara Shuzo Co., Ltd.), HpaI (Takara Shuzo Co., Ltd.), and *Xho*I (Toyobo) and electrophoresed in 0.8% agarose gel, followed by alkali blotting on Gene Screen Plus^{TM} hybridization transfer membrane (NEN™ Life Science Products, Inc.). This filter was subjected to Southern hybridization by use of PS11 probe obtained by amplifying the gene sequence in AF006752 by PCR to perform identification of homologous recombinants (Fig. 11). PS11 probe was prepared by performing PCR by use of the following primers and genome DNA of DT40-#7 as a template and performing random priming by use of the PCR product as a template, thereby preparing ³²P labeled DNA probe (Amersham, according to the attached protocol).

### (Primers for preparing PS11 Probe)

PSF11 : 5'-AGCTTTGGGGACAAAACAT-3' (SEQ ID No. 69)
PSR11 : 5'-CAAAACAGTCTGAACAGACATTCC-3' (SEQ ID No. 70)

In PCR, GeneAmp 2400 produced by Perkin-Elmer, Inc. was used as a thermal cycler, Ex Taq (Takara Shuzo Co., Ltd.) was used as a Taq polymerase, and as the buffer and dNTP (dATP, dGTP, dCTP and dTTP) those attached to Ex Taq (Takara Shuzo Co., Ltd.) were used under the reccommended conditions. The temperature and cycle condition were such that after thermal denaturation at 93°C for 5 minutes, a cycle of 93°C for 1 minute, 54°C for 1 minute and 72°C for 1 minute was performed 35 times. It was anticipated that by Southern hybridization, about 14.9 kb band would be detected in nonhomologous recombinants and about 9.6 kb band would be detected in homologous recombinants (Fig. 11). As a result of Southern hybridizatin, 15 clones out of 145 clones in the case of clones obtained by transfecting targeting vector pTELPuro5.3(F), and 1 clone out of 143 clones in the case of clones obtained by transfecting targeting vector pTELPuro5.3(R) were objective homologous recombinants (called DF and DR clones, respectively).

Then, whether or not cleavage of chromosome 7 occurred in the genome region AF006752 in homologous recombinant F clones (DF87, DF117, DF124, DF137, and DF141 and R clone (DR107) was examined by PCR and FISH analysis.

### (1) PCR Analysis

The existence of polymorphic marker in the vicinity of CYP3A family gene locus on the chromosome 7 (Fig. 12) was detected by PCR by use of the following primers. As the primers for detecting D7S479, D7S651, D7S518, D7S658, D7S2420 and D7S523, Mouse MapPairs™ (Research Genetics, Inc.) was used. PCR was performed by use of GeneAmp 2400 produced by Perkin-Elmer, Inc. as a thermal cycler, Ex Taq (Takara Shuzo Co., Ltd.) as a Taq polymerase, and as the buffer and dNTP (dATP, dCTP, dGTP and dTTP) those attached to Ex Taq (Takara Shuzo Co., Ltd.) under the recommended conditions. The temperature and cycle condition were such that after thermal denaturation at 95°C for 2 minutes, the cycle of 94°C for 45 seconds, 55 to 60°C for 45 seconds, and 72°C for 1 minute was performed 35 times. The results obtained are shown in Table 4. o indicates positive and x indicates negative.

**Table 4**

| | DF87 | DF117 | DF124 | DF137 | DF141 | DR107 | ST40-#7 | DT40 |
|---|---|---|---|---|---|---|---|---|
| D7S479 | o | o | o | o | o | o | o | x |
| CYP3A4 | o | o | o | o | o | o | o | x |
| D7S651 | o | o | o | o | o | o | o | x |
| D7S518 | o | o | x | x | x | o | o | x |
| D7S658 | o | o | x | x | x | o | o | x |
| D7S2420 | o | o | x | x | x | o | o | x |
| D7S523 | o | o | x | x | x | o | o | x |

In DT40-#7 harboring full-length human chromosome 7, all the markers were detected while in homologous recombinant F clones (DF124, DF137 and DF141), those markers located closer to the telomere side than AF006752 (D7S518, D7S658. D7S2420, and D7S523) were all lost while the markers on the centromere side were all detected. On the other hand, in R clones, all the markers were detected.

### (2) FISH Analysis

In order to visually judge that human chromosome 7 is cleaved in the genome region AF006752, FISH by use of a pGKPuro probe that can detect puromycin resistant gene in the targeting vector was performed. The method was according to Kuroiwa et al. (Nucleic Acid Research, 26:3447-3448, 1998). As a result of COT1 dyeing (Rhodamine label, red), it was confirmed from the size of human chromosome 7 and chromosome band pattern that the chromosome 7 was fragmented more in DF clone and DR clone than in DT40-#7. Furthermore, a signal originated from pGKPuro probe (FITC label, yellow) was detected on one end of telomere newly formed in the vicinity of 7q22. This showed that AF006752 gene locus constitutes one end of telomere of human chromosome 7 fragment.

From the results of PCR and FISH described above, it was considered that in homologous recombinants, human chromosome 7 was cleaved in the genome region AF006752. In particular, with respect to clone DF141, PCR confirmed that the marker on the telomere side of AF006752 gene locus, which was the telomere insertion site, was deficit and in all of 50 cell fission images observed by FISH, fragmentation of chromosome was visually confirmed. From these, it was judged that the clone DF141 was a truncated form and the clone DF141 was provided for subsequent experiments.

### Example 20 Site-specific Insertion of loxPHyg cassette on human chromosome 7 in chicken DT40 cells

It was contemplated to transfect targeting vectors pBSCOL1A2lox(F) and (R) to chicken DT40 cells (DF141 clone) harboring human chromosome 7 fragment cleaved in the genome region AF006752 obtained in Example 19 above to insert loxPHyg cassette in the genome region COL1A2.

Cultivation of DF141 clone was performed in Puro-DT40 medium. The transfection was performed by the same procedure as in Example 19 above. That is, about 10⁷ cells were washed with FBS-free RPMI1640 medium once and suspended in 0.5 ml of FBS-free RPMI1640 medium. After adding thereto 25 to 30 µg of targeting vector pBSCOL1A2lox(F) or (R) linearized with restriction enzyme *Srf*I (Toyobo) or *Sal*I (Rosche Diagnostics), the suspension was transferred into a cuvette (Biorad) for electroporation and left to stand at room temperature for 10 minutes. The cuvette was set in Gene Pulser (Biorad) and voltage was applied thereto under conditions of 550 V and 25 µF. After standing at room temperature for 10 minutes, the cells were incubated in DT40 medium for 24 hours. After 24 hours, the medium was exchanged for DT40 medium containing hygromycin (1 mg/ml) (hereinafter, abbreviated as "Hyg-DT40 medium") and the culture was dispensed to five (5) 96-well cultivation plates and selective cultivation was performed for about 2 weeks. Genome DNA was extracted from hygromycin B resistant clones by use of Puregene DNA Isolation Kit (Gentra System, Inc.). Identification of homologous recombinant at the COL1A2 site was performed in accordance with PCR by use of the following primers (Fig. 9).

### (Primers for identifying homologous recombinants at the COL1A2 site)

COLHR5 : 5'-TTTCTAGGAAACTGTAAGAAAGGTAAGAGT-3' (SEQ ID No.
71)
COLHR6 : 5'-TGGTATTCACTTTAATATCACATTATCTCC-3' (SEQ ID No.
72)

In PCR, GeneAmp 2400 produced by Perkin-Elmer, Inc. was used as a thermal cycler, Ex Taq (Takara Shuzo Co., Ltd.) was used as a Taq polymerase, and as the buffer and dNTP (dATP. dGTP. dCTP and dTTP), those which are attached were used under the reccommended conditions. The temperature and cycle condition were such that after thermal denaturation at 94°C for 1 minute, a cycle of 98°C for 10 seconds and 65°C for 15 minutes was performed 35 times. In PCR by use of primers COLHR5 and COLHR6 described above in combination, amplification occurred so as to sandwich the site-specifically introduced loxPHyg cassette (Fig. 9), so that it was considered that in nonhomologous recombinants a band of about 8.5 kb was detected. On the other hand, in homologous recombinants, it was anticipated that a band of about 14.1 kb was detected or no band was detected because of GC-rich sequence contained in β-actin promoter in the loxPHyg cassette. As a result of PCR, the PCR product was not amplified in 34 clones out of 168 clones obtained by transfecting the targeting vector pBSCOL1A2lox(F) (called "TF clones") and in 32 clones out of 156 clones obtained by transfecting the targeting vector pBSCOL1A2lox(R) (called "TR clones") and hence these were considered to be homologous recombinants. Furthermore, optionally several clones were selected from TF and TR clones that were considered to be such homologous recombinants and each subjected to PCR by use of the following primers in combination and again it was confirmed that these were homologous recombinants.

### (Primers for confirming homologous recombinants at TF clone)

COLHR2 : 5' -TATCCTTTTTAAAGTTATTTTGAACACTCA-3' (SEQ ID No.
73)
HYGRO1 : 5' -ATGGTTTCTACAAAGATCGTTATGTTTATC-3' (SEQ ID No.
74)
(Primers for confirming homologous recombinants at TR
clone)
COLHR1 : 5' -ATTTATTATTGTCCTGTTTGTATCTTTCCT-3' (SEQ ID No.
75)
HYGRO1 : 5' -ATGGTTTCTACAAAGATCGTTATGTTTATC-3' (SEQ ID No.
76

PCR was performed by use of GeneAmp 2400 produced by Perkin-Elmer, Inc. as a thermal cycler, La Taq (Takara Shuzo Co., Ltd.) as a Taq polymerase, and as the buffer and dNTP (dATP, dCTP, dGTP and dTTP) those attached to La Taq (Takara Shuzo Co., Ltd.) under the recommended conditions. The temperature and cycle condition were such that after thermal denaturation at 85°C for 3 minutes and 94°C for 15 minutes, the cycle of 98°C for 10 seconds and 65°C for 15 minutes was performed 30 times. As a result, a band of about 4.3 kb in TF clones and a band of 8.1 kb in TR clones were detected and they were confirmed to be homologous recombinants (Fig. 9).

### Example 21 Preparation of targeting vector pRNR2loxPbsr

A targeting vector pRNR2loxPbsr for inserting loxP sequences on the RNR2 gene locus on human chromosome 14 was prepared as follows. First, the genome region of human RNR2 gene locus was amplified by PCR by use of the following primers.

### (Primers for amplifying gene region RNR2)

Sense: 5'-TCGAGAATTCAGTAGCTGGCACTATCTTTTTGGCCATC-3' (SEQ
ID No. 77)
Antisense:    5'-TCGAGAATTCGGAGAAAGAACACACAAGGACTCGGTC-3'
(SEQ ID No. 78)

In PCR, GeneAmp 9600 produced by Perkin-Elmer, Inc. was used as a thermal cycler, Ex Taq (Takara Shuzo Co., Ltd.) was used as a Taq polymerase, and as the buffer and dNTP (dATP. dGTP. dCTP and dTTP) those attached to Ex Taq (Takara Shuzo Co., Ltd.) were used under the reccommended conditions. The temperature and cycle condition were such that after thermal denaturation at 94°C for 1 minute, a cycle of 98°C for 10 seconds and 65°C for 15 minutes was performed 35 times. The PCR product was treated with proteinase K (Gibco) and then subjected to gel filtration by use of CHROMA SPIN-TE400 (Clonetech). Thereafter, it was cleaved with restriction enzyme *Eco*RI (Boehringer) and subjected to gel filtration by use of CHROMA SPIN-TE1000 (Clonetech). On the other hand, the *Kpn*I site of plasmid pBluescriptII was cleaved with restriction enzyme *Kpn*I (Boehringer), blunted and self-ligated to delete the *Kpn*I site and further an *Srf*I linker was inserted to the *Not*I site thereof to prepare a plasmid vector [pBS(K)Sr]. Note that as the *Srf*I linker, an oligo DNA having the following sequence was used.

### (SrfI Linker)

### 5'-pGCCCGGGC-3'

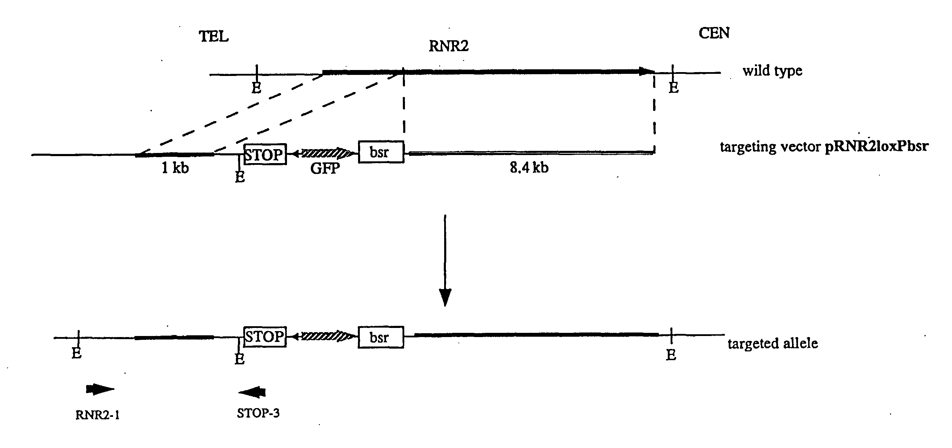

A plasmid pRNR2 was prepared by cloning the above-mentioned PCR fragment of the above-mentioned RNR2 gene at the *Eco*RI site of plasmid [pBS(K)Sr]. On the other hand, a *Kpn*I linker (Takara Shuzo Co., Ltd.) was inserted in the *Sfi*I of cassette vector ploxPbsr and a DNA fragment containing a loxP sequence was cut out with restriction enzyme *Kpn*I (Boehringer) and cloned to the *Kpn*I site of plasmid pRNR2. Since it was reported that the RNR2 gene is transcripted in the direction of telomere→centromere (R. G. Worton et al., SCIENCE, 239:64-68, 1988), the plasmid of which the direction of the PCR fragment of the cloned RNR2 gene was opposite to the direction of the loxP sequence was used as the targeting vector (pRNR21oxPbsr, Fig. 13).

### Example 22 Site-specific Insertion of loxPbsr cassette on human chromosome 14 in chicken DT40 cells

It was contemplated to transfect the targeting vector pRNR2loxPbsr prepared in Example 21 above to chicken DT40 cells harboring human chromosome 14 fragment SC20 (endowed by Professor Oshimura, Medical Department of Tottori University) to insert loxPbsr cassette into RNR2 gene locus.

In the same manner as above, the targeting vector pRNR2loxPbsr linearized with restriction enzyme *Srf*I (Toyobo) was transfected and the transfected cells were subjected to selective cultivation in DT40 medium containing blastocidin S (10 µg/ml) (hereinafter, abbreviated as "bsr-DT40 medium") for about 2 weeks. Genome DNA was extracted from resistant clones and identification of homologous recombinants was performed by PCR by use of the following primers (Fig. 13).

### (Primers for identifying homologous recombinants)

Sense (RNR2-1) : 5'-TGGATGTATCCTGTCAAGAGACC-3' (SEQ ID
No. 79)
Antisense (STOP-3): 5'-CAGACACTCTATGCCTGTGTGG-3' (SEQ ID
No. 80)

In PCR, GeneAmp 9600 produced by Perkin-Elmer, Inc. was used as a thermal cycler, Ex Taq (Takara Shuzo Co., Ltd.) was used as a Taq polymerase, and as the buffer and dNTP (dATP, dGTP, dCTP and dTTP) those attached to Ex Taq (Takara Shuzo Co., Ltd.) were used under the reccommended conditions. The temperature and cycle condition were such that after thermal denaturation at 94°C for 1 minute, a cycle of 98°C for 10 seconds and 65°C for 5 minutes was performed 35 times. As a result, in 8 clones out of 60 clones, an about 2.5 kb PCR product was amplified as expected to obtain homologous recombinant (called "R clone").

### Example 23 Construction of human artificial chromosome #7-HAC having human CYP3A gene cluster

As described in the general description, in order to more stably and efficiently express complete human CYP3A genes in a mouse, it was contemplated to translocate human chromosome 7 fragment consisting of COL1A2-CYP3A-AF006752tel to RNR2 gene locus on human chromosome 14 fragment SC20 to thereby construct human artificial chromosome #7-HAC having a human CYP3A gene cluster.

First, the homologous recombinant clones TF and TR obtained in Example 20 as described above were subjected to cell fusion with homologous recombinant R clone to prepare a DT40 hybrid harboring both the human chromosome 7 fragment and chromosome 14 fragment SC20.

### (1) Preparation of DT40 hybrid harboring both human chromosome 7 fragment and chromosome 14 fragment SC20

R clone was cultivated in bsr-DT40 medium and clones TF and TR were cultivated in Hyg-DT40 medium. After mixing and centrifuging 1-2×10⁷ cells of TF and R clones, they were washed in serum-free RPMI1640 medium twice. After completely removing residual medium, 0.5 ml of 50% PEG 1500 (Rosche Diagnostics) that had been warmed to 37°C in advance was gently added and the mixture was vigorously stirred with a pipette for about 2 minutes. Thereafter, 1 ml of serum-free RPMI1640 medium was slowly added over 1 minute and then 9 ml of serum-free RPMI1640 medium was added over about 3 minutes. The mixture was left to stand at 37°C for 10 minutes. Thereafter, it was centrifuged at 1,200 rpm for 5 minutes, the supernatant was removed and the residue was cultivated in DT40 medium for 24 to 48 hours.

Thereafter, the medium was exchanged for DT40 medium containing blastocidin S (10 µg/ml) and hygromycin B (1 mg/ml) and the culture was dispensed to five (5) 24-hole cultivation plates and cultivated for 3 to 4 weeks. Similarly, the TR and R clones were subjected to cell fusion. Genome DNA was extracted from a hybrid obtained by cell fusion between the TF and R clones and a hybrid obtained by cell fusion between the TR and R clones and PCR was performed by use of the following primers to confirm that two human chromosomes, i.e., humanchromosome 14 and chromosome 7 were harbored.

### (Primers for detecting human chromosome 7)

3A4F7 : 5'-GCAAGACTGTGAGCCAGTGA-3' (SEQ ID No. 81)
3A4R7 : 5'-GGCTGCATCAGCATCATCTA-3' (SEQ ID No. 82)
(Primers for detecting human chromosome 14)
VH3F : 5'-AGTGAGATAAGCAGTGGATG-3' (SEQ ID No. 83)
VH3R : 5'-CTTGTGCTACTCCCATCACT-3' (SEQ ID No. 84)

In PCR, GeneAmp 2400 produced by Perkin-Elmer, Inc. was used as a thermal cycler, Ex Taq (Takara Shuzo Co., Ltd.) was used as a Taq polymerase, and as the buffer and dNTP (dATP, dGTP, dCTP and dTTP) those attached to Ex Taq (Takara Shuzo Co., Ltd.) were used under the reccommended conditions. The temperature and cycle condition were such that after thermal denaturation at 94°C for 1 to 5 minutes, a cycle of 98°C for 30 seconds, 54 to 58°C for 30 seconds to 1 minutes and 72°C for 30 seconds was performed 40 times. By this experiment, it was confirmed that the above-mentioned two sets of primers gave rise to PCR products. From the experiments described above, a hybrid clone harboring two human chromosomes, i.e., 14 and 7 was confirmed (TF clone×R clone was named RTF clone and TR clonexR clone was named RTR clone).

### (2) Site-specific translocation of human chromosome 7 fragment to chromosome 14 fragment SC20 in RTF and RTR hybrid clones

### (2)-1 Construction of Cre recombinase stably expressing vector pBS185hisD

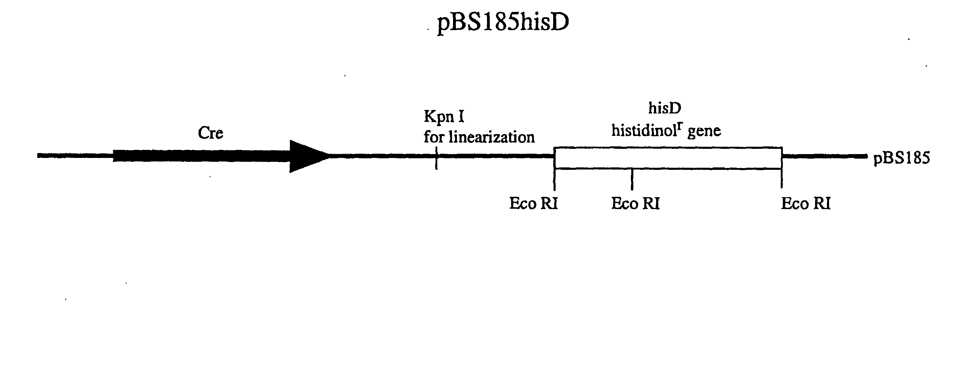

As described in the general description, site-specific translocation of human chromosome was performed by use of a Cre-loxP system. In this system too, it was anticipated that recombination efficiency between nonhomologous chromosomes as in this time was very low. Accordingly, in consideration of expressing Cre enzyme not temporarily but stably, the following expression vector pBS185hisD was constructed (Fig. 14).

Cre recombinase expression vector pBS185 (Gibco) was cleaved with restriction enzyme *Eco*RI (Boehringer) and *Bgl*II linker was inserted thereto (pBS185Bg). On the other hand, plasmid #1-132 containing a histidinol resistant gene cassette (endowed by Professor Shunichi Takeda of Medical Department of Kyoto University) was cleaved with restriction enzyme *Bam*HI (Boehringer) to cut out the histidinol resistant gene cassette, which was cloned to the *Bgl*IIsite of pBS185Bg (pBS185hisD). (2)-2 Site-specific translocation of human chromosome 7 fragment to chromosome 14 fragment SC20 in RMF and RMR hybrid clones by use of a Cre-loxP system

In the same manner as described above, Cre recombinase stably expressing vector pBS185hisD linearized with restriction enzyme *Kpn*I (Boehringer) was transfected to respective RTF and RTR hybrid clones and the clones were selectively cultivated in the presence of histidinol (1 mg/ml) for about 2 weeks. Thereafter, the obtained histidinol resistant cells were dispensed to 6-hole cultivation plates and genome DNA was extracted. PCR was performed by use of the primers described below with the extracted genome DNA used as a template and recombination between the chromosomes was confirmed.

### (Primers for detecting recombinants)

Sense (PGK): 5'-ATAGCAGCTTTGCTCCTTCG-3' (SEQ ID No. 85)
Antisense (GFP): 5'-TTCTCTCCTGCACATAGCCC-3' (SEQ ID No.
86)

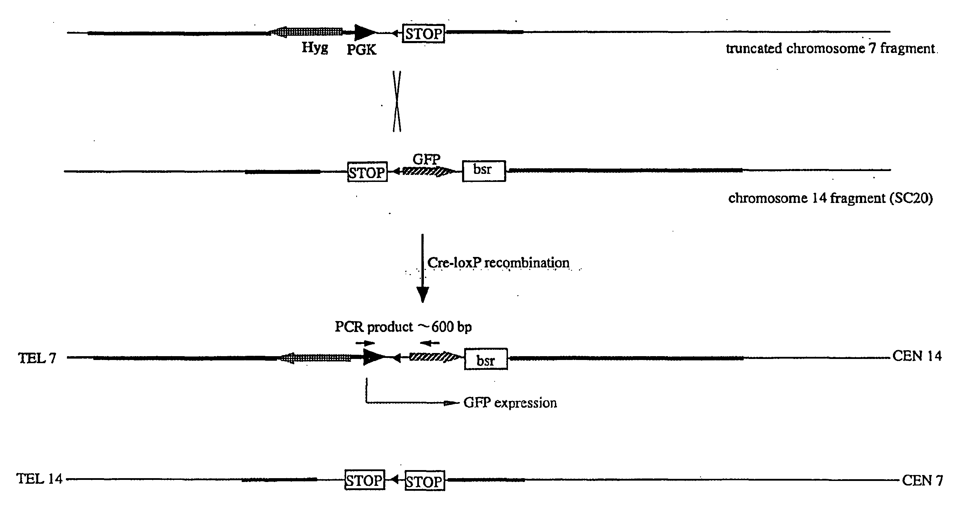

In PCR, GeneAmp 2400 produced by Perkin-Elmer, Inc. was used as a thermal cycler, Ex Taq (Takara Shuzo Co., Ltd.) was used as a Taq polymerase, and as the buffer and dNTP (dATP, dGTP, dCTP and dTTP) those attached to Ex Taq (Takara Shuzo Co., Ltd.) were used under the reccommended conditions. The temperature and cycle condition were such that after thermal denaturation at 94°C for 1 minute, a cycle of 98°C for 10 seconds, 61°C for 30 seconds and 72°C for 1 minute was performed 35 times. As a result, a PCR product of about 600 bp as shown in Fig. 15 was obtained. However, since it was anticipated that the efficiency of recombination by translocation between chromosomes was very low (1/10,000,000), nested PCR using the product obtained by this PCR as a template was performed to detect recombinants. The nested PCR was performed by use of 1 µl of PCR product amplified by primers PGK (SEQ ID No. 85) and GFP (SEQ ID No. 86) as a template and the PCR was further purformed using the same primers as the primers used for identifying the above-mentioned mouse Cyp3a cluster-deleted cell line (SEQ ID Nos. 60 and 61) as primers for nested PCR.

In PCR, GeneAmp 2400 produced by Perkin-Elmer, Inc. was used as a thermal cycler, Ex Taq (Takara Shuzo Co., Ltd.) was used as a Taq polymerase, and as the buffet and dNTP (dATP, dGTP, dCTP and dTTP), those which are attached were used under the reccommended conditions. The temperature and cycle condition were such that after thermal denaturation at 94°C for 1 minute, a cycle of 98°C for 10 seconds, 59°C for 30 seconds and 72°C for 30 seconds was performed 35 times. As a result, it was confirmed that cells that underwent translocation were included among the obtained resistant cell mass in which about 200 bp of PCR product was obtained. The resistant cell mass was further dispensed to four (4) 6-hole cultivation plates and cultivated in the presence of puromycin (0.3 µg/ml). Thereafter, the cells proliferated on 6-hole cultivation plates were repeatedly subjected to the selection by the nested PCR as described above and cultivation in the presence of puromycin to thereby concentrate the cells having human artificial chromosome #7-HAC. The cell pool after the concentration was cultivated until a cell density of about 10⁷ was reached and then suspended in 4 ml of PBS (phosphate buffer solution) to which 5% FBS and 1 mg/ml of propidium iodide (PI) had been added and analyzed by FACSVantage (Vecton Dickinson). As described above, if recombination translocation occurred between loxP's, GFP gene was reconstructed and expressed, so that the cell that underwent translocation could be detected by FACS. By repeating the sorting which corresponds to cell fraction conceivable of expressing GFP positive several times, the GFP-positive cells were cloned.

Further, the GFP-positive clone was subjected to FISH by use of human 14q ter-specific probe (for detecting human chromosome 14 long-arm telomere region, FITC labeled) and pGKPuro probe (for detecting human chromosome 7 fragment long-arm telomere region, rhodamine labeled) to confirm that FITC signal and rhodamine signal were detected at both terminal telomere regions on the same chromosome. Also, FISH was performed with human chromosome 14-specific probe and human chromosome 7-specific probe to confirm that signals derived from the both probes were observed on the same chromosome.

From the experiments described above, it could be concluded that in the GFP-positive clone, translocation as expected occurred and an artificial chromosome #7-HAC containing both human CYP3A gene cluster and MDR gene cluster on the same chromosome was constructed.

### Example 24 Introduction of human artificial chromosome #7-HAC from DT40 hybrid cell into Chinese hamster CHO cell

To introduce human artificial chromosome #7-HAC into mouse ES cell, first it was introduced into CHO cells.

The respective GFP-positive DT40 hybrids was cultivated in eight (8) dishes of 150 mm in diameter and when the cells became confluent, the medium was exchanged for RPMI1640 medium to which 20% FBS, 1% chicken serum, 10⁻⁴ M 2-mercaptoethanol, and 0.05 mg/ml colcemid had been added, and further cultivated for 36 hours to form microcells. The cells were suspended in 24 ml of serum RPMI1640 medium and 2 ml aliquots were dispensed to twelve (12) 25-cm³ centrifugation flasks (Corning) coated with 100 mg/ml of polyL-lysine in advance and cultivated at 37°C for 1 hour to cause the cells to adhere on the bottom of the flask.

The culture solution was removed and a cytochalasin B (10 mg/ml, sigma) solution that had been warmed to 37°C in advance was filled in the centrifugation flasks, followed by 1 hour's centrifugation at 34°C at 8,000 rpm. Microcells were suspended in serum-free DMEM medium and purified through 8 µm filter and then 5 µm filter. After completion of the purification, the microcells were centrifuged at 1,700 rpm for 10 minutes and suspended in 5 ml of serum-free DMEM medium. On the other hand, about 10⁷ CHO cells were peeled by trypsin treatment, washed in serum-free DMEM medium twice, and suspended in 5 ml of serum-free DMEM medium. Again, the microcells were centrifuged at 1,700 rpm for 10 minutes and without removing the supernatant, 5 ml of the previously obtained CHO suspension was carefully overlaid thereon. After centrifugation, the culture solution was removed and 0.5 ml of a 1:1.4 PEG solution [5 g of PEG1000 (Wako Pure Chemical Industry, Ltd.) and 1 ml of DMSO (Sigma) were dissolved in 6 ml of DMEM] was added, followed by vigorous stirring with a pipette for about 2 minutes. Thereafter, 10 ml of serum-free DMEM medium was slowly added over about 3 minutes and left to stand at 37°C for 10 minutes. After centrifugation, the cells were suspended in 10% FBS-added F12 medium (Gibco), dispensed to ten (10) 24-hole cultivation plates and cultivated at 37°C for 24 hours. Thereafter, the medium was exchanged for 800 mg/ml G418-containing F12 medium and elective culture was performed for 3 to 4 weeks.

Genome DNA was extracted from G418 resistant clone and PCR was performed under the same conditions as described above by use of primers for detecting CYP3A4 and VH3. Further, FISH was performed by use of human chromosome 14-specific probe and human chromosome 7-specific probe. From these results, it could be concluded that a CHO cell clone harboring objective human artificial chromosome #7-HAC was obtained.

### Example 25 introduction of #7-HAC from a CHO cell to a mouse ES cell

To prepare a chimeric mouse harboring #7-HAC, #7-HAC from the CHO cell harboring #7-HAC obtained in Example 24 above was introduced into a mouse ES cell.

According to the method of Tomizuka et al. (Nature Genet., 16:133-143, 1997), microcells were purified from about 10⁸ CHO cells harboring #7-HAC and suspended in 5 ml of DMEM. About 10⁷ mouse ES cells TT2F were peeled by trypsin treatment and after washing with DMEM three times, they were suspended in 5 ml of DMEM. The suspension was added to the centrifuged microcells and the mixture was centrifuged at 1,250 rpm for 10 minutes, followed by completely removing the supernatant. The precipitate was sufficiently loosened by tapping and 0.5 ml of a 1:1.4 PEG solution [5 g of PEG1000 (Wako Pure Chemical Industry, Ltd.) and 1 ml of DMSO (Sigma) were dissolved in 6 ml of DMEM] was added and sufficiently stirred for about 1.5 minutes. Thereafter, 10 ml of DMEM was slowly added thereto and the mixture was centrifuged at 1,250 rpm for 10 minutes. The cells were suspended in 30 ml of ES medium and dispensed to three (3) dishes of 100 mm in diameter (Corning), which had been plated with feeder cells in advance, followed by cultivation. After 24 hours, the medium was exchanged for a medium containing 300 mg/ml of G418 and elective culture was performed for about 1 week. Genome DNA was extracted from the obtained drug resistant colonies and PCR was performed under the same conditions as described above by use of primers for detecting CYP3A4 and VH3. Further, FISH was performed by use of human chromosome 14 -specific probe and human chromosome 7 specific probe. From these results, it could be concluded that an ES cell clone harboring objective human artificial chromosome #7-HAC was obtained.

### Example 26 Preparation of chimeric mice harboring human artificial chromosome #7-HAC

The ES cell clone obtained in Example 25 described above was injected to 8-cell stage embryos obtained by mating ICR or MCH (CLEA Japan) female and male mice in an amount of 10 to 12 cells per embryo. The embryos were cultivated in a medium for cultivating ES cells overnight to develop blastocysts. Thereafter, the injected embryos were transplanted in uterus of foster mother ICR mice in 2.5 days after pseudopregnancy treatment in an amount of about 10 embryos per uterus on one side to prepare chimeric mice.

### Industrial Applicability

The present invention has the following effects.
(1) By preparing a chimeric nonhuman mammalian having introduced therein human P450 genes on the level of chromosome in size, a mouse that expresses human P450 in an individual can be provided.
(2) A chimeric nonhuman mammalian having introduced therein human P450 genes on the level of chromosome in size can express P450 gene specifically in liver and small intestine, which are main organs for expression in humans, so that tissue specificity in drug metabolism in humans can be reproduced in an individual of nonhuman mammalian.
(3) Also, since the nonhuman mammalian of the present invention has introduced therein human P450 genes in a size on the level of chromosome, gene sequences necessary for the control of expression is considered to be contained therein. Therefore, in chimeric nonhuman mammalians, expression of human P450 can be induced in an individual of the nonhuman mammalian by use of a chemical that induces the expression of human P450.
(4) The above effect can be considered to be obtained not only in chimeric nonhuman mammalians themselves but also in their progeny.
(5) Furthermore, knockout nonhuman mammalians in which nonhuman mammalian endogenous P450 gene serving as counterpart has been disrupted can provide important knowledge on the function of human P450 gene and in addition thereto offsprings obtained by mating such a knockout nonhuman mammalian with a nonhuman mammalian having introduced therein human P450 genes or a progeny thereof are considered to be completely humanized with respect to specified P450 molecular species. Therefore, nonhuman mammalians having introduced therein human P450 that are completely humanized not only in tissue specificity and drug induction of expression or ratio of expression but also in function of metabolism with respect to a specified P450 molecular species can be considered to be provided.
(6) By use of the human P450-introduced nonhuman mammalians completely humanized with respect to a specified P450 molecular species, influence of drugs on humans, such as pharmacological effect, drug metabolism or toxicity, can be studied or anticipated without actually administering the drug to humans.
(7) It is possible to obtain human P450 protein having biological activity from chimeric nonhuman mammalians or a progeny thereof or individuals of completely humanized, human P450-introduced nonhuman mammalian, tissues or cells thereof. Such protein can readily provide human P450 protein that has conventionally been difficult to obtain and also provide a protein closer to P450 produced in the human body in consideration of modification of proteins, represented by phosphorylation or modification with sugar chains, that could not be solved by use of expression systems using bacteria represented by Escherichia coli.
(8) Also, in the case where tissues or cells derived from the above-mentioned nonhuman mammalians are used in cultivation systems after immortalization, human P450 protein can be supplied in a stable manner. Furthermore, they can be used as a material for studying drug metabolism in cultivation systems in humans.

## Claims

1. A nonhuman mammalian which harbors at least one human cytochrome P450 gene, wherein the expression of the gene is induced by a compound serving as a substrate for a product of the gene.

2. A nonhuman mammalian according to claim 1, wherein said human cytochrome P450 gene belongs to a CYP3A family.

3. A nonhuman mammalian according to claim 1, wherein said human cytochrome P450 gene is introduced by introduction of a YAC vector containing the gene or microcell fusion using a chromosome fragment containing the gene.

4. A nonhuman animal according to claim 3, wherein the mammalian is a chimeric animal.

5. A nonhuman animal, which is obtained by mating a wild-type nonhuman animal of the same kind as the nonhuman animal according to claim 4 with the nonhuman animal according to claim 4 and which harbors a chromosome fragment containing a human cytochrome P450 gene.

6. A nonhuman mammalian according to claim 1, wherein a cytochrome P450 gene inherent to said nonhuman mammalian that is a homolog of said human cytochrome P450 gene has been disrupted and expression of the inherent gene has been reduced or lost.

7. A nonhuman mammalian according to claim 4, wherein disruption of the cytochrome P450 gene inherent to said nonhuman mammalian has been performed using a Cre-loxP system.

8. A nonhuman mammalian according to any one of claims 1 to 7, wherein the mammalian is a mouse.

9. A cell, or organ or tissue containing the cell, the cell being derived from the nonhuman mammalian according to any one of claims 1 to 8 and capable of expressing a human cytochrome P450 gene.

10. A method for preparing a physical map for determining arrangement of mouse Cyp3a genes on a chromosome, comprising the steps of:
(a) screening a mouse BAC library by PCR or hybridization using PCR primers or a probe for hybridization for specifically detecting each mouse Cyp3a genes;
(b) repeating a cycle of screening the BAC library several times to prepare a BAC contig, the cycle comprising determining a terminal nucleotide sequence of the BAC clones selected in the above step and preparing a primer or a probe based on the nucleotide sequence; and
(c) determining both ends of a Cyp3a gene cluster by preparing a full-length cDNA probe of an unprescribed mouse Cyp3a gene and performing hybridization of the above-mentioned BAC contig using the probe under the gentle conditions.

11. A physical map which can be prepared by the method according to claim 10 and having elucidated the arrangement of the mouse Cyp3a genes on the chromosome.

12. A method of preparing a targeting vector for deleting mouse Cyp3a genes, comprising cloning a genome DNA corresponding to respective terminals of a mouse Cyp3a gene cluster based on the physical map according to claim 11 and inserting each of the obtained cloned fragments into a vector containing a loxP sequence, which is a recognition sequence of recombinase Cre derived from bacteriophage P1.

13. A pair of targeting vectors for deleting mouse Cyp3a genes that can be prepared by the method according to claim 12, wherein the respective vectors are to be incorporated in a mouse chromosome and only when said enzyme Cre is present, homologous recombination occurs between the loxP sequences, thereby deleting the whole Cyp3a gene cluster.

14. A method of deleting Cyp3a genes of a mouse cell, comprising the steps of introducing the vector according to claim 13 into a cell retaining pluripotency of a mouse and expressing enzyme Cre.

15. A mouse cell that can be prepared by the method according to claim 14, being deficient in Cyp3a genes and retaining pluripotency.

16. A method of preparing a knockout mouse deficient in Cyp3a genes, comprising the step of differentiating the mouse cell according to claim 15.

17. A chimeric mouse or a progeny thereof prepared by the method according to claim 16, being deficient in Cyp3a genes.

18. A mouse or a progeny thereof deficient in Cyp3a genes, obtained by mating the chimeric mouse according to claim 17 or a progeny thereof with a wild-type mouse.

19. A tissue derived from the chimeric mouse according to claim 16 or a progeny thereof or the chimeric mouse according to claim 17 or a progeny thereof.

20. A cell derived from the chimeric mouse according to claim 16 or a progeny thereof or the chimeric mouse according to claim 17 or a progeny thereof.

21. A mouse or a progeny thereof obtained by mating the mouse according to claim 8 or a progeny thereof with the chimeric mouse according to claim 17 or a progeny thereof, or the mouse according to claim 18 or a progeny thereof, the mouse or a progeny harboring a human chromosome containing human P450 genes and being deficient in mouse Cyp3a genes.

22. A method of producing biologically active human cytochrome P450, comprising the steps of cultivating an individual, tissue or cell of the mouse according to claim 8 or a progeny thereof, expressing the human cytochrome P450 genes harbored therein to produce biologically active human cytochrome P450, and recovering the human cytochrome P450.

23. A method of producing biologically active human cytochrome P450, comprising the steps of cultivating an individual, tissue or cell of the mouse according to claim 21 or a progeny thereof, expressing the human cytochrome P450 genes harbored therein to produce biologically active human cytochrome P450, and recovering the human cytochrome P450.

24. A method of examining pharmacological effect and/or metabolism of a drug, comprising the step of administering the drug to an individual, tissue or cell of the mouse according to claim 8 or a progeny thereof or the mouse according to claim 21 or a progeny thereof.
